# FASCICULE DE BREVET EUROPEEN

(11) **EP 2 097 374 B1**
(45) Date de publication et mention de la délivrance du brevet: **09.06.2010**
(21) Numéro de dépôt: 07871823.6
(22) Date de dépôt: 10.12.2007
(51) Int. Cl.: C07D 209/34, C07D 401/12, C07D 403/10, C07D 403/12, C07D 487/04, A61K 31/404

(54) **DERIVES DE 5-ALKYLOXY-INDOLIN-2-ONE, LEUR PREPARATION ET LEURS APPLICATIONS EN THERAPEUTIQUE COMME LIGANDS SELECTIVES DES RECEPTEURS V2 DE LA VASOPRESSINE**
5-ALKYLOXY-INDOLIN-2-ON-DERIVATE, IHRE HERSTELLUNG UND IHRE THERAPEUTISCHE ANWENDUNG ALS SELEKTIVE LIGANDEN VON V2-VASOPRESSIN-REZEPTOREN
5-ALKYLOXY-INDOLIN-2-ONE DERIVATIVES, PREPARATION THEREOF AND APPLICATION THEREOF IN THERAPY AS SELECTIVE LIGANDS OF V2 VASOPRESSIN RECEPTORS

(30) Priorité: 12.12.2006 FR 0610803
(43) Date de publication de la demande: 09.09.2009
(73) Titulaire: Sanofi-Aventis, 75013 Paris (FR)
(72) Inventeur: FOULON, Loïc, F-75013 Paris (FR); ROCHARD, Pierrick, F-75013 Paris (FR); SERRADEIL-LE GAL, Claudine, F-75013 Paris (FR); VALETTE, Gérard, F-75013 Paris (FR)
(74) Mandataire: Werner, Alain Henri
(86) Numéro de dépôt international: PCT/FR2007/002028
(87) Numéro de publication internationale: WO 2008/087278

(56) Documents cités:
- WO-A-93/15051
- FR-A1- 2 874 920
- FR-A1- 2 874 921

## Description

L'invention a pour objet des dérivés de 5-alkyloxy-indolin-2-one, leur procédé d'obtention et leurs applications thérapeutiques. Ces nouveaux dérivés sont affins et sélectifs des récepteurs V₂ de la vasopressine (« récepteurs V₂ ») et peuvent donc constituer des principes actifs de compositions pharmaceutiques.

La vasopressine (V) est une hormone connue pour son effet antidiurétique et son effet dans la régulation de la pression artérielle. Elle stimule plusieurs types de récepteurs : V₁ (V₁ₐ, V_{1b} ou V₃), V₂.

L'ocytocine (OT) a une structure peptidique proche de celle de la vasopressine.

Les récepteurs V₁ (V₁ₐ, V_{1b}), V₂ et OT sont localisés dans des tisus et organes communs (Jard S. et al., « Vasopressin and oxytocin receptors : an overview in progress » dans Endocrinology, Himura H. and Shizume K ed., ou encore : Pharmacol. Rev., 1991 43(1), 73-108).

Plusieurs documents décrivent des séries de composés non peptidiques possédant une affinité pour les récepteurs de la vasopressine et/ou de l'ocytocine, tels que WO2006/100080, WO2006/072458, WO2005/030755, WO2006/005609, EP 0 636 608, WO95/18105, WO03/008407, WO93/15051 ou encore WO97/15556.

L'objet de la présente invention est de trouver de nouveaux composés ayant une puissante sélectivité sur les récepteurs V₂ de la vasopressine, tout en présentant de bonnes propriétés pharmacologiques. Les composés selon l'invention présentent notamment une bonne stabilité métabolique, les rendant particulièrement aptes à leur utilisation en tant que médicament.

A cet effet, les inventeurs ont développé une nouvelle famille de composés, affins et sélectifs des récepteurs V₂ de la vasopressine. Ces nouveaux composés sont de puissants antagonistes de la liaison des récepteurs V₂ de la vasopressine. De plus, les composés de formule (I) selon l'invention, présentent une bonne stabilité métabolique, notamment sur microsomes hépatiques humains, confirmant ainsi l'intérêt de ces composés pour leur usage en tant que médicament.

L'invention a pour objet les composés de formule générale (I) qui suit. 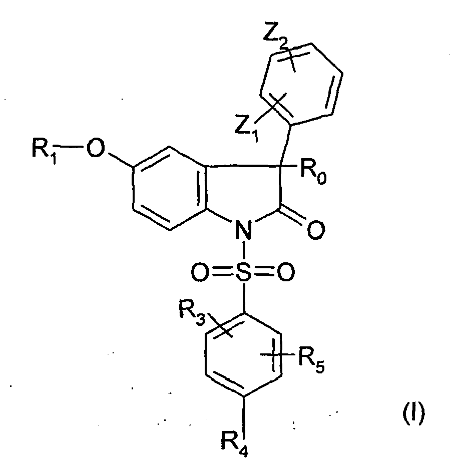 dans laquelle
- R₀ représente un groupe (C₁-C₄)alkyle, un groupe mono ou polyfluoro-(C₁-C₄)alkyle, un groupe -(CH₂)ₙ-cyclopropyle,
ou bien R₀ représente un groupe (C₂-C₄)alcényle ou (C₂-C₄)alcynyle ;
- R₁ représente un atome d'hydrogène, un groupe (C₁-C₅)alkyle, un groupe mono ou polyfluoro-(C₁-C₅)alkyle, un groupe hydroxy-(C₁-C₅)alkyle, un groupe -(CH₂)ₘ-(C₃-C₅)cycloalkyle ;
- Z₁ représente un atome d'hydrogène ou d'halogène, un groupe (C₁-C₄)alkyle, un groupe mono ou polyfluoro-(C₁-C₄)alkyle, un groupe (C₁-C₄)alcoxy, un groupe mono ou polyfluoro-(C₁-C₄)alcoxy, un groupe -(CH₂)ₙ-cyclopropyle, ledit groupe cyclopropyle étant non substitué ou substitué par un ou plusieurs atomes de fluor ;
- Z₂ représente un atome d'halogène ou un groupe T₁W, dans lequel T₁représente un groupe -(CH₂)ₙ- et W représente un atome d'hydrogène, un groupe (C₁-C₄)alkyle, mono ou polyfluoro-(C₁-C₄)alkyle ou un groupe cyclopropyle non substitué ou substitué par un ou plusieurs atomes de fluor,
   - ou bien W représente un groupe -C(O)NR₆R₇ dans lequel R₆ et R₇ représentent indépendamment l'un de l'autre un atome d'hydrogène, un groupe (C₁-C₆)alkyle, un groupe mono ou polyfluoro-(C₁-C₆)alkyle, un groupe -(CH₂)ₘ-(C₃-C₆)cycloalkyle ledit groupe cycloalkyle étant non substitué ou substitué par un ou plusieurs atomes de fluor , un hydroxy, un groupe NRR',
      **.** ou bien R₆ et R₇ représentent indépendamment l'un de l'autre un groupe -(CH₂)ₚ-pyrrofidinyle, -(CH₂)ₚ-pipéridyle, -(CH₂)ₚ₋pyridyle, lesdits groupes pyrrolidinyle, pipéridyle et pyridyle étant non substitués ou substitués par un ou plusieurs atomes d'halogène, un groupe (C₁-C₄)alkyle, un groupe mono ou polyfluoro(C₁-C₄)alkyle, un benzyle ou par un groupe -OR,
      . ou bien R₆ et R₇ représentent indépendamment l'un de l'autre un groupe -(CH₂)_{q}-NRₐR_{b},
      . ou bien R₆ et R₇ représentent indépendamment l'un de l'autre un groupe -(CH₂)ₛ-C(O)NRₐR_{b},
      . ou bien R₆ et R₇ représentent indépendamment l'un de l'autre un groupe -(CH₂)_{q}-OR,
      . ou bien R₆ et R₇ forment ensemble avec l'atome d'azote auquel ils sont rattachés un hétérocycle monocyclique non substitué ou substitué par un ou plusieurs atomes de fluor, un ou plusieurs groupes (C₁-C₄)alkyle, mono ou polyfluoro-(C₁-C₄)alkyle, -NR'R, ou -OR,
      . ou bien R₆ et R₇ forment ensemble avec l'atome d'azote auquel ils sont rattachés un hétérocycle bicyclique non substitué ou substitué par un ou plusieurs atomes de fluor, (C₁-C₄)alkyle, mono ou polyfluoro-(C₁-C₄)alkyle, -OR ou -NR'R ;
         ■ ou bien W représente un groupe -NR₈C(O)R₉ dans lequel :
      . R₈ représente un atome d'hydrogène, un groupe (C₁-C₄)alkyle, un groupe mono ou polyfluoro-(C₁-C₄)alkyle,
      . R₉ représente un atome d'hydrogène, un groupe (C₁-C₄)alkyle, un groupe mono ou polyfluoro(C₁-C₄)alkyle, -(CH₂)ᵣ-NR_{d}Rₑ, -(CH₂)ₘ-pyrrolidinyle, -(CH₂)ₘ-pipéridyle, -(CH₂)ₘ-pyridyle, lesdits groupes pyrrolidinyle, pipéridyle, ou pyridyle étant non substitués ou substitués par un ou plusieurs groupes (C₁-C₄)alkyle, halogène, mono ou polyfluoro-(C₁-C₄)alkyle, benzyle ;
         ■ ou bien W représente un groupe -NR₁₀R₁₁ dans lequel R₁₀ et R₁₁ représentent indépendamment l'un de l'autre un atome d'hydrogène, un groupe hydroxy, un (C₁-C₆)alkyle ou un groupe mono ou polyfluoro-(C₁-C₆)alkyle,
      . ou bien R₁₀ et R₁₁ forment ensemble avec l'atome d'azote auquel ils sont rattachés un hétérocycle monocyclique non substitué ou substitué par un ou plusieurs groupes (C₁-C₄)alkyle, un groupe mono ou polyfluoro-(C₁-C₄)alkyle ou oxo ;
         ■ ou bien W représente un groupe -OR₁₂ dans lequel R₁₂ représente un atome d'hydrogène, un groupe (C₁-C₄)alkyle, un groupe mono ou polyfluoro-(C₁-C₄)alkyle, un benzyle ou un groupe -(CH₂)_{q}-NR'R ;
         ■ ou bien W représente un groupe -C(O)O R₁₉
   - R₄ représente un groupe (C₁-C₄)alkyle, un groupe mono ou polyfluoro-(C₁-C₄)alkyle, un groupe -OR, (C₂-C₄) alcényle, nitro, COOR_{c}, un benzyloxy,
      ou bien R₄ représente un groupe -C(O)NR₁₃R₁₄ dans lequel R₁₃ et R₁₄ représentent indépendamment l'un de l'autre un atome d'hydrogène, un groupe (C₁-C₆)alkyle, ledit groupe alkyle étant non substitué ou substitué par un ou plusieurs atomes de fluor, un hydroxy, un groupe NRR', un groupe (C₁-C₆)alkyloxycarbonylamino, un groupe (C₁-C₆)alkyloxycarbonyl, ou dans lequel R₁₃ et R₁₄ représentent indépendamment l'un de l'autre, un groupe -(CH₂)ₙ-(C₃-C₆)cycloalkyle, ledit groupe cycloalkyle étant non substitué ou substitué par un ou plusieurs atomes de fluor,
      . ou bien R₄ représente un groupe -NR₁₅R₁₆ dans lequel R₁₅ et R₁₆ représentent indépendamment l'un de l'autre un atome d'hydrogène, un hydroxy, un groupe (C₁-C₄)alkyle , un groupe mono ou polyfluoro-(C₁-C₄)alkyle, un groupe -(CH₂)ₙ-(C₃-C₅)cycloalkyle non substitué ou substitué par un ou plusieurs atomes de fluor,
      . ou bien R₁₅ et R₁₆ forment ensemble avec l'atome d'azote auquel ils sont rattachés, un hétérocycle monocyclique,
      . ou bien R₄ représente un groupe -NR₁₇C(O)R₁₈ dans lequel :
         - R₁₇ représente un atome d'hydrogène, un groupe (C₁-C₄)alkyle, un groupe mono ou polyfluoro-(C₁-C₄)alkyle,
         - R₁₈ représente un groupe (C₁-C₆)alkyle, un groupe mono ou polyfluoro-(C₁-C₆)alkyle, -(CH₂)n-(C₃-C₆)cycloalkyle, un groupe -NR_{d}Rₑ, un groupe phényle, ledit groupe phényle étant lui-même non substitué ou susbtitué par un ou plusieurs groupes (C₁-C₄)alkyle, un groupe mono ou polyfluoro-(C₁-C₄) alkyle;
         - R₁₉ représente un atome d'hydrogène, un groupe (C₁-C₆)alkyle, un groupe mono ou polyfluoro-(C₁-C₆)alkyle, un groupe -(CH₂)_{q}-NRₐR_{b}, un groupe -(CH₂)_{q}-OR, un groupe -(CH₂)ₚpyrrolidinyle ou un groupe -(CH₂)ₚ-pipéridyle, lesdits groupes pyrrolidinyle et pipéridyle étant non substitués ou substitués par un ou plusieurs atomes de fluor, un groupe (C₁-C₄)alkyle, un groupe mono ou polyfluoro(C₁-C₄) alkyle.
   - R₃ et R₅ représentent indépendamment l'un de l'autre un atomes d'hydrogène, d'halogène, un groupe (C₁-C₄)alkyle, un groupe mono ou polyfluoro-(C₁-C₄)alkyle, un groupe (C₁-C₄)alcoxy ou mono ou polyfluoro-(C₁-C₄)alcoxy ;
   - Rₐ et R_{b} représentent indépendamment l'un de l'autre :
      . un atome d'hydrogène, un groupe (C₁-C₄)alkyle, un groupe mono ou polyfluoro-(C₁-C₄)alkyle, un groupe -(CH₂)ₙ-cyclopropyle, ledit cyclopropyle étant non substitué ou substitué par un ou plusieurs atomes de fluor,
      . ou bien Rₐ et R_{b} forment ensemble avec l'atome d'azote auquel ils sont rattachés un groupe hétérocycle monocycle, ledit groupe hétérocycle monocycle étant non substitué ou substitué par un ou plusieurs groupes hydroxyle, (C₁-C₄)alkyle, mono ou polyfluoro-(C₁-C₄)alkyle, (C₁-C₄)alcoxy, mono ou polyfluoro(C₁-C₄)alcoxy, ou par un groupe -NRR' ;
   - R' et R représentent indépendamment l'un de l'autre un atome d'hydrogène, un groupe (C₁-C₄)alkyle ou mono ou polyfluoro-(C₁-C₄)alkyle ;
   - R_{c} représente un atome d'hydrogène, un groupe (C₁-C₄)alkyle, mono ou polyfluoro-(C₁-C₄)alkyle ou un benzyle ;
   - R_{d} et Rₑ représentent indépendamment l'un de l'autre un atome d'hydrogène, d'halogène, un groupe (C₁-C₆)alkyle ou mono ou polyhalogéno-(C₁-C₆)alkyle,
      . ou bien R_{d} et Rₑ forment ensemble avec l'atome d'azote auquel ils sont rattachés un groupe hétérocycle monocycle, non substitué ou substitué par un ou plusieurs atomes de fluor, un ou plusieurs groupes (C₁-C₄)alkyle ou mono ou polyfluoro-(C₁-C₄)alkyle ou un groupe -OR ;
   - m peut représenter la valeur 0, 1 ou 2 ;
   - n peut représenter la valeur 0 ou 1 ;
   - p peut représenter la valeur 0, 1, 2 ou 3 ;
   - q peut représenter la valeur 2, 3, 4 ou 5 ;
   - r peut représenter la valeur 0, 1, 2, 3 ou 4 ;
   - s peut représenter la valeur 1, 2 ou 3.

Les composés de formule générale (I) peuvent exister sous forme de tautomères. Ainsi, l'invention a pour objet les composés de l'invention sous toutes leurs formes tautomères.

Les composés de formule générale (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule générale (I) peuvent comporter des doubles liaisons ou un ou plusieurs cycles saturés. Ils peuvent donc exister sous forme d'isomères cis/trans. Ces isomères ainsi que leurs mélanges font partie de l'invention.

Les composés de formule générale (I) peuvent exister à l'état de bases ou d'acides et de sels d'addition. De tels sels d'addition font partie de l'invention.

Ces sels sont avantageusement préparés avec des acides ou des bases pharmaceutiquement acceptables, mais les sels d'autres acides ou bases utiles, par exemple, pour la purification ou l'isolement des composés de formule générale (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Selon la présente invention, les N-oxydes des composés comportant une amine font également partie de l'invention.

Les composés de formule (I) selon la présente invention comprennent également ceux dans lesquels un ou plusieurs atomes d'hydrogène, de carbone ou d'halogène, notamment de chlore ou de fluor ont été remplacés par leurs isotopes radioactifs par exemple le tritium pour remplacer l'hydrogène ou le carbone 14 pour remplacer le carbone 12. De tels composés marqués sont utiles dans des travaux de recherche, de métabolisme ou de pharmacocinétique, dans des essais biochimiques en tant que ligands de récepteurs.

Les prodrogues des composés de formule (I) selon la présente invention sont également. On entend par prodrogues des composés qui sont métabolisés in vivo en composés de l'invention. Des exemples de prodrogues sont décrits notamment dans John B. Taylor (Vol. Ed): Comprehensive Medicinal Chemistry, Pergamon Press, 1990, Vol. 5 p.122-132 et dans C. Wermuth (Ed.): The Practice of Medicinal Chemistry, Elsevier Academic Press, 2003, p.561-585. On peut citer comme exemple de prodrogues des composés dans lesquels un groupe ester -COO(C₁-C₆)alkyle, un groupe -OC(O)R ou un groupe amino dans lesquel un ou deux hydrogènes sont remplacés par des groupes (C₁-C₆)alkyle sont les prodrogues de composé (I) selon l'invention contenant respectivement un groupe acide carboxylique -COOH, un groupe alcool -OH ou un groupe amine primaire -NH2 . En outre, les composés de l'invention selon (I) peuvent eux-mêmes se comporter in vivo comme prodrogues d'autres composés de l'invention selon (I). A titre d'exemple, on peut citer un groupe aminocarbonyle -C(O)NR₆R₇ , R₆ et R₇ étant définis tel que précédemment susceptible de mener à un composé (I) selon l'invention comportant un groupe -COOH.

Dans le cadre de l'invention, on entend par :
- Cₜ-C_{z} où t et z peuvent prendre les valeurs de 1 à 6, une chaîne carbonée pouvant avoir de t à z atomes de carbone, par exemple C₁₋₆ représente une chaîne carbonée qui peut avoir de 1 à 6 atomes de carbone ou encore C₁₋C₃ représente une chaîne carbonée qui peut avoir de 1 à 3 atomes de carbone ;
- ou bien Cₓ₋C_{y} où x et y peuvent prendre les valeurs de 3 à 6, un cycle carboné saturé pouvant avoir de x à y atomes de carbone, par exemple C₃₋₆ représente un cycle carboné saturé qui peut avoir de 3 à 6 atomes de carbone ;
- alkyle, un groupe aliphatique saturé, linéaire ou ramifié; par exemple, un groupe C₁₋C₆ alkyle représente une chaîne carbonée de 1 à 6 atomes de carbone, linéaire ou ramifiée, notamment un méthyle, éthyle, propyle, isopropyle, butyle, isobutyle, secbutyle, tertbutyle, pentyle ;
- un groupe alcényle : un groupe aliphatique mono- ou poly-insaturé, linéaire ou ramifié, comprenant par exemple une ou deux insaturations éthyléniques. Par exemple, un groupe C₂₋C₆ alcényle peut représenter un éthényle, propényle ...;
- un groupe alcynyle : un groupe aliphatique mono- ou poly-insaturé, linéaire ou ramifié, comprenant par exemple une ou deux insaturations acétyléniques. Par exemple, un groupe C₂₋ C₆ alcynyle peut représenter un éthynyle, propynyle ... ;
- alcoxy, un groupe alkyloxy à chaîne aliphatique saturée, linéaire ou ramifiée ;
- atome d'halogène : un fluor, un chlore, un brome ou un iode ;
- un groupe polyfluoroalkyle : un groupe alkyle tel que défini précédemment, dont 1 ou plusieurs atomes d'hydrogène sont remplacés par un atome de fluor respectivement. A titre d'exemple on peut citer le groupe trifluorométhyle (-CF₃) dans lequel 3 atomes d'hydrogène ont été remplacé par 3 atomes de fluor ;
- un groupe polyfluoroalcoxy : un groupe alcoxy tel que défini précédemment, dont 1 ou plusieurs atomes d'hydrogène sont remplacés par un atome de fluor respectivement. A titre d'exemple on peut citer le groupe trifluorométhoxy (-OCF₃) dans lequel 3 atomes d'hydrogène ont été remplacé par 3 atomes de fluor ;
- un groupe hétérocycle monocyclique: un groupe monocyclique saturé de 5 à 7 chaînons comportant un à plusieurs hétéroatomes tels que les atomes d'azote, d'oxygène ou de soufre. A titre d'exemples, on peut citer les groupes pyrrolidinyle, dihydropyrrolidinyle, pipéridyle, tétrahydropyranyle, morpholinyle, pipérazinyle, tétrahydrofuranyle, thiomorpholine, azépinyle, homomorpholinyle, homopipérazinyle ;
- un hétérocycle bicyclique: une structure bicyclique comprenant un à plusieurs hétéroatomes tels que les atomes d'azote, d'oxygène ou de soufre, ladite structure étant composée de 2 groupes cycliques saturés ou partiellement insaturés, fusionnés (c'est-à-dire que lesdits groupes possèdent entre eux une liaison en commun) ou pontés (c'est-à-dire qu'au moins 2 atomes de la structure bicyclique sont reliés par un une liaison simple ou une chaîne carbonée pouvant comporter de 1 à 4 atomes de carbone), pouvant comporter de 6 à 18 chaînons.

A titre d'exemple d'hétérocycle bicyclique fusionné saturé, on peut citer le groupe octahydro-pyrrolo[3,4-b]pyrrole :

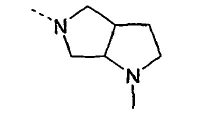

A titre d'exemple d'hétérocycle bicyclique ponté saturé, on peut citer le groupe 2,5-diazabicyclo[2.2.1]heptane

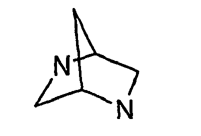

La présente invention a également pour objet des procédés de préparation des composés de formule générale (I).

Ainsi, les composés de l'invention peuvent être préparés par les méthodes illustrées dans les schémas qui suivent, dont les conditions opératoires sont classiques pour l'homme du métier.

On entend par groupe protecteur PG, un groupe permettant d'empêcher la réactivité d'une fonction ou position, lors d'une réaction chimique pouvant l'affecter, et qui restitue la molécule après clivage selon des méthodes connues de l'homme du métier.

Par groupe protecteur temporaire des amines ou alcools, on entend les groupes protecteurs tels que ceux décrits dans Protective Groups in Organic Synthesis, Greene T.W. et Wuts P.G.M., Ed. Willey Intersciences 1999 et dans Protecting Groups, Kocienski P.J., 1994, Georg Thieme Verlag.

On peut citer par exemple des groupements protecteurs temporaires des amines : benzyles, carbamates, (tels que *tert*-butyloxycarbonyle clivables en milieu acide, benzyloxycarbonyle clivables par hydrogénolyse), des groupements protecteurs temporaires d'acides carboxyliques : esters d'alkyle (tels que méthyle ou éthyle, *tert-*butyle hydrolysables en milieu basique ou acide) et benzyliques hydrogénolysables, des des groupements protecteurs temporaires d'alcools ou des phénols tels que les éthers de tétrahydropyranyle, métyloxyméthyle ou méthyléthoxyméthyle, *tert*-butyle et benzyle, des groupements protecteurs temporaires de dérivés carbonylés tels que les acétals linéaires ou cycliques comme par exemple 1,3-dioxane-2-yle ou 1,3-dioxolan-2-yle ; et se référer aux méthodes générales bien connues décrites dans Protective Groups, cité ci-dessus.

L'homme de l'art sera à même de choisir les groupes protecteurs appropriés. Les composés de formule (I) peuvent comporter des groupes précurseurs d'autres fonctions qui sont générées ultérieurement en une ou plusieurs autres étapes.

On entend par groupe partant dans ce qui suit, un groupe pouvant être facilement clivé par rupture hétérolytique d'une liaison ; on peut citer par exemple, les halogènes (I, Br, Cl, F) ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « March's Advanced Organic Chemistry », J. March, 5th Edition, Wiley Interscience, p. 449.
On entend par groupe précurseur, dans ce qui suit, tout groupe susceptible d'être transformé en une ou plusieurs étapes en un autre groupe par les réactions chimiques connues de l'homme du métier.

Parmi les composés objet de l'invention, on peut citer un premier groupe de composés de formule générale (I), dans laquelle Rₒ représente un groupe (C₁-C₃)alkyle en particulier un méthyle, les autres groupes étant tels que définis pour le composé de formule générale (I).

Parmi les composés objet de l'invention, on peut citer un second groupe de composés de formule générale (I), dans laquelle R₀ représente un groupe (C₁-C₃)alkyle en particulier un méthyle et R₁ représente un groupe (C₁-C₅)alkyle, les autres groupes étant tels que définis pour le composé de formule générale (I).
Parmi les composés objet de l'invention, on peut citer un troisième groupe de composés de formule générale (I), dans laquelle R₀ représente un groupe méthyle et R₁ représente un groupe (C₁-C₂)alkyle, plus particulièrement un éthyle, les autres groupes étant tels que définis pour le composé de formule générale (I).
Parmi les composés objet de l'invention, on peut citer un quatrième groupe de composés de formule générale (I), dans laquelle Z₁ représente un atome d'halogène, plus particulièrement un atome de fluor ou de chlore, les autres groupes étant tels que définis pour le composé de formule générale (I).
Parmi les composés objet de l'invention, on peut citer un cinquième groupe de composés de formule générale (I), dans laquelle Z₂ représente un groupe T₁W, dans lequel T₁ représente un groupe -(CH₂)ₙ- avec n égal à 0 et W représente :
■ un groupe -C(O)O R₁₉,
■ un groupe -C(O)NR₆R₇,
dans lesquels R₁₉, R₆ et R₇ et les autres groupes sont tels que définis pour le composé de formule générale (I).
Parmi les composés objet de l'invention, on peut citer un sixième groupe de composés de formule générale (I), dans laquelle Z₁ est en position -2.
Parmi les composés objet de l'invention, on peut citer un septième groupe de composés de formule générale (I), dans laquelle Z₁ est en position -2 et Z₂ est en position -5.
Parmi les composés objet de l'invention, on peut citer un huitième groupe de composés de formule générale (I), dans laquelle R₄ représente un groupe -C(O)NR₁₃R₁₄, dans lequel R₁₃, R₁₄ et les autres groupes étant tels que définis pour le composé de formule générale (I).
Parmi les composés objet de l'invention, on peut citer un neuvième groupe de composés de formule générale (I), dans laquelle Z₂ représente un groupe T₁W, dans lequel T₁ représente un groupe -(CH₂)ₙ- avec n égal à 0 et W représente un groupe -OR₁₂ dans lequel R₁₂ représente un atome d'hydrogène.
Parmi les composés objet de l'invention, on peut citer un dixième groupe de composés de formule générale (I), dans laquelle Z₁ est en position -2 et Z₂ représente un halogène.
Parmi les composés objet de l'invention, on peut citer les composés suivants :
N-tert-Butyl-4-[3-(2-chloro-phényl)-5-éthoxy-3-méthyl-2-oxo-2,3-dihydro-indole-1-sulfonyl]-benzamide (ex.2)
N-tert-Butyl-4-[3-(2-fluoro-phényl)-5-éthoxy-3-méthyl-2-oxo-2,3-dihydro-indole-1-sulfonyl]-benzamide (ex.6)
3-[1-(4-tert-Butylcarbamoyl-benzènesulfonyl)-5-éthoxy-3-méthyl-2-oxo-2, 3-dihydro-1H-indol-3-yl]-4-chloro-benzoate de méthyle(ex.9)
N-tert-Butyl-4-[3-(2-chloro-phényl)-5-éthoxy-3-méthyl-2-oxo-2,3-dihydro-indole-1-sulfonyl]-benzamide (ex.12)
N-Cyclopentyl-4-[3-(2-chloro-phényl)-5-éthoxy-3-méthyl-2-oxo-2,3-dihydroindole-1-sulfonyl]-benzamide (ex.13)
N-tert-Butyl-3-méthoxy-4-[3-(2-chloro-phényl)-5-éthoxy-3-méthyl-2-oxo-2, 3-dihydro-indole-1-sulfonyl]-benzamide (ex.15)
N-tert-Butyl-3-méthoxy-4-[3-(2-fluoro-phényl)-5-éthoxy-3-méthyl-2-oxo-2,3-dihydro-indole-1-sulfonyl]-benzamide (ex.16)
Acide 3-[1-(4-tert-Butylcarbamoyl-2-méthoxy-benzènesulfonyl)-5-éthoxy-3-méthyl-2-oxo-2,3-dihydro-1H-indol-3-yl]-4-chloro-benzoïque (ex.19)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-éthyl-benzamide(ex.21)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-pyridyl)-benzamide(ex.22)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(3-diméthylamino-propyl)-benzamide(ex.25)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(1-méthyl-pipéridin-4-yl)-benzamide(ex.28)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-pyrrolidin-1-yl-éthyl)-benzamide(ex.29)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-diéthylamino-éthyl)-benzamide(ex.33)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-morpholin-4-yl-éthyl)-benzamide(ex.36)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-[2-(4-méthyl-pipérazin-1-yl)-éthyl]-benzamide(ex.37)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-[3-(4-méthyl-pipérazin-1-yl)-propyl]-benzamide(ex.40)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(R)-pyrrolidin-3-yl-benzamide(ex.43)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-pipéridin-4-yl-benzamide(ex.44)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-2-amino-éthyl-benzamide(ex.47)
Acide 3-[1-(4-tert-butylcarbamoyl-benzènesulfonyl)-5-éthoxy-3-méthyl-2-oxo-2,3-dihydro-1H-indol-3-yl]-4-chloro-benzoïque(ex.48)
4-Chloro-3-[1-(4-tert-butylcarbamoyl-benzènesulfonyl)-5-éthoxy-3-méthyl-2-oxo-2,3-dihydro-1H-indol-3-yl]-N-(1-méthyl-pipéridin-4-yl)-benzamide(ex.51)
4-Chloro-3-[1-(4-tert-butylcarbamoyl-benzènesulfonyl)-5-éthoxy-3-méthy!-2-oxo-2,3-dihydro-1H-indol-3-yl]-N-(2-diéthylamino-éthyl)-benzamide(ex.53)
N-tert-Butyl-4-{3-[2-chloro-5-(3-dimethylamino-propionylamino)-phényl]-5-éthoxy-3-méthyl-2-oxo-2,3-dihydro-indole-1-sulfonyl}-3-méthoxy-benzamide(ex.58)
1-Méthyl-pipéridine-4-carboxylic acid {3-[1-(4-tert-butylcarbamoyl-2-méthoxybenzènesulfonyl)-5-éthoxy-3-méthyl-2-oxo-2,3-dihydro-1H-indol-3-yl]-4-chloro-phényl}-amide(ex.62)
N-(2-fluoro-1,1-diméthyl-éthyl)-4-[3-(2-chloro-phényl)-5-éthoxy-3-méthyl-2-oxo-2,3-dihydro-indole-1-sulfonyl]- benzamide(ex.66)
4-[3-(2-Chloro-phényl)-5-éthoxy-3-méthyl-2-oxo-2,3-dihydro-indole-1-sulfonyl]-N-(2-hydroxyl-1,1-diméthyl-éthyl)-benzamide(ex.72)
4-Chloro-3-[1-(4-tert-butylcarbamoyl-benzènesulfonyl)-5-éthoxy-3-méthyl-2-oxo-2,3-dihydro-1H-indol-3-yl]-N-[2-(4-hydroxy₋pipéridin-1-yl)éthyl]-benzamide(ex.79)
4-Chloro-3-[1-(4-tert-butylcarbamoyl-benzènesulfonyl)-5-éthoxy-3-méthyl-2-oxo-2,3-dihydro-1H-indol-3-yl]-N-2,2,2-trifluoroéthyl-benzamide(ex.80)
4-Chloro-3-[1-(4-tert-butylcarbamoyl-benzènesulfonyl)-5-éthoxy-3-méthyl-2-oxo-2,3-dihydro-1H-indol-3-yl]-N-2-hydroxy-éthyl-benzamide(ex.83)
4-Chloro-3-[1-(4-tert-butylcarbamoyl-benzènesulfonyl)-5-éthoxy-3-méthyl-2-oxo-2,3-dihydro-1H-indol-3-yl]-N-cyclopentyl-benzamide(ex.87)
4-Chloro-3-[1-(4-tert-butylcarbamoyl-benzènesulfonyl)-5-éthoxy-3-méthyl-2-oxo-2,3-dihydro-1H-indol-3-yl]-N-[trans-(4-hydroxy-cyclohexyl)]-benzamide(ex.88)
4-Chloro-3-[1-(4-tert-butylcarbamoyl-benzènesulfonyl)-5-éthoxy-3-méthyl-2-oxo-2,3-dihydro-1H-indol-3-yl]-N-[trans-(4-diméthylamino-cyclohexyl)]-benzamide(ex.90)
N-tert-Butyl-4-[3-méthyl-3-phényl-5-éthoxy-2-oxo-2,3-dihydro-indole-1-suifonyl]-benzamide (ex.98)
N-tert-Butyl-3-méthoxy-4-[3-méthyl-3-phényl-5-éthoxy-2-oxo-2,3-dihydro-indole-1-sulfonyl]-benzamide (ex.99)
N-tert-Butyl-4-[3-méthyl-3-(2-fluoro-phényl)-5-éthoxy-2-oxo-2,3-dihydro-indole-1-sulfonyl]-benzamide (ex.101)
N-tert-Butyl-4-[3-méthyl-3-(2-chloro-6-fluoro-phényl)-5-éthoxy-2-oxo-2,3-dihydro-indole-1-sulfonyl]-benzamide (ex.125)

Selon un mode de réalisation, les composés de formule générale (I) peuvent être obtenus selon le schéma 1 qui suit.

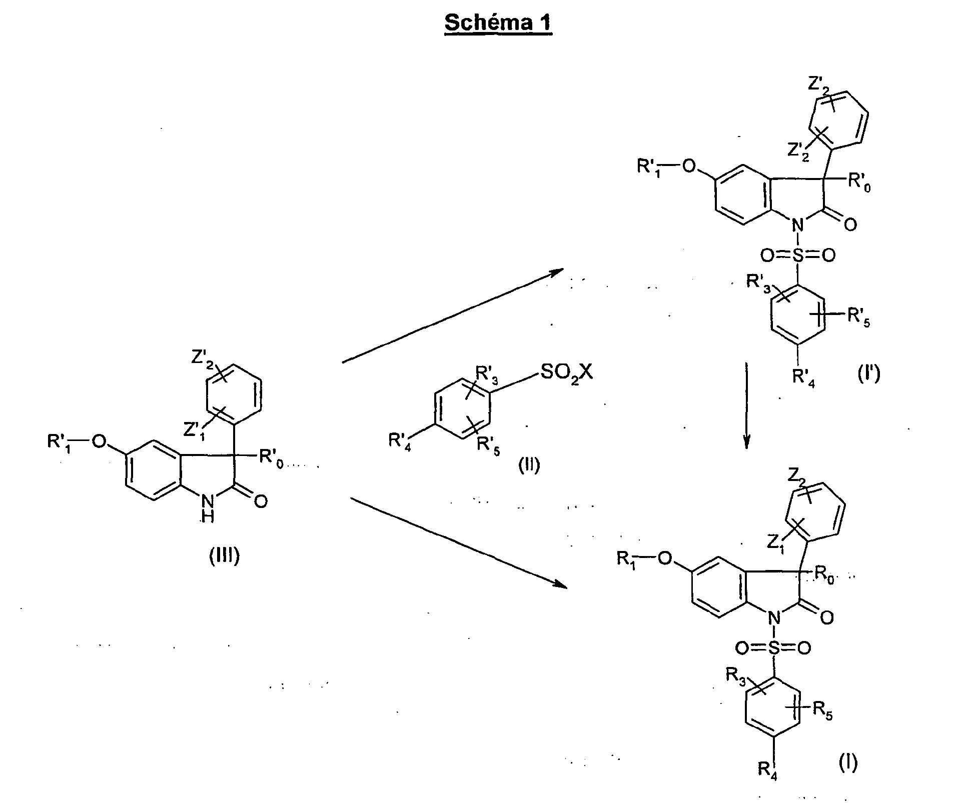

Selon le schéma 1, on obtient les composés de formule (I) par réaction d'un composé de formule (II) avec un composé de formule (III). Dans les composés de formule (II), X représente un atome d'halogène et les groupes R'₃, R'₄ et R'₅ représentent respectivement et indépendemment les uns des autres les groupes R₃, R₄ et R₅ tels que définis pour le composé de formule (I) ou des groupes précurseurs des groupes R₃, R₄ et R₅. Dans les composés de formule (III), les groupes R'₀, R'₁, Z'₁ et Z'₂ représentent respectivement et indépendemment les uns des autres les groupes R₀, R₁, Z₁ et Z₂ tels que définis pour le composé de formule (I) ou des groupes précurseurs des groupes R₀, R₁, Z₁ et Z₂. Les composés de formule (I) sont obtenus directement par réaction d'un composé de formule (II) avec un composé de formule (III) quand les groupes R'₀, R'₁, R'₃, R'₄, R'₅, Z'₁ et Z'₂ représentent respectivement les groupes R₀, R₁, R₃, R₄, R₅, Z₁ et Z₂ tels que définis pour le composé de formule (I). Alternativement, les composés de formule (I) sont obtenus indirectement via un composé de formule (I) quand au moins un des groupes R'₀, R'₁, R'₃, R'₄, R'₅, Z'₁ et Z'₂ représente respectivement un groupe précurseur des goupes R₀, R_{1,} R₃, R₄, R₅, Z₁ et Z₂ tels que définis pour le composé de formule (I).

L'addition d'un composé de formule (II) avec un composé de formule (III) s'effectue en présence d'un hydrure métallique comme par exemple l'hydrure de sodium ou d'un alcoolate alcalin comme par exemple le *tert*-butylate de potassium à des températures comprises entre -40° et 25°C, dans un solvant anhydre tel que le tétrahydrofurane (THF).

Les composés de formule (I') peuvent également représenter des composés de formule (I).

Les transformations des groupes précurseurs R'₀, R'₁, Z'₁, Z'₂, R'₃, R'₄ et R'₅ respectivement et indépendemment les uns des autres en groupes R₀, R₁, Z₁, Z₂, R₃, R₄ et R₅ tels que définis, sont obtenues selon des techniques classiques et générales bien connues de l'homme du métier, par exemple par des réactions d'alkylation, d'acylation, d'oxydation ou de réduction.

Les composés de formule (II) sont commerciaux, connus ou préparés selon des méthodes connues de l'homme du métier. On peut notamment se référer aux méthodes décrites dans le document WO98/25901 (page 20 lignes 25 à 34 et page 21, lignes 1 à 20).

On peut également dans l'invention préparer des composés de formule (II) dans lesquels R'₄ représente un groupe -C(O)OR_{c} ou un groupe -C(O)NR₁₃R₁₄ par réaction chimiosélective respectivement d'un alcool H-OR_{c} ou d'une amine H-NR₁₃R₁₄ sur un composé de formule (II) dans lequel X représente un atome de chlore et R'₄ représente un groupe -C(O)Cl. On opère à des températures comprises entre -30°C et 0°C dans des solvants anhydres peu polaires tels que les éthers ou les solvants chlorés par exemple le dichlorométhane.

Les composés de formule (III) peuvent être préparés selon le schéma 2 qui suit.

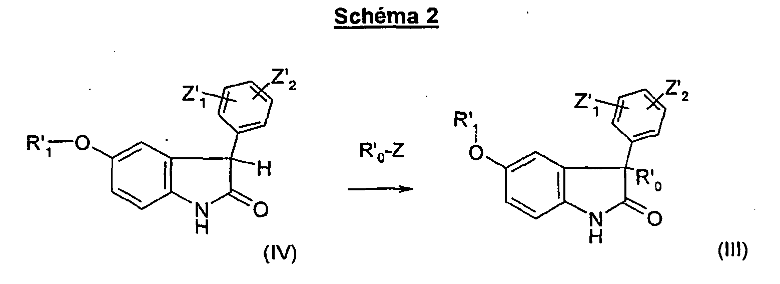

Selon le schéma 2, les composés de formule (III) peuvent être préparés par la réaction d'un composé de formule (IV) dans laquelle R'₁, 2'₁ et Z'₂ sont tels que définis pour le composé de formule (III), en présence d'une base telle que un alcoolate alcalin, tertiobutylate de potassium par exemple avec un dérivé R'₀-Z dans lequel R'₀ est tel que défini pour le composé de formule (III) et Z représente un groupe partant tel qu'un iode ou un brome ou bien un ester d'acide sulfonique tel que mésylate ou tosylate, dans un solvant anhydre tel que la DMF ou le THF à des températures comprises entre -40°C et 20C.

Alternativement, les composés de formule (III) peuvent être obtenus selon le schéma 3 qui suit (Ph = phényle).

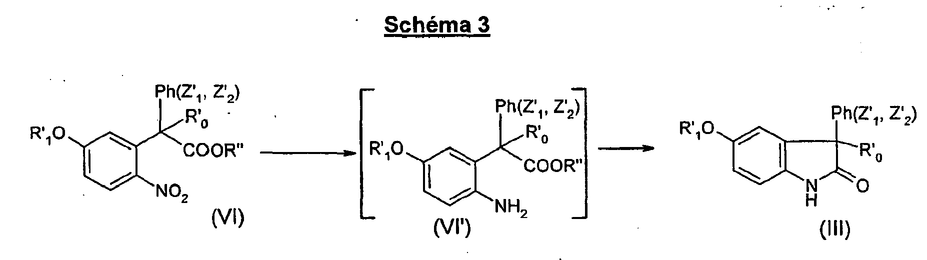

Selon le schéma 3, les composés de formule (III) peuvent être obtenus à partir des composés de formule dans laquelle R'₁, R'₀, Z'₁ et Z'₂ sont tels que définis pour les composés de formule (III) et R" représente un groupe (C₁-C₃)alkyle - par cyclisation de l'amine de formule (VI') - dans laquelle R'₁, R'₀, Z'₁ et Z'₂ sont tels que définis pour les composés de formule (III) et R" représente un groupe (C₁-C₃)alkyle - générée in situ lors de la réduction du groupe nitro porté par le composé de formule (VI). On opère en présence d'un métal en milieu acide tel que l'étain ou le fer en milieu acide tel que l'acide acétique à des températures comprises entre 30 et 100°C.

Les composés de formule (IV) peuvent être obtenus selon le schéma 4 qui suit.

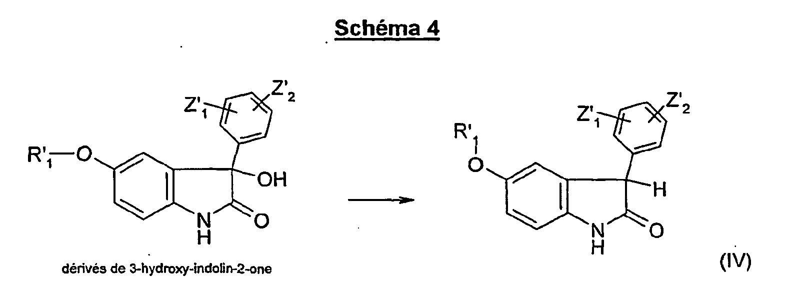

Selon le schéma 4, les composés de formule (IV) peuvent être obtenus par réduction de composés dérivés de 3-hydroxy-indolin-2-one en faisant réagir du chlorure d'étain en millieu acide, par analogie avec la méthode décrite dans Tetrahedron Letters 1996, 52(20), 7003-7012 ou Bioorganic and Medicinal Chemistry Letters 1997, 7(10), 1255-1260. R'₁. Z'₁ et Z'₂ sont tels que définis pour les composés de formule (III).

Les dérivés 3-hydroxy-indolin-2-one sont connus ou préparés à partir d'isatines commerciales ou connues par réaction avec un dérivé organométallique tel qu'un organolithien ou un organomagnésien selon, par exemple Biorg.Med.Lett. 7(10) ;1997 ; 1255.

Les composés de formule (IV) peuvent également être obtenus par les méthodes reprises notamment dans le document WO01/74775 (page 19, lignes 16 à 25 et page 20, lignes 1, 2). On peut citer par exemple réaction de Brunner décrite dans Tetrahedron 1986 ; 42(15), 4267-4272, la réaction de cyclisation de dérivés mandélamides décrite dans J. Org. Chem. 1968, 33 (4), 1640-1643, la réaction de cyclisation en présence d'acide formique décrite dans J. Chem. Soc. Perkin Trans., 1986, 1, 349-360, la réaction de cyclisation selon J. Am. Chem. Soc., 1985, 107(2), 435-443.

Les composés de formule (IV) peuvent aussi être préparés selon le schéma 5 qui suit.

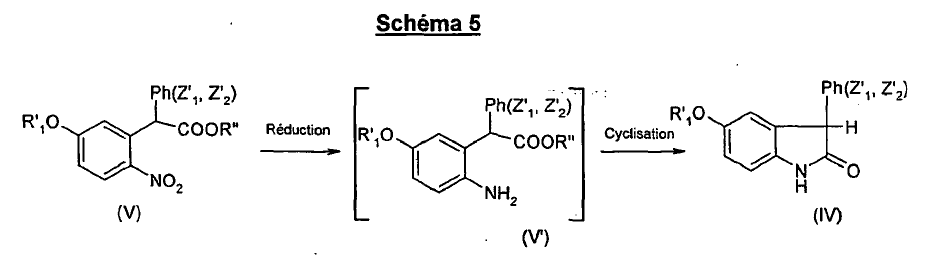

Selon le schéma 5, on peut également obtenir les composés de formule (IV) - dans laquelle R'₁, Z'₁ et Z'₂ sont tels que définis pour le composé de formule (III) - par cyclisation de l'amine (V') - dans laquelle R'₁, Z'₁ et Z'₂ sont tels que définis pour le composé de formule (III) et R" représente un (C₁-C₃)alkyle - générée in situ lors de la réduction du groupe nitro porté par le composé de formule (V) - dans laquelle R'₁, Z'₁ et Z'₂ sont tels que définis pour le composé de formule (III) et R" représente un groupe (C₁-C₃)alkyle. On opère en présence d'un métal en milieu acide tel que l'étain ou le fer en milieu acide tel que l'acide acétique à des températures comprises entre 30 et 100°C.

On peut préparer les composés de formule (V), selon le schéma 6 qui suit.

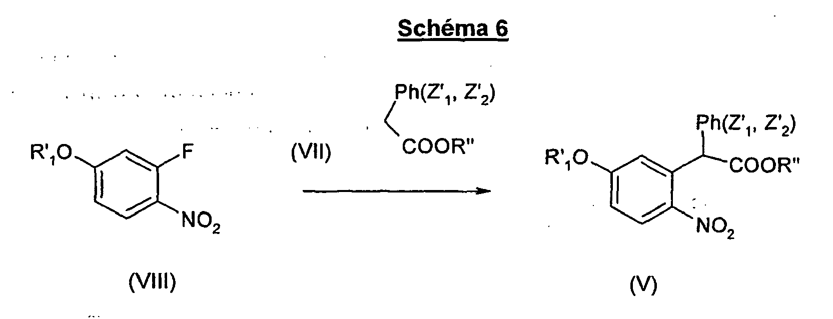

Selon le schéma 6, les composés de formule (V) - dans lesquels R'₁, Z'₁ et Z'₂ sont tels que définis pour le composé de formule générale (III) et R" représente un (C₁-C₃)alkyle - peuvent être obtenus par une réaction de substitution nucléophile aromatique d'un composé ortho-halogéno nitré de formule (VIII) - dans laquelle R'₁ est tel que défini pour le composé de formule (III) - de préférence ortho fluoré et d'un anion d'un dérivé de formule (VII) - dans laquelle Z'₁ et Z'₂ sont tels que définis pour le composé de formule (III) et R" représente un groupe (C₁-C₃)alkyle - préparé par action d'une base forte telle qu'un alcoolate alcalin, le tertio-butylate de potassium ou hydrure de sodium par exemple dans un solvant anhydre tel que la DMF.

On peut préparer les composés de formule (VI), selon le schéma 7 qui suit.

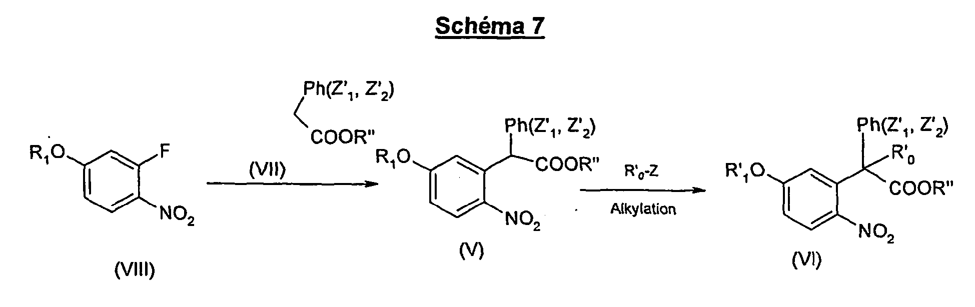

Selon le schéma 7, on obtient les composés de formule (VI) - dans laquelle R'₁, Z'₁, Z'₂, R'₀ sont tels que définis pour le composé de formule (III) et R" représente un groupe (C₁-C₃)alkyle - par alkylation du composé de formule (V) tel que défini précédemment au schéma 6, en présence d'une base telle que l'hydrure de sodium, avec par exemple un dérivé R'₀-Z dans lequel R'₀ est tel que défini pour le composé de formule (III) et Z représente un groupe partant tel qu'un iode ou un brome ou bien un ester d'acide sulfonique tel que mésylate ou tosylate, dans un solvant anhydre tel que la DMF ou le THF à des températures comprises entre -40°C et 20°C.

On préfère ne pas isoler les composés de formule (V) et les soumettre in situ aux dérivés R'₀-Z tel que défini précédemment pour obtenir les composés de formule (VI).

Les composés de formule (VII) et (VIII) sont commerciaux, connus ou préparés selon des méthodes bien connues de l'homme du métier.

Les N-oxydes des composés comportant une amine sont préparés selon les méthodes connues de l'homme du métier par réaction de l'amine avec des peracides organiques tels que les acides peracétique, trifluoroperacétique, performique, perbenzoïques ou ses dérivés tel l'acide l'acide 3-chloroperbenzoïque, à des températures comprises entre 0°C et 90°C, de préférence à des températures inférieures à 50°C.

Dans les schémas généraux de synthèse 1 à 7, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les énantiomères purs des composés de l'invention peuvent être préparés à partir de précurseurs énantiomèriquement purs ou bien par chromatographie sur des phases chirales ou bien, lorsque les composés comportent des fonctions acides ou des amines par cristallisations sélectives de sels diasatéréoisomèriques obtenus par réaction des composés (I) avec, respectivement, des amines ou des acides chiraux.

Les préparations et exemples suivants décrivent la préparation de certains composés conformes à l'invention. Ces préparations et exemples ne sont pas limitatifs et ne font qu'illustrer la présente invention.

Dans les préparations et exemples ci-dessous :
- Les spectres de masse sont réalisés sur un spectromètre quadripolaire de type Platform LCZ (WATERS) ou de type ZQ 4000 (WATERS) en mode d'ionisation par électrospray positif ;
- Les spectres de RMN (résonnance magnétique nucléaire) sont réalisés sur un spectromètre à transformée de Fourier (BRUKER), à la température de 300°K (protons échangeables non enregistrés) ;
- s = singulet,
- m = multiplet,
- t = triplet,
- q = quadruplet
- DMSO-d₆ = diméthylsulfoxyde deutéré
- CDCl3 = chloroforme deutéré ;
- PF = Point de Fusion (en degré Celsius) mesuré sur banc Kofler ;
- Eb = point d'ébullition (en degré Celsius)
- Les pouvoirs rotatoires [α]D/20 sont mesurés sur un Polarimètre PERKIN-ELMER 241 dans des conditions standards, à la température de 20°C, la concentration c est exprimée en g de soluté pour 100ml de solution ;

Les mélanges de solvants sont quantifiés en rapports volumètriques ;

Les microanalyses et spectres RMN confirment les structures des composés obtenus selon les exemples ci-dessous.

### PREPARATIONS (Composés de formule (III))

### PREPARATION 1:

### 3-(2-Chloro-phényl)-5-éthoxy-3-méthyl-1,3-dihydro-indol-2-one

### A) Préparation du 2-fluoro-4-éthoxy-nitrobenzène

A la solution de 100g de 3-fluoro-4-nitrophénol dans 2,5L d'acétonitrile, on ajoute 229g de carbonate de césium puis 65mL d'iodoéthane lentement. Après 24h d'agitation à 50°C, on traite avec 2,4L d'eau et 2,4L acétate d'éthyle. Après décantation, la phase aqueuse est réextraite par 1L d'acétate d'éthyle. Les phases organiques jointes sont séchées sur sulfate de sodium, concentrées sous pression réduite. Le résidu est repris par du pentane, filtré, séché. On obtient le produit attendu sous la forme de poudre. PF = 48°C

### B) 2-(2-Chloro-phényl)-2-(5-éthoxy-2-nitro-phényl)-propionate de méthyle

A 10,5g d'hydure de sodium à 60% dans l'huile en suspension dans 170mL de diméthylformamide, on additionne à 0°C, goutte à goutte, le mélange de 16,15 g de 2-fluoro-4-éthoxy-nitrobenzène et 16,2 g de 2-chlorophényl acétate de méthyle en solution dans 200 mL de diméthylformamide. On agite le mélange réactionnel pendant 3 heures en laissant la température remonter à température ambiante. Le mélange réactionnel est refroidi à 0°C puis on ajoute 16,40 mL de iodométhane. Le mélange réactionnel est agité à température ambiante la nuit. On ajoute 17 mL de méthanol et 500 mL d'une solution aqueuse saturée d'hydrogénocarbonate de sodium, et extrait avec 1000 mL l'acétate d'éthyle. On lave la phase organique trois fois avec 500 mL d'une solution aqueuse saturée d'hydrogénocarbonate de sodium, puis trois fois avec 500 mL d'eau salée. On sèche la phase organique sur sulfate de sodium, puis on évapore les solvants sous pression réduite. Le résidu ainsi obtenu est repris avec 50 mL de 2-propanol, agité la nuit, filtré, rincé au pentane et séché à 50°C sous vide. PF = 89°C

### C) 3-(2-Chloro-phényl)-5-éthoxy-3-méthyl-1,3-dihydro-indol-2-one

A 25,9g de 2-(2-Chloro-phényl)-2-(5-éthoxy-2-nitro-phényl)-propionate de méthyle en suspension dans 260 mL d'ethanol on ajoute 19,9 g de fer puis 28,5 mL d'acide acétique. On chauffe le mélange réactionnel sous agitation à reflux pendant 2 heures. On évapore partiellement les solvants sous pression réduite puis ajoute 250 mL d'une solution aqueuse saturée d'hydrogénocarbonate de sodium et 600 mL d'acétate d'éthyle. Le mélange réactionnel est agité 1 heure à température ambiante, puis filtré et rincé avec l'acétate d'éthyle. Après décantation, la phase organique est lavée avec 200 mL d'une solution aqueuse saturée d'hydrogénocarbonate, puis avec 200 mL d'eau salée. La phase organique est séchée sur sulfate de sodium et concentrée sous pression réduite. Le résidu ainsi obtenu est repris avec du pentane, filtré, séché à 50°C sous pression réduite.
PF = 140°C
RMN ¹H 250MHz (DMSO-d₆) : 1,21(t,3H) ; 1,65(s,3H); 3,88(q,2H); 6,34(s,1H); 6,71-6,82(m,2H); 7,3-7,5(m,3H); 7,75-7,8(m,1H)

### D) Résolution énantiomérique

La résolution énantiomèrique du composé précédent préparé en C) est effectuée par chromatographie supercritique sur phase chirale dans les conditions suivantes :
Appareillage : Système de chromatographie supercritique Berger Prep SFC avec logiciel Pronto.
Colonne chirale : CHIRALPAK AD-H 5µm, Longueur : 25 cm, diamètre : 21 mm
Phase mobile : CO₂/ Méthanol
Débit : 50 mL/min
Pression : 100 bar
Détection UV : 220 nm
On obtient ainsi le 3-(2-Chloro-phényl)-5-éthoxy-3-méthyl-1,3-dihydro-indol-2-one lévogyre :
PF= 80°C
[α]D/20= -7,1° (c=0,5 dans AcOEt) ainsi que son énantiomère dextrogyre.

### PREPARATION 2:

### Acide 4-Chloro-3-(5-éthoxy-3-méthyl-2-oxo-2,3-dihydro-1H-indol-3-yl)-benzoique

### A) 4-Chloro-3-(1-méthoxycarbonyl-éthyl)-benzoate de méthyle :

### A1) 4-chloro-3-méthylbenzoate de méthyle

A une solution de 25g d'acide 4-chloro-3-méthylbenzoïque dans 550mL de diméhylformamide, on ajoute 50g de carbonate de césium et 10,95mL d'iodure de méthyle gouttes à gouttes. On agite le milieu réactionnel à température ambiante pendant 16h, puis on additionne 800mL d'une solution aqueuse d'hydrogénocarbonate de sodium et on extrait avec de l'acétate d'éthyle. Après décantation, la phase aqueuse est réextraite avec 400mL d'acétate d'éthyle. Les phases organiques jointes sont lavées avec 250mL d'une solution aqueuse d'hydrogénocarbonate de sodium puis avec 250mL d'une solution aqueuse de chlorure de sodium. La phase organique obtenue est séchée sur sulfate de sodium et concentrée sous pression réduite. Le produit désiré est purifié par distillation sous pression réduite, on obtient une huile.
Eb = 78°C / 0,08mbar

### A2) 3-bromométhyl-4-chlorobenzoate de méthyle

A une solution de 25,26g 4-chloro-3-méthylbenzoate de méthyle dans 20mL de tétrachlorure de carbone, on additionne 24,85g de N-bromosuccinimide. On agite le milieu réactionnel à reflux pendant 6h, puis on additionne 500mL de dichlorométhane et 300mL d'une solution aqueuse de carbonate de potassium à 25°C. Après décantation, la phase aqueuse est réextraite avec 200mL de dichlorométhane. Les phases organiques jointes sont lavées avec 300mL d'une solution aqueuse de carbonate de potassium, séchées sur sulfate de sodium et concentrées sous pression réduite. Le produit désiré qui est purifié par distillation sous pression réduite, cristallise à température ambiante.
PF=93°C

### A3) 4-chloro-3-cyanométhylbenzoate de méthyle

A une solution de 25,52g de 3-bromométhyl-4-chlorobenzoate de méthyle dans 170mL de 1,4-dioxane, on additionne une solution 7,12g de cyanure de sodium dans 100mL d'eau à 10°C.

Le milieu réactionnel est agité à température ambiante pendant 16h, puis on additionne 500mL d'une solution aqueuse de carbonate de potassium et 600mL d'acétate d'éthyle. Après décantation la phase aqueuse est réextraite avec 250mL d'acétate d'éthyle. Les phases organiques jointes sont lavées avec 300mL d'une solution aqueuse de carbonate de potassium, séchées sur sulfate de sodium et concentrées sous pression réduite.
On obtient le produit attendu sous la forme de poudre.
PF=87°C

### A4) 4-Chloro-3-(1-méthoxycarbonyt-éthyl)-benzoate de méthyle :

A une solution de 19,68g de 4-chloro-3-cyanomethylbenzoate de méthyle dans 207mL de méthanol, on additionne de l'acide chlorhydrique anhydre gazeux pendant 3h à 0°C.

Le milieu réactionnel est agité pendant 16h à température ambiante, puis concentrée sous pression réduite. Le résidu est repris dans 600mL d'acétate d'éthyle et 500mL d'eau. La phase organique est lavée avec 200mL d'une solution aqueuse de chlorure de sodium, séchée sur sulfate de sodium et concentrée sous pression réduite.

On obtient le produit attendu sous la forme de cristaux.
PF = 55°C

### B) 4-Chloro-3-[1-(5-éthoxy-2-nitro-phényl)-1-méthoxycarbonyl-éthyl]-benzoate de méthyle

A 19,76g d'une dispersion d'hydrure de sodium à 60% dans l'huile, dans 300mL de diméthylformamide, on additionne, à -5°C un mélange de 30,5g de 2-fluoro-4-éthoxy-nitrobenzène (Préparation 1A) et de 39,97g de 4-chloro-3-(1-méthoxycarbonyl-éthyl)-benzoate de méthyle solubilisé dans 200mL de diméthylformamide. On agite le milieu réactionnel à température ambiante pendant 2h30, puis on additionne 30,7mL d'iodure de méthyle, à 10°C. Le milieu réactionnel est agité à température ambiante pendant 16h puis on additionne 30mL de méthanol à 10°C, ainsi que une solution aqueuse diluée d'hydrogénocarbonate de sodium. On extrait à l'acétate d'éthyle, sèche la phase organique sur sulfate de sodium et évapore les solvants sous pression réduite.
On obtient le produit attendu sous la forme de poudre.
PF = 141°C

### C) 4-Chloro-3-(5-éthoxy-3-méthyl-2-oxo-2,3-dihydro-1H-indol-3-yl)-benzoate de méthyle

A la solution de 4-chloro-3-[1-(5-éthoxy-2-nitro-phényl)-1-méthoxycarbonyl-éthyl]-benzoate de méthyle dans 650mL d'éthanol, on ajoute 41,49g de fer et 59,49mL d'acide acétique. Apres 5h d'agitation à reflux, le milieu est concentré sous pression réduite, on ajoute 700mL d'une solution aqueuse diluée d'hydrogénocarbonate de sodium et on extrait avec 1400mL d'acétate d'éthyle. Le milieu réactionnel est agité pendant 1 heure à température ambiante, puis filtré et rincé avec de l'acétate d'éthyle.

Après décantation, la phase organique est lavée avec 600mL d'une solution aqueuse diluée d'hydrogénocarbonate de sodium, puis avec 600mL d'une solution de chlorure de sodium. La phase organique est séchée sur sulfate de sodium et concentrée sous pression réduite. Le résidu est repris avec du pentane, filtré, séché.
On obtient le produit attendu sous la forme de poudre.
PF = 176°C
RMN ¹H 250MHz (DMSO-d₆) : 1,2(t,3H) ; 1,7(s,3H) ; 3,79-3,88(m,5H) ; 6,4/6,43(m,1H) ; 6,71-6,82(m,2H) ; 7,5-7,55(m,1H) ; 7,88-7,97(m,1H) ; 8,25-8,9(m,1H).

### D) Acide 4-Chloro-3-(5-éthoxy-3-méthyl-2-oxo-2,3-dihydro-1H-indol-3-yl)-benzoïque

A la solution de 18g 4-Chloro-3-(5-éthoxy-3-méthyl-2-oxo-2,3-dihydro-1*H*-indol-3-yl)-benzoate de méthyle dans 90mL de méthanol et 180mL de dioxanne, on ajoute 90mL d'une solution d'hydroxyde de sodium 2N. Après 16h d'agitation à température ambiante, le milieu est concentré sous pression réduite.

Le milieu réactionnel est refroidi à une température d'environ 0°C. On ajoute 500mL d'eau et 180mL d'une solution aqueuse d'acide chlorhydrique 1N. Le précipité est filtré puis rincé avec de l'eau. Le résidu obtenu est séché sous vide.
On obtient le produit attendu sous la forme de poudre.
PF=234°C

### E) Résolution énantiomérique

La résolution énantiomèrique de l'acide précédent est réalisée par chromatographie supercritique sur phase chirale dans les conditions suivantes :
Appareillage : Système de chromatographie supercritique Berger Prep SFC avec logiciel Pronto
Colonne chirale : CHIRALPAK AD-H 5µm, Longueur : 25 cm, diamètre : 21 mm
Phase mobile : CO₂ / Méthanol (60 % / 40 %)
Débit : 50 mL/min
Pression : 100 bar
Détection UV : 220 nm
On obtient ainsi l'acide 4-Chloro-3-(5-éthoxy-3-méthyl-2-oxo-2,3-dihydro-1*H*-indol-3-yl)-benzoïque dextrogyre :
PF= 236°C
RMN¹H 400MHz (DMSO-d₆) : 1,22(t,3H) ; 1,69(s,3H) ; 3,8-3,9(m,2H) ; 6,38-6,42(m,1H) ; 6,71-6,82(m,2H) ; 7,45-7,51 (m, 1H) ; 7,85-7,92(m,1H) ; 8,28(s,1H)
[α]D/20= + 125 ° (c=1, dans Acétate d'éthyle) ainsi que son énantiomère lévogyre.

### PREPARATION 3:

### 3-(5-Amino-2-chloro-phényl)-5-éthoxy-3-méthyl-1,3-dihydro-indol-2-one

### A) (2-Chloro-5-nitro-phényl)-acétate de méthyle

A la solution de 28g de (2-chloro-5-nitrophényl)-acétonitrile [décrit par Lisitsyn, V.N.; Lugovskaya dans J.Org.Chem.USSR (Engl.Transl.); EN; 10; 1974; 92-95] dans 300mL de méthanol, refroidie à 0°C, on dissout du chlorure d'hydrogène, par un tube plongeant diffuseur, jusqu'à saturation à 0°C. On maintient l'agitation sous léger courant d'azote à 20°C pendant 15h. Après concentration sous pression réduite, on reprend par 500mL d'acétate d'éthyle et 500mL d'un mélange d'eau et de glace. La phase organique est séchée sur sulfate de sodium, concentrée sous pression réduite. Le résidu est purifié par chromatographie sur une colonne de 400g de silice en éluant avec un mélange cyclohexane/acétate d'éthyle. On obtient le produit attendu sous la forme liquide.
RMN¹H 250MHz (CDCl3) : 3,79 (s, 3H) ;3,92 (s, 2H) ; 7,61 (d, 1H) ; 8,1-8,3 (m, 2H).

### B) 2-(2-Nitro-5-éthoxy-phényl)-2-(2-chloro-5-nitro-phényl)-propionate de méthyle

A la suspension de 12,4g d'hydrure de sodium dans 200mL de diméthylformamide, refroidie à -10°C et sous atmosphère d'azote, on ajoute goutte à goutte la solution de 18,3g de (2-Chloro-5-nitro-phényl)-acétate de méthyle et 14,76g de 2-fluoro-4-éthoxy-nitrobenzène (Préparation 1A) dans 160mL de diméthylformamide. On agite en laissant remonter la température à 20°C pendant 4h. A 0°C on ajoute, goutte à goutte, 15mL d'iodure de méthyle puis on laisse agiter 16h à 20°C. On traite, à 10°C, par 20mL de méthanol, 1,5L de solution aqueuse de bicarbonate de sodium et 1,5L d'acétate d'éthyle. Après décantation, la phase organique est lavée 2 fois par 1.5L de solution aqueuse de bicarbonate de sodium, séchée sur sulfate de sodium, concentrée sous pression réduite. Le résidu est cristallisé dans du toluène, filtré, séché. On obtient le produit attendu sous la forme de poudre.
PF = 195°C

### C) 3-(5-Amino-2-chloro-phényl)-5-éthoxy-3-méthyl-1,3-dihydro-indol-2-one

A la solution de 32,6g de 2-(2-amino-5-éthoxy-phényl)-2-(2-chloro-5-nitro-phényl)-propionate de méthyle dans 900mL de méthanol et 70mL d'acide acétique , on ajoute 33g de fer. Après 3h d'agitation à reflux et une nuit à 20°C, le milieu est concentré sous pression réduite. On ajoute 1 L d'une solution aqueuse diluée d'hydrogénocarbonate de sodium et on extrait avec 0,8L d'acétate d'éthyle. Le milieu réactionnel est agité pendant 1 heure à 20°C, puis filtré et rincé avec de l'acétate d'éthyle. Après décantation, la phase organique est lavée avec 0,5L d'une solution aqueuse diluée d'hydrogénocarbonate de sodium, puis avec 0,5L d'une solution de chlorure de sodium. La phase organique est séchée sur sulfate de sodium et concentrée sous pression réduite. Le résidu est cristallisé dans un mélange toluène/2-propanol, filtré, séché.

On obtient le produit attendu sous la forme d'une poudre beige.
PF = 166°C
RMN ¹H 250MHz (DMSO-d₆) : 1,23(t,3H) ; 1,54(s,3H) ; 3,88(q,2H) ; 6,31-6,38(m,1H) ; 6,48-6,51(m,1H) ; 6,89-6,99(m,2H) ; 7,15-7,3(m,2H)

### D) Résolution énantiomérique

La résolution énantiomèrique du composé précédent préparé en C) est réalisée par chromatographie supercritique sur phase chirale dans les conditions suivantes :
Appareillage : Système de chromatographie supercritique Berger Prep SFC avec logiciel Pronto
Colonne chirale : CHIRALPAK AD-H 5µm, Longueur : 25 cm, diamètre : 21 mm
Phase mobile : CO₂ / Méthanol (60 % / 40 %)
Débit : 50 mL/min
Pression : 100 bar
Détection UV : 220 nm
On obtient ainsi le 3-(5-amino-2-chloro-phényl)-5-éthoxy-3-méthyl-1,3-dihydro-indol-2-one lévogyre :
PF= 172°C
[α]D/20= -9,7° (c= 1 dans MeOH)] ainsi que son énantiomère dextrogyre.

### PREPARATION 4:

### 4-Chloro-3-(5-éthoxy-3-méthyl-2-oxo-2,3-dihydro-1H-indol-3-yl)-benzaldéhyde

### A) Préparation du 4-Chloro-3-(5-éthoxy-3-méthyl-2-oxo-2,3-dihydro-1H-indol-3-yl)-benzoate de méthyle lévogyre

On dissout 4g d'acide 4-Chloro-3-(5-éthoxy-3-méthyl-2-oxo-2,3-dihydro-1*H*-indol-3-yl)-benzoïque lévogyre obtenus dans la préparation 2, dans 50mL de DMF. On ajoute 2,35g de carbonate de potassium puis 1,08mL d'iodométhane. Après une nuit d'agitation à 20°C, on verse sur un mélange d'eau et de glace et on extrait avec de l'acétate d'éthyle. La phase organique est séchée sur sulfate de sodium, évaporée à sec puis on purifie par chromatographie sur silice pour obtenir le composé désiré sous forme de poudre.
PF = 84°C
[α]D/20=-91,9° (c=1 dans MeOH)
RMN ¹H 250MHz (DMSO-d₆): 1,21(t, 3H); 1,69(s, 3H); 3,79-3,81(m, 2H); 3,92(s, 3H); 6,43(s, 1H); 6,71-6,81 (m, 2H); 7,49-7,54(m, 1H); 7,89-7,92(m, 1H); 8,28(s, 1H)

### B) Préparation du 3-(2-Chloro-5-hydroxyméthyl-phényl)-5-éthoxy-3-méthyl-1,3-dihydro-indol-2-one

On dissout 2g de l'ester précédent dans 40mL de dichlorométhane. Après refroidissement à -70°C, on ajoute lentement 7,48mL d'hydrure de diisobutylaluminium 1,5M dans du toluène. Après 2h d'agitation à 20°C, on rajoute 3,7mL d'hydrure de diisobutylaluminium à -70°C puis laisse agiter une nuit à 20°C. L'évaporation partielle du solvant est réalisée sous pression réduite. On reprend par 175mL d'acide chlorhydrique 1N, puis on extrait avec de l'acétate d'éthyle. La phase organique est séchée sur sulfate de sodium, évaporée à sec puis on purifie par chromatographie sur silice pour obtenir le composé désiré sous forme de poudre.
Spectomètrie de masse : MS[(+)ESI,m/z]:331 (MH+)
RMN ¹H 250MHz (DMSO-d₆) : 1,2(t,3H) ; 1,62(s,3H) ; 3,85(q,2H) ; 4,9(d,2H) ; 6,31-6,36(m,1H) ; 6,7-6,81 (m,2H) ; 7,28-7,32(m,2H) ; 7,69-7,71 (m,1H)
MS[(+)ESI,m/z]:332(MH+)

### C) Préparation du 4-Chloro-3-(5-éthoxy-3-méthyl-2-oxo-2,3-dihydro-1H-indol-3-yl)-benzaldéhyde

A 1,67g de l'alcool précédent dissout dans 22mL de dichlorométhane on ajoute 1,89g de dichromate de pyridinium; à 0°C Après une nuit d'agitation à 20°C, on filtre sur célite, rince avec 50mL de dichlorométhane, évapore le filtrat puis purifie par chromatographie sur silice pour obtenir le composé désiré sous forme de poudre.
RMN ¹H 250MHz (DMSO-d₆): 1,2(t,3H); 1,74(s,3H); 3,85(q,2H); 6,4-6,48(m,1H); 6,7-6,86(m,2H); 7,58-7.,65(m,1H) ; 7,85-7,95(m,1H); 8,3(m,1H); 10,1(s,1H)
MS[(+)ESI,m/z]:330(MH+)

### PREPARATION 5

### 3-(2-fluoro-phényl)-5-éthoxy-3-méthyl-1,3-dihydro-indol-2-one

### A) 2-(2-Fluoro-phényl)-2-(5-éthoxy-2-nitro-phényl)-propionate de méthyle

On obtient le 2-(2-fluoro-phényl)-2-(5-éthoxy-2-nitro-phényl)-propionate de méthyle de la même manière que pour la préparation 1B à partir du 2-fluorophénylacétate de méthyle.
RMN ¹H 250MHz (DMSO) : 1,28(t,3H); 2,07(s,3H); 3,57(s,3H); 3,95-4,1(m,2H); 6,55-6,58(m,1H); 7,07-7,31(m,3H); 7,4-7,49(m,1H); 7,56-7,64(m,1H); 7,97-8,02(m,1H)

### B) 3-(2-fluoro-phényl)-5-éthoxy-3-méthyl-1,3-dihydro-indol-2-one

On obtient le 3-(2-fluoro-phényl)-5-éthoxy-3-méthyl-1,3-dihydro-indol-2-one de la même manière que pour la préparation 1C à partir du composé précédent.
RMN ¹H 250MHz (DMSO-d₆): 1,23(t,3H); 1,64(s,3H); 3,8-3,92(m,2H); 6,48-6,51(m,1H) ; 6,71-6,82(m,2H) ; 7,01-7,11(m,1H) ; 7,26-7,41(m,2H) ; 7,61-7,7(m,1H)

### C) Résolution énantiomérique

La résolution énantiomérique est réalisée de la même manière que pour la préparation 1D, à partir du composé précédent.

On obtient ainsi le 3-(2-fluoro-phényl)-5-éthoxy-3-méthyl-1,3-dihydro-indol-2-one dextrogyre :
[α]D/20= +9,6° (c=1 dans AcOEt) ainsi que son énantiomère lévogyre.

### PREPARATION 6 :

### 3-phényl-5-éthoxy-3-méthyl-1,3-dihydro-indol-2-one

### A) 2-phényl-2-(5-éthoxy-2-nitro-phényl)-propionate de méthyle

On obtient le 2-phényl-2-(5-éthoxy-2-nitro-phényl)-propionate de méthyle de la même manière que pour la préparation 1 B à partir du phénylacétate de méthyle.
RMN¹H 250MHz (DMSO-d₆) : 1,24(t,3H); 2,07(s,3H); 3,53(s,3H); 3,92-4,1(m,2H); 6,26-6,29(m,1H); 7,03-7,09(m,1H); 7,35-7,7,48(m,5H); 8,03-8,08(m,1H)

### B) 3-phényl-5-éthoxy-3-méthyl-1,3-dihydro-indol-2-one

On obtient le 3-phényl-5-éthoxy-3-méthyl-1,3-dihydro-indol-2-one de la même manière que pour la préparation 1C à partir du composé précédent.
RMN¹H 250MHz (DMSO-d₆): 1,27(t,3H) ; 1,66(s,3H) ; 3,87-3,98(m,2H) ; 6,75-6,87(m,3H) ; 7,20-7,36(m,5H)

### C) 3-phényl-5-éthoxy-3-méthyl-1,3-dihydro-indol-2-one isomère lévogyre et isomère dextrogyre obtenus par résolution énantiomérique

La résolution énantiomérique est réalisée de la même manière que pour la préparation 1 D, à partir du composé précédent.

On obtient ainsi le 3-phényl-5-éthoxy-3-méthyl-1,3-dihydro-indol-2-one :
- Dextrogyre
   [α]D/20= +98° (c=1 dans MeOH)
   RMN¹H 250MHz (DMSO-d₆): 1,28(t,3H) ; 1,66(s,3H) ; 3,88-3,98(m,2H) ; 6,75-6,87(m,3H) ; 7,21-7,37(m,5H)
- Lévogyre
   [α]D/20= -110° (c=1 dans MeOH)

Dans des conditions semblables aux étapes A, B et C décrites précédemment, on obtient les composés des préparations rassemblés dans le tableau I suivants, en partant du 2-fluoro-4-éthoxy-nitrobenzène (Préparation 1A) et des phényl-acétates de méthyle ou d'éthyle diversement substitués, commerciaux ou décrits, appropriés.

**Tableau I**

| | | | | | | | |
|---|---|---|---|---|---|---|---|
| 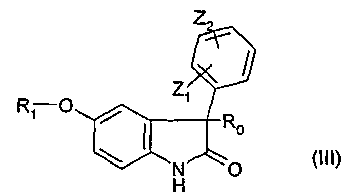 | | | | | | | |

| Préparation | R₀ | R₁ | Z₁ | Z₂ | [α]D/20 | PF (°C) | MH+ |
|---|---|---|---|---|---|---|---|
| 7 dextrogyre | Me | Et | 3-OMe | H | +154°(c=1, AcOEt) | 109 | 298 |
| 7 lévogyre | Me | Et | 3-OMe | H | -163°(c=1, AcOEt) | 101 | 298 |
| 8 dextrogyre | Me | Et | 2-OMe | H | +26° (c=1, AcOEt) | 152 | 298 |
| 8 lévogyre | Me | Et | 2-OMe | H | -24° (c=1, AcOEt) | 152 | 298 |
| 9 dextrogyre | Me | Et | 4-Me | H | +189° (c=1, AcOEt) | 104 | 282 |
| 9 lévogyre | Me | Et | 4-Me | H | -186° (c=1, AcOEt) | 104 | 282 |
| 10 dextrogyre | Me | Et | 3-Me | H | +163°(c=1, AcOEt) | 108 | 282 |
| 10 lévogyre | Me | Et | 3-Me | H | -163°(c=1, AcOEt) | 107 | 282 |
| 11 dextrogyre | Me | Et | 2-OCF3 | H | +49° (c=1, AcOEt) | 120 | 352 |
| 11 lévogyre | Me | Et | 2-OCF3 | H | -49° (c=1, AcOEt) | 120 | 352 |
| 12 dextrogyre | Me | Et | 2-CF3 | H | +432° (c=1, AcOEt) | 152 | 336 |
| 12 lévogyre | Me | Et | 2-CF3 | H | -419° (c=1, AcOEt) | 181 | 336 |
| 13 dextrogyre | Me | Et | 2-Cl | 3-Cl | +51 ° (c=1, AcOEt) | 192 | 336 |
| 13 lévogyre | Me | Et | 2-Cl | 3-Cl | -51° (c=1, AcOEt) | 191 | 336 |
| 14 dextrogyre | Me | Et | 2-Cl | 6-F | +75° (c=1, AcOEt) | 139 | 320 |
| 14 lévogyre | Me | Et | 2-Cl | 6-F | -80° (c=1, AcOEt) | 139 | 320 |
| 15 dextrogyre | Me | Et | 2-Cl | 5-OMe | +43 (c=1, MeOH) | 87 | 332 |
| 15 lévogyre | Me | Et | 2-Cl | 5-OMe | -71 (c=1, MeOH) | 85 | 332 |
| 16 dextrogyre | Me | Et | 2-OBn | H | +66° (c=1, AcOEt) | 100 | 374 |
| 16 lévogyre | Me | Et | 2-OBn | H | -58° (c=1, AcOEt) | 92 | 374 |
| 17 dextrogyre | Me | Et | 4-OBn | H | +161° (c=1, AcOEt) | 147 | 374 |
| 17 lévogyre | Me | Et | 4-OBn | H | -164° (c=1, AcOEt) | 148 | 374 |

### PREPARATION 18:

### 3-(2-Chloro-phényl)-5-éthoxy-3-éthyl-1,3-dihydro-indol-2-one

### A) 2-(2-Chloro-phényl)-2-(5-éthoxy-2-nitro-phényl)-butyrate de méthyle

A 7,07g d'hydure de sodium à 60% dans l'huile en suspension dans 160mL de diméthylformamide, on additionne à 0°C, goutte à goutte, le mélange de 10 g de 2-fluoro-4-éthoxy-nitrobenzène préparé en A de la préparation 1 et 11,96 g de 2-chlorophényl acétate de méthyle en solution dans 60 mL de diméthylformamide. On agite le mélange réactionnel pendant 3 heures en laissant la température remonter à température ambiante. Le mélange réactionnel est refroidi à 0°C puis on ajoute 33.7 g de iodoéthane. Le mélange réactionnel est agité à température ambiante la nuit. On ajoute 17 mL de méthanol et 500 mL d'une solution aqueuse saturée d'hydrogénocarbonate de sodium, et extrait avec 1000 mL l'acétate d'éthyle. On lave la phase organique trois fois avec 500 mL d'une solution aqueuse saturée d'hydrogénocarbonate de sodium, puis trois fois avec 500 mL d'eau salée. On sèche la phase organique sur sulfate de sodium, puis on évapore les solvants sous pression réduite. Le résidu ainsi obtenu est repris avec 50 mL de 2-propanol, agité la nuit, filtré, rincé au pentane et séché à 50°C sous vide.
PF = 95°C

### B) 3-(2-Chloro-phényl)-5-éthoxy-3-éthyl-1,3-dihydro-indol-2-one

A 19,6g de 2-(2-Chloro-phényl)-2-(5-éthoxy-2-nitro-phényl)-butyrate de méthyle en suspension dans 208 mL de méthanol on ajoute 14,5 g de fer puis 21,03 mL d'acide acétique. On chauffe le mélange réactionnel sous agitation à reflux pendant 2 heures. On évapore partiellement les solvants sous pression réduite puis ajoute 250 mL d'une solution aqueuse saturée d'hydrogénocarbonate de sodium et 600 mL d'acétate d'éthyle. Le mélange réactionnel est agité 1 heure à température ambiante, puis filtré et rincé avec l'acétate d'éthyle. Après décantation, la phase organique est lavée avec 200 mL d'une solution aqueuse saturée d'hydrogénocarbonate, puis avec 200 mL d'eau salée. La phase organique est séchée sur sulfate de sodium et concentrée sous pression réduite. Le résidu ainsi obtenu est repris avec du pentane, filtré, séché à 50°C sous pression réduite.
PF = 157°C

### C) Résolution énantiomérique

La résolution énantiomèrique du 3-(2-Chloro-phényl)-5-éthoxy-3-éthyl-1,3-dihydro-indol-2-one est effectuée par chromatographie supercritique sur phase chirale dans les conditions suivantes :
Appareillage : Système de chromatographie supercritique Berger Prep SFC avec logiciel Pronto.
Colonne chirale : CHIRALPAK AD-H 5µm, Longueur : 25 cm, diamètre : 21 mm
Phase mobile : CO₂ / Méthanol
Débit : 50 mUmin
Pression: 100 bar
Détection UV : 220 nm
On obtient ainsi le 3-(2-Chloro-phényl)-5-éthoxy-3-éthyl-1,3-dihydro-indol-2-one lévogyre :
PF= 89°C
[α]D/20= -34,6° (c=1 dans AcOEt) ainsi que son énantiomère dextrogyre.

### PREPARATION 19:

### 3-(2-Chloro-phényl)-5-éthoxy-3-propyl-1,3-dihydro-indol-2-one

Dans des conditions semblables à la préparation 18, en remplaçant l'iodoéthane par de l'iodopropane dans l'étape A, on obtient le 3-(2-Chloro-phényl)-5-éthoxy-3-propyl-1,3-dihydro-indol-2-one lévogyre :
PF= 136°C
[α]D/20= -43,1° (c=1 dans AcOEt) ainsi que son énantiomère dextrogyre.

### EXEMPLES

Dans les tableaux II à XI qui suivent, on entend par Me, un groupe méthyle, Et, un groupe éthyle, Pr un groupe n-propyle et Bn un groupe benzyle.

### EXEMPLE 1

### 3-(2-Chloro-phényl)-5-éthoxy-1-(2,4-diméthoxy-benzènesulfonyl)-3-méthyl-1,3-dihydro-indol-2-one

A la solution de 0,3g de 3-(2-chloro-phényl)-5-éthoxy-3-méthyl-1,3-dihydro-indol-2-one lévogyre (Préparation 1) dans 7mL de tétrahydrofuranne refroidie à -30°C, on ajoute 0,123g de ter-butylate de potassium. On laisse remonter la température à 0°C, on refroidit à -60°C puis ajoute 0,259g de chlorure de 2,4-diméthoxy-benzènesulfonyle. Après une nuit d'agitation à 20°C, on hydrolyse à l'eau et extrait avec de l'acétate d'éthyle. La phase organique est séchée sur sulfate de sodium, évaporée à sec. On reprend le résidu avec de l'éther isopropylique sous agitation, on filtre et sèche sous vide le produit attendu sous forme de poudre blanche.
PF = 197°C
RMN ¹H 250MHz (DMSO-d₆): 1,25(t,3H) ; 1,68(s,3H); 3,61(s,3H); 3,8-3,99(m,5H); 6,33(d,1H); 6,69-6,78(m,2H); 6,9-6,98(m,1H); 7,28-7,5(m,3H); 7,7-7,81(m,2H); 7,89-7,92(m, 1H)

### EXEMPLE 2

### N-tert-Butyl-4-[3-(2-chloro-phényl)-5-éthoxy-3-méthyl-2-oxo-2,3-dihydro-indole-1-sulfonyl]-benzamide

### A) Préparation du chlorure de 4-tert-butylcarbamoyl-benzènesulfonyle

### a) Chlorure de 4-chlorosulfonyl-benzoyle

A la solution de 25g d'acide 4-chlorosulfonyl-benzoïque dans 250mL de toluène, on ajoute 33mL de chlorure de sulfonyle. On chauffe à reflux pendant 5h. A la température de 40°C sous pression réduite, on évapore les solvants et reprend le résidu avec 100mL d'heptane. Par filtration et rinçage avec un peu d'heptane on obtient le produit désiré sous forme de poudre blanche.
RMN ¹H 250MHz (CDCl₃) : 8,21(m,2H) ; 8,41 (m,2H)

### b) Chlorure de 4-tert-Butylcarbamoyl-benzènesulfonyle

A la solution de 24,5g du composé précédent dans 250mL de dichlorométhane refroidie à 0°C, on ajoute très lentement 15g de *tert*-butylamine diluée dans 70mL de dichlorométhane. On filtre sur fritté, rince au dichlorométhane, sèche sous vide à 40°C pour obtenir le produit attendu sous forme d'une poudre blanche.
PF = 179°C
RMN ¹H 250MHz (CDCl₃) : 1,51(s,9H); 7,92-8(m,2H); 8,09-8,16(m,2H)

### B) N-tert-Butyl-4-[3-(2-chloro-phényl)-5-éthoxy-3-méthyl-2-oxo-2,3-dihydro-indole-1-sulfonyl]-benzamide

A la solution de 0,15g de 3-(2-Chloro-phényl)-5-éthoxy-3-méthyl-1,3-dihydro-indol-2-one lévogyre (préparation 1) dans 4mL de tétrahydrofuranne refroidie à -30°C, on ajoute 0,0614g de ter-butylate de potassium. On laisse remonter la température à 0°C, puis on refroidit à -60°C pour ajouter 0,15g de chlorure de 4-*tert*-Butylcarbamoyl-benzenesulfonyle. Après une nuit d'agitation à 20°C, on hydrolyse à l'eau et extrait avec de l'acétate d'éthyle. La phase organique est séchée sur sulfate de sodium, évaporée à sec et purifiée par chromatographie sur silice en éluant avec un mélange cyclohexane/acétate d'éthyle 6/4. On obtient le produit désiré sous forme d'une poudre blanche.
PF = 157°C
RMN ¹H 400MHz (DMSO-d₆): 1,22(t,3H); 1,38(s,9H); 1,71(s,3H); 3,84-3,94(m,2H); 6,92(m,1H); 7,28(m,1H); 7,35(m,1H); 7,45(m,1H); 7,75-7,81 (m,2H); 7,99-8,11(m,5H)

### EXEMPLE 3

### 4-[3-(2-Chloro-phényl)-5-éthoxy-3-méthyl-2-oxo-2,3-dihydro-indole-1-sulfonyl]-N-cyclopentyl-benzamide

### A) Préparation du chlorure de 4-cyclopentylcarbamoyl-benzènesulfonyle

A la solution de 1g de chlorure de 4-chlorosulfonyl-benzoyle (exemple 2Aa) dans 15mL de dichlorométhane refroidie à 0°C, on ajoute très lentement 0,83mL de cyclopentylamine. Après 3h d'agitation à 20°C, on lave à l'eau, sèche sur sulfate de sodium, évapore à sec, purifie par chromatographie sur silice en éluant avec un mélange cyclohexane / acétate d'éthyle dans les proportions 90/10, respectivement. On obtient le produit désiré sous forme d'une poudre blanche.
PF = 143°C
RMN ¹H 250MHz (CDCl3) : 1,47-1,64(m,2H) ; 1,69-1,89(m,4H) ; 2,08-2,26(m,2H) ; 4,38-4,54(m,1H) ; 7,95-8,03(m,2H) ; 8,1-8,18(m,2H)

### B) 4-[3-(2-Chloro-phényl)-5-éthoxy-3-méthyl-2-oxo-2,3-dihydro-indole-1-sulfonyl]-N-cyclopentyl-benzamide

On obtient le 4-[3-(2-Chloro-phényl)-5-éthoxy-3-méthyl-2-oxo-2,3-dihydro-indole-1-sulfonyl]-*N-*cyclopentyl-benzamide de manière analogue à l'exemple 2, à partir de 3-(2-chloro-phényl)-5-éthoxy-3-méthyl-1,3-dihydro-indol-2-one lévogyre (préparation 1) et du chlorure de sulfonyle préparé selon l'étape A de l'exemple 3.
PF=152°C
RMN ¹H 250MHz (DMSO-d₆): 1,29(t,3H) ; 1,5-2(m,11H) ; 3,85-3,98(m,2H) ; 4,18-4,3(m,1H) ; 6,91-6,99(m,1H); 7,22-7,28(m,1H); 7,3-7,49(m,2H) ; 7,75-7,85(m,2H) ; 8,05-8,18(m,4H) ; 8,57-8,63.(m,1H)

### EXEMPLE 4

### 4-[3-(2-Chloro-phényl)-5-éthoxy-3-méthyl-2-oxo-2,3-dihydro-indole-1-sulfonyl]-N-(1,1-diméthyl-propyl)-benzamide

### A) Préparation du chlorure de 4-(1,1-diméthyl-propyl)-carbamoyl-benzènesulfonyle

A la solution de 1g de chlorure de 4-chlorosulfonyl-benzoyle (exemple 2Aa) dans 15mL de dichlorométhane refroidie à 0°C, on ajoute très lentement 0,98mL de 1,1-diméthyl-propyl amine. Après 3h d'agitation à 20°C, on lave à l'eau, sèche sur sulfate de sodium, évapore à sec, purifie par chromatographie sur silice en éluant avec un mélange cyclohexane / acétate d'éthyle dans les proportions 90/10, respectivement. On obtient le produit désiré sous forme d'une poudre blanche.
PF = 126°C
RMN ¹H 250MHz (CDCl3) : 0,95(t,3H) ; 1,47(s,6H); 1,9(q,2H) ; 7,93-7,98(m,2H) ; 8,1-8,16(m,2H)

### B) 4-[3-(2-Chloro-phényl)-5-éthoxy-3-méthyl-2-oxo-2,3-dihydro-indole-1-sulfonyl]-N-(1,1-diméthyl-propyl)-benzamide

On obtient le 4-[3-(2-Chloro-phényl)-5-éthoxy-3-méthyl-2-oxo-2,3-dihydro-indole-1-sulfonyl]-*N-*(1,1-diméthyl-propyl)-benzamide de manière analogue à l'exemple 2, à partir de 3-(2-chloro-phényl)-5-éthoxy-3-méthyl-1,3-dihydro-indol-2-one lévogyre (préparation 1) et du chlorure de sulfonyle préparé à l'étape A de l'exemple 4.
PF= 138°C
RMN ¹H 400MHz (DMSO-d₆): 0,82(t,3H) ;1,24(t,3H); 1,32(s,6H); 1,71(s,3H); 1,78(q,2H); 3,85-3,92(m,2H); 6,37(s,1H); 6,93-6,96(m,1H); 7,22-7,28(m,1H); 7,34-7,38(m,1H); 7,42-7,48(m,1H); 7,75-7,81 (m,2H); 7,98-8,01 (m,2H); 8,08-8,11 (m,2H)

### EXEMPLE 5

### N-tert-Butyl-4-[3-(2-chloro-phényi)-5-éthoxy-3-méthyl-2-oxo-2,3-dihydro-indole-1-sulfonyl]-3-méthoxybenzamide

On obtient le *N-tert*-Butyl-4-[3-(2-chloro-phényl)-5-éthoxy-3-méthyl-2-oxo-2,3-dihydro-indole-1-sulfonyl]-3-méthoxybenzamide de manière analogue à l'exemple 2, à partir de 3-(2-chloro-phényl)-5-éthoxy-3-méthyl-1,3-dihydro-indol-2-one lévogyre (préparation 1) et du chlorure de 4-*tert*-butylcarbamoyl-2-méthoxybenzènesulfonyle décrit dans le document WO97/1556 (Préparation 13, Réactif (2).2).
PF = 139°C
RMN ¹H 400MHz (DMSO-d₆): 1,25(t,3H); 1,39(s,9H); 1,69(s,3H); 3,7(s,3H); 3,85-3,96(m,2H); 6,91-6,98(m,1H); 7,28-7,54(m,5H); 7,69-7,71(m,1H); 7,74-7,8(m,1H); 8-8,03(m,2H)

### EXEMPLE 6

### N-tert-Butyl-4-[3-(2-fluoro-phényl)-5-éthoxy-3-méthyl-2-oxo-2,3-dihydro-indole-1-sulfonyl]-benzamide

On obtient le *N-tert*-Butyl-4-[3-(2-fluoro-phényl)-5-éthoxy-3-méthyl-2-oxo-2,3-dihydro-indole-1-sulfonyl]-benzamide de manière analogue à l'exemple 5 à partir de 3-(2-fluoro-phényl)-5-éthoxy-3-méthyl-1,3-dihydro-indol-2-one lévogyre (préparation 5).
PF = 116°C
RMN ¹H 400MHz (DMSO-d₆): 1,24(t,3H); 1,38(s,9H); 1,66(s,3H); 3,67(s,3H); 3,88-3,95(m,2H); 6,53(s,1H); 6,91-6,95(m,1H); 7,02-7,09(m,1H); 7,28-7,42(m,2H); 7,49-7,57(m,2H); 7,63-7,71 (m,2H); 8-8,04(m,1H)

### EXEMPLE 7

### 1-(4-Amino-2-méthoxy-benzènesulfonyl)-3-(2-chloro-phényl)-5-éthoxy-3-méthyl-1,3-dihydro-indol-2-one

### A) 3-(2-Chloro-phényl)-5-éthoxy-1-(2-méthoxy-4-nitro-benzènesulfonyl)-3-méthyl-1,3-dihydro-indol-2-one

A la solution de 0,6g de 3-(2-Chloro-phényl)-5-éthoxy-3-méthyl-1,3-dihydro-indol-2-one lévogyre (préparation 1) dans 15mL de tétrahydrofuranne refroidie à -30°C, on ajoute 0,245g de ter-butylate de potassium. On laisse remonter la température à 0°C puis on refroidit à -60°C pour ajouter 0,550g de chlorure de 4-nitro-2-méthoxybenzènesulfonyle. Après une nuit d'agitation à 20°C, on traite à l'eau et on extrait avec de l'acétate d'éthyle. La phase organique est séchée sur sulfate de sodium, évaporée à sec pour donner le produit désiré sous forme d'une mousse jaune directement engagée dans l'étape suivante.

### B) 1-(4-Amino-2-méthoxy-benzènesulfonyl)-3-(2-chloro-phényl)-5-éthoxy-3-méthyl-1,3-dihydro-indol-2-one

On porte à reflux pendant 3h le mélange de 1,028g du composé précédent (obtenu à l'étape A de l'exemple 7), 0,56g de fer en poudre, 7mL de méthanol et 1 mL d'acide acétique, puis on évapore partiellement les solvants sous vide. Le résidu est repris avec une solution aqueuse d'hydrogènocarbonate de sodium, extrait avec de l'acétate d'éthyle, filtré sur talc et décanté. La phase organique est séchée sur sulfate de sodium, évaporée à sec. On reprend le résidu avec de l'éther isopropylique, filtre, sèche pour donner le produit désiré sous forme d'une poudre blanche.
PF=211°C
RMN ¹H 250MHz (DMSO-d₆): 1,25(t,3H) ; 1,66(s,3H); 3,5(s,3H); 3,81-3,97(m,2H); 6,15-6,21(m,2H); 6,29-6,35(m,1H); 6,88-6,91 (m,1H); 7,29-7,48(m,3H); 7,52-7,54(m,1H); 7,65-7,7(m,1H); 7,72-7,79(m,1H)

### EXEMPLE 8

### 3-{4-[3-(2-Chloro-phényl)-5-éthoxy-3-méthyl-2-oxo-2,3-dihydro-indole-1-sulfonyl]-3-méthoxy-phényl}-1,1-diéthyl-urée:

### A) {4-[3-(2-Chloro-phényl)-5-éthoxy-3-méthyl-2-oxo-2,3-dihydro-indole-1-sulfonyl]-3-méthoxy-phényl}-carbamate de phényle

A la solution de 0,53g du composé de l'exemple 7 dans 18mL de tétrahydrofuranne refroidie à 0°C, on ajoute 1,5mL d'une solution aqueuse de soude 2N puis lentement 0,48mL de chlorocarbonate de phényle. Après une nuit d'agitation à 20°C, on traite à l'eau et extrait avec de l'acétate d'éthyle. La phase organique est séchée sur sulfate de sodium, évaporée à sec, purifiée par chromatographie sur silice en éluant avec un mélange cyclohexane/acétate d'éthyle pour donner le produit désiré sous forme d'une mousse blanche directement engagée dans l'étape suivante.

### B) 3-{4-[3-(2-Chloro-phényl)-5-éthoxy-3-méthyl-2-oxo-2,3-dihydro-indole-1-sulfonyl]-3-méthoxy-phényl}-1,1-diéthyl-urée

A la solution de 0,25g du composé précédent (étape A de l'exemple 8) dans 6,8mL de dichorométhane refroidie à 0°C, on ajoute 0,22mL de diéthylamine. Après 24h d'agitation à 20°C, on traite à l'eau et extrait avec du dichlorométhane. La phase organique est séchée sur sulfate de sodium, évaporée à sec, purifiée par chromatographie sur silice en éluant avec un mélange cyclohexane/acétate d'éthyle pour donner le produit désiré sous forme d'une poudre blanche.
PF = 137°C
RMN ¹H 400MHz (DMSO-d₆): : 1,1(t,6H) ; 1,27(t,3H) ; 1,69(s,3H); 3,38-3,41(m,4H) ; 3,57(s,3H) ; 3,82-3,97(m,2H) ; 6,31(m,1H) ; 6,89-6,92(m,1H) ; 7,29-7,54(m,5H) ; 7,68-7,71(m,1H) ; 7,73-7,81 (m,2H)

### EXEMPLE 9

### 3-[1-(4-tert-Butylcarbamoyl.benzènesulfonyl)-5-éthoxy-3-méthyl-2-oxo-2,3-dihydro-1H-indol-3-yl]-4-chloro-benzoate de méthyle

On obtient le 3-[1-(4-*tert*-Butylcarbamoyl-benzenesulfonyl)-5-éthoxy-3-méthyl-2-oxo-2,3-dihydro-1*H-*indol-3-yl]-4-chloro-benzoate de méthyle de manière analogue à l'exemple 2, à partir du 4-chloro-5-éthoxy-3-méthyl-2-oxo-2,3-dihydro-1*H-*indol-3-yl)-benzoate de méthyle lévogyre (préparation 4-A).
PF= 140°C
RMN ¹H 400MHz (DMSO-d₆): : 1,22(t, 3H) ; 1,38(s, 9H); 1,77(s, 3H); 3,83-3,95(m, 5H); 6,48(m, 1H); 6,96-6,97(m, 1H); 7,43-7,45(m, 1H); 7,80-7,83(m, 1H); 7,90-7,92(m, 1H); 8,00-8,11 (m, 5H)

### EXEMPLE 10

### 3-[1-(4-tert-Butylcarbamoyl-2-méthoxy-benzènesulfonyl)-5-éthoxy-3-méthyl-2-oxo-2,3-dihydro-1H-indol-3-yl]-4-chloro-benzoate de méthyle

On obtient le 3-[1-(4-*tert*-butylcarbamoyl-2-méthoxy-benzènesulfonyl)-5-éthoxy-3-méthyl-2-oxo-2,3-dihydro-1*H-*indol-3-yl]-4-chloro-benzoate de méthyle de manière analogue à l'exemple 5 à partir du 4-chloro-5-éthoxy-3-méthyl-2-oxo-2,3-dihydro-1*H-*indol-3-yl)-benzoate de méthyle lévogyre (préparation 4-A).
PF= 142°C
RMN ¹H 400MHz (DMSO-d₆): 1,24(t, 3H); 1,39(s, 9H); 1,75(s, 3H); 3,72(s, 3H); 3,83-3,95(m, 5H); 6,49(m, 1H); 6,94-6,98(m, 1H) ;7,48-7,58(m, 3H); 7,70-7,78(m, 1H); 7,90-7,93(m, 1H); 8,02-8,06(m, 2H)

Dans les mêmes conditions que pour les exemples précédents, on obtient respectivement les inverses optiques des exemples précédents, en partant des composés III dextrogyres et des chlorures de sulfonyles appropriés.

**Tableau II**

| | | | | | | | | | |
|---|---|---|---|---|---|---|---|---|---|
| 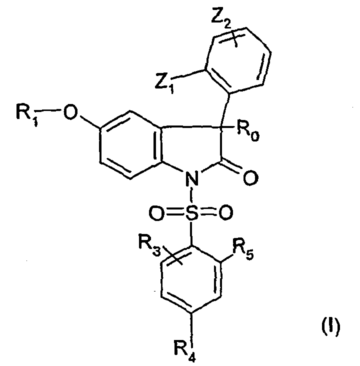 | | | | | | | | | |

| N° | R₀ | R₁ | Z₁ | Z₂ | R₄ | R₃ | R₅ | PF(°C) | MH+ |
|---|---|---|---|---|---|---|---|---|---|
| 11 | Me | Et | Cl | H | OMe | H | OMe | 198 | 502 |
| 12 | Me | Et | Cl | H | 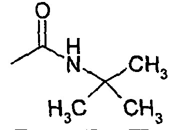 | H | H | 153 | 541 |
| 13 | Me | Et | Cl | H | 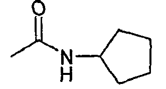 | H | H | 150 | 553 |
| 14 | Me | Et | Cl | H | 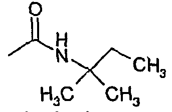 | H | H | 130 | 555 |
| 15 | Me | Et | Cl | H | 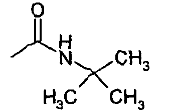 | H | OMe | 143 | 571 |
| 16 | Me | Et | F | H | 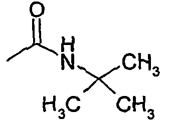 | H | OMe | 128 | 555 |
| 17 | Me | Et | Cl | H | -NH₂ | H | OMe | 119 | 487 |
| 18 | Me | Et | Cl | H | 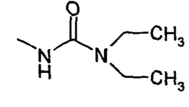 | H | OMe | 124 | 586 |

### EXEMPLE 19

### Acide 3-[1-(4-tert-Butylcarbamoyl-2-méthoxy-benzènesulfonyl)-5-éthoxy-3-méthyl-2-oxo-2,3-dihydro-1H-indol-3-yl]-4-chloro-benzoïque

On obtient "acide 3-[1-(4-*tert*-butylcarbamoyl-2-méthoxy-benzènesulfonyl)-5-éthoxy-3-méthyl-2-oxo-2,3-dihydro-1*H-*indol-3-yl]-4-chloro-benzoïque de la même manière que dans l'exemple 5 à partir d'acide 4-chloro-3-(5-éthoxy-3-méthyl-2-oxo-2,3-dihydro-1*H-*indol-3-yl)-benzoïque lévogyre (préparation 2)
PF = 200°C
RMN ¹H 400MHz (DMSO-d₆): 1,24(t,3H); 1,39(s,9H); 1,73(s,3H); 3,71(s,3H); 3,85-3,96(m,2H); 6,44-6,45(m,1H); 6,93-6,98(m,1H); 7,38-7,41(m,1H); 7,5-7,58(m,2H); 7,7-7,74(m,1H); 7,86-7,89(m,1H); 8,02-8,08(m,2H)

### EXEMPLE 20

### N-tert-Butyl-4-{3-[2-chloro-5-(4-méthyl-pipérazine-1-carbonyl)-phényl]-5-éthoxy-3-méthyl-2-oxo-2,3-dihydro-indole-1-sulfonyl}-3-méthoxy-benzamide

A la solution de 0,2g d' acide 3-[1-(4-*tert*-butylcarbamoyl-2-méthoxy-benzènesulfonyl)-5-éthoxy-3-méthyl-2-oxo-2,3-dihydro-1*H-*indol-3-yl]-4-chloro-benzoïque (exemple 19) dans 5mL de dichlorométhane refroidie à 0°C, on ajoute dans l'ordre 0,07mL de N-méthylpipérazine, 0,182g d'hexafluorophosphate de benzotriazolyl-N-oxytrisdiméthylamino phosphonium et 0,1mL de triéthylamine. Après une nuit d'agitation à 20°C, on ajoute 5mL d'eau, sépare la phase organique sur une cartouche hydrophobe, purifie par chromatographie sur une colonne de silice, en éluant avec un mélange dichlorométhane/méthanol dans les proportions 97/3 respectivement, pour obtenir le produit attendu sous forme d'une poudre blanche.
PF = 96°C
RMN ¹H 400MHz (DMSO-d₆): 1,25(t,3H); 1,39(s,9H); 1,70(s,3H); 2,22(s,3H); 2,31-2,48(m,4H); 3,25-3,35(m,4H); 3,72(s,3H); 3,95(q,2H); 6,92-6,99(m,1H); 7,38-7,4(m,2H); 7,5-7,59(m,2H); 7,7-7,73(m,2H); 8-8,05(m,2H)

Dans les mêmes conditions on obtient les exemples suivants en partant de l'acide 3-[1-(4-*tert*-Butylcarbamoyl-2-méthoxy-benzènesulfonyl)-5-éthoxy-3-méthyl-2-oxo-2,3-dihydro-1*H-*indol-3-yl]-4-chloro-benzoïque (exemple 19) et des amines commerciales appropriées.

**Tableau III**

| | | | | | | | | | |
|---|---|---|---|---|---|---|---|---|---|
| 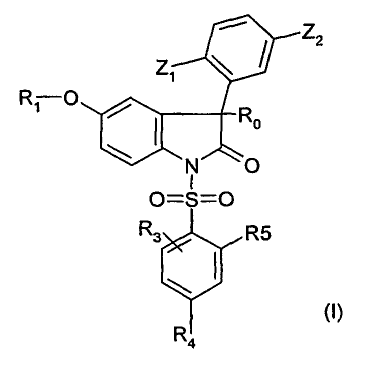 | | | | | | | | | |

| N° | R₀ | R₁ | Z₁ | Z₂ | R₄ | R₃ | R₅ | PF (°C) | MH⁺ |
|---|---|---|---|---|---|---|---|---|---|
| 21 | Me | Et | Cl | 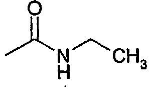 | 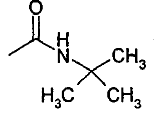 | H | OMe | 176 | 642 |
| 22 | Me | Et | Cl | 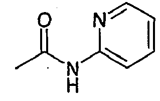 | 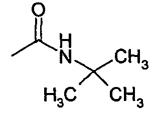 | H | OMe | 176 | 691 |
| 23 | Me | Et | Cl | 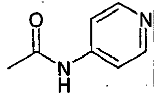 | 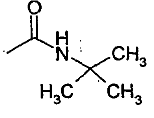 | H | OMe | 186 | 691 |
| 24 | Me | Et | Cl | 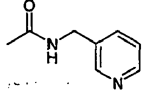 | 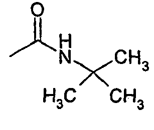 | H | OMe | 170 | 705 |
| 25 | Me | Et | Cl | 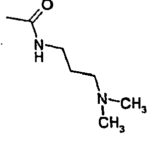 | 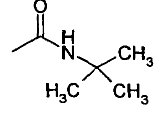 | H | OMe | 148 | 699 |
| 26 | Me | Et | Cl | 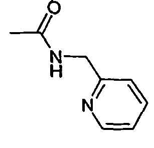 | 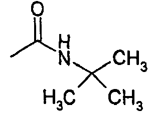 | H | OMe | 146 | 705 |
| 27 | Me | Et | Cl | 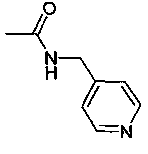 | 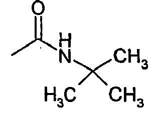 | H | OMe | 146 | 705 |
| 28 | Me | Et | Cl | 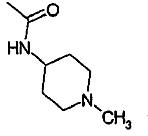 | 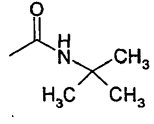 | H | OMe | 134 | 711 |
| 29 | Me | Et | Cl | 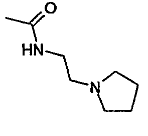 | 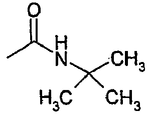 | H | OMe | 136 | 711 |
| 30 | Me | Et | Cl | 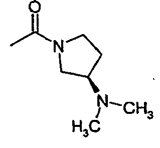 | 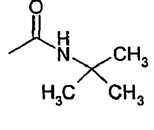 | H | OMe | 146 | 711 |
| 31 | Me | Et | Cl | 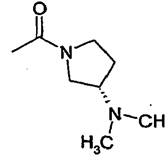 | 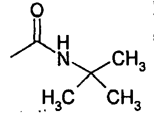 | H | OMe | 166 | 711 |
| 32 | Me | Et | Cl | 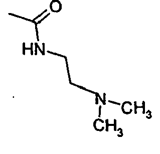 | 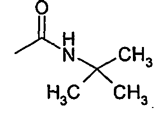 | H | OMe | 115 | 684 |
| 33 | Me | Et | Cl | 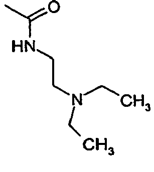 | 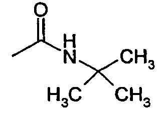 | H | OMe | 150 | 713 |
| 34 | Me | Et | Cl | 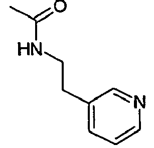 | 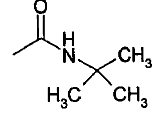 | H | OMe | 104 | 719 |
| 35 | Me | Et | Cl | 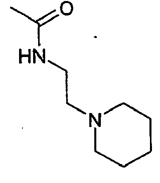 | 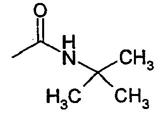 | H | OMe | 126 | 725 |
| 36 | Me | Et | Cl | 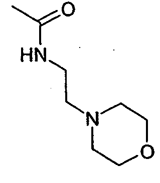 | 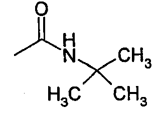 | H | OMe | 124 | 727 |
| 37 | Me | Et | Cl | 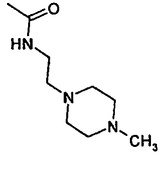 | 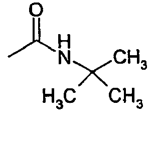 | H | OMe | 160 | 740 |
| 38 | Me | Et | Cl | 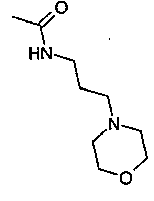 | 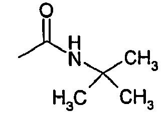 | H | OMe | 124 | 741 |
| 39 | Me | Et | Cl | 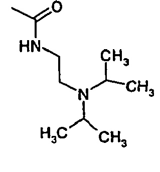 | 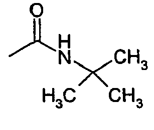 | H | OMe | 152 | 741 |
| 40 | Me | Et | Cl | 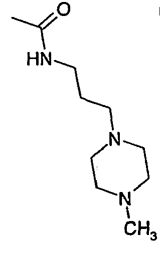 | 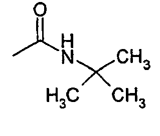 | H | OMe | 152 | 754 |
| 41 | Me | Et | Cl | 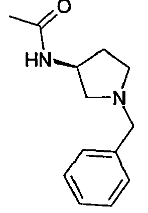 | 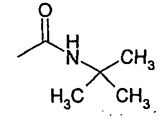 | H | OMe | 154 | 773 |
| 42 | Me | Et | Cl | 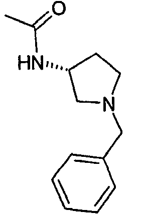 | 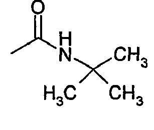 | H | OMe | 152 | 773 |
| 43 | Me | Et | Cl | 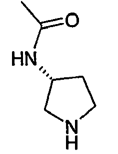 | 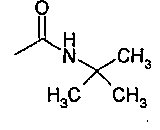 | H | OMe | 216 | 683 |
| 44 | Me | Et | Cl | 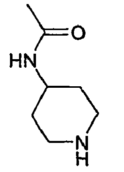 | 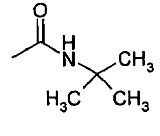 | H | OMe | 240 | 697 |

### EXEMPLE 45

### N-tert-Butyl-4-{3-[2-chloro-5-(N'-éthyl-N'-(3-pyridyl)méthylaminocarbonyl)-phényl]-5-éthoxy-3-méthyl-2-oxo-2,3-dihydro-indole-1-sulfonyl}-3-méthoxy-benzamide

### A) Préparation de la N-éthyl-3-pyridyl-méthylamine

On ajoute 5g de 3-pyridylcarboxaldéhyde au mélange de 3,8g de chlorhydrate d'éthylamine, 60mL de toluène, 110mL d'éthanol et 13,2 mL de triéthylamine. Après 5 minutes d'agitation à 20°C, on ajoute 25g de tamis moléculaire 4 A et on maintient sous agitation à 20°C. On filtre l'insoluble, puis on lave au dichlorométhane et on évapore à sec et on reprend le résidu avec 50 mL de méthanol. A une température d'environ 0°C, on ajoute 1,8g de borohydrure de sodium puis agite à 20°C une nuit. On évapore le solvant sous vide, reprend le résidu avec du dichlorométhane, lave à la soude 1 N puis avec une solution aqueuse de chlorure de sodium, sèche sur sulfate de sodium, évapore le solvant puis distille sous vide.
Eb = 77°C sous 530 Pa
RMN ¹H 250MHz (CDCl3) : 1,18(t,3H) ;2,73(q, 4H) ; 3,85(s, 2H) ; 7,25-7,32(m, 1H) ;7,65-7,75(m, 1H) ; 8,50-8,65(m, 2H)

### B) N-tert-Butyl-4-{3-[2-chloro-5-(N'-éthyl-N'-(3-pyridyl)méthylamino-1-carbonyl)-phényl]-5-éthoxy-3-méthyl-2-oxo-2,3-dihydro-indole-1-sulfonyl}-3-méthoxy-benzamide

Dans les conditions de l'exemple 20, à partir de l'acide 3-[1-(4-*tert*-Butylcarbamoyl-2-méthoxy-benzènesulfonyl)-5-éthoxy-3-méthyl-2-oxo-2,3-dihydro-1*H-*indol-3-yl]-4-chloro-benzoïque (exemple 19) et de l'amine précédente (étape A de l'exemple 45), on obtient le composé désiré sous forme d'une poudre blanche.
PF = 148°C
MS[(+)ESI,m/z]:733(MH+)

### EXEMPLE 46

### N-tert-Butyl-4-{3-[2-chloro-5-(1-méthyl-hexahydro-pyrrolo[3,4-b]pyrrole-5-carbonyl)-phényl]-5-éthoxy-3-méthyl-2-oxo-2,3-dihydro-indole-1-sulfonyl}-3-méthoxy-benzamide

Dans les conditions de l'exemple 20, à partir de l'acide 3-[1-(4-*tert*-butylcarbamoyl-2-methoxy-benzenesulfonyl)-5-éthoxy-3-méthyl-2-oxo-2,3-dihydro-1*H-*indol-3-yl]-4-chloro-benzoïque (exemple 19) et du 1-méthyl-octahydro-pyrrolo[3,4-*b*]pyrrole obtenu selon les conditions décrites dans "Justus Liebigs Ann. Chemie 677, 154 (1964)", on obtient le composé désiré sous forme d'une poudre blanche.
PF = 130°C
MS[(+)ESI,m/z]:723(MH+)
RMN ¹H 400MHz (DMSO-d₆) : 1,23(t,3H) ; 1,38-1,41(m,9H) ; 1,42-1,61(m,1H); 1,71(s,3H) ; 1,82-2,08(m,1H) ; 2,11-2,21(m,3H) ; 2,23-2,3(m,1H) ; 2,7-2,82(m,1H) ; 2,95-3,05(m,1H) ;3,33-3,71(m,4H); 3,72(s,3H) ; 3,87-4(m,2H) ; 6,4-6,46(m,1H); 6,92-7(m,1H) ; 7,32-7,4(m,1H) ; 7,45-7,58(m,3H) ; 7,68-7,72(m,1H) ; 7,79-7,88(m,1H) ; 7,99-8,09(m,2H)

### EXEMPLE 47

### N-tert-Butyl-4-{3-[2-chloro-5-(2-aminoéthylamino-carbonyl)-phényl]-5-éthoxy-3-méthyl-2-oxo-2,3-dihydro-indole-1-sulfonyl}-3-méthoxy-benzamide

**A)** A la solution de 0,15g d' acide 3-[1-(4-*tert*-butylcarbamoyl-2-méthoxy-benzènesulfonyl)-5-éthoxy-3-méthyl-2-oxo-2,3-dihydro-1*H-*indol-3-yl]-4-chloro-benzoïque (exemple 19) dans 5mL de dichlorométhane refroidie à 0°C, on ajoute dans l'ordre 0,07mL de 2-*tert*-butoxycarbonylamino-éthylamine, 0,139g d'hexafluorophosphate de benzotriazolyl-N-oxytrisdiméthylamino phosphonium et 0,08mL de triéthylamine. Après une nuit d'agitation à 20°C, on ajoute 5mL d'eau, puis on sépare la phase organique sur une cartouche hydrophobe, purifie par chromatographie sur une colonne de silice, en éluant avec un mélange dichlorométhane/méthanol dans les proportions 97/3 respectivement, pour obtenir le produit attendu sous forme d'une poudre blanche.
   MS06/04/125 : MS[(+),ESI,m/z]: 757(MH+)
   RMN ¹H 400MHz (DMSO-d₆): 1,22(t,3H); 1,35-1,42(m,18H); 1,76(s,3H); 3,1-3,19(m,2H); 3,29-3,32(m,2H); 3,72(s,3H); 3,82-3,97(m,2H); 6,42(s,1H); 6,9-6,99(m,2H); 7,5/7,58(m,2H); 7,7-7,75(m,1H); 7,78-7,81(m,1H); 8,02-8,09(m,2H)
**B)** On obtient le chlorhydrate du composé désiré sous forme de poudre blanche par déprotection du composé précédent dans une solution éthérée d'acide chlorhydrique. PF = 204°C
   MS[(+)ESI,m/z]:657(MH+)

### EXEMPLE 48

### Acide 3-[1-(4-tert-butylcarbamoyl-benzènesulfonyl)-5-éthoxy-3-méthyl-2-oxo-2,3-dihydro-1H-indol-3-yl]-4-chloro-benzoïque

On obtient l'acide 3-[1-(4-*tert*-butylcarbamoyl-benzènesulfonyl)-5-éthoxy-3-méthyl-2-oxo-2,3-dihydro-1*H-*indol-3-yl]-4-chloro-benzoïque de manière analogue à la méthode décrite à l'exemple 2, à partir d'acide 4-chloro-3-(5-éthoxy-3-méthyl-2-oxo-2,3-dihydro-1*H-*indol-3-yl)-benzoïque lévogyre (préparation 2).
PF = 188°C
RMN ¹H 400MHz (DMSO-d6) : 1,23(t,3H); 1,38(s,9H); 1,77(s,3H); 3,84-3,95(m,2H); 6,47(s,1H); 6,92-6,98(m,1H); 7,37-7,4(m,1H); 7,78-7,9(m,2H); 7,98-8,04(m,2H); 8,08-8,11(m.3H)

### EXEMPLE 49

### N-tert-butyl-4-{3-[2-chloro-5-(3-pyridylméthylaminocarbonyl)-phényl]-5-éthoxy-3-méthyl-2-oxo-2,3-dihydro-indole-1-sulfonyl}-benzamide

On obtient le *N-tert*-butyl-4-{3-[2-chloro-5-(3-pyridylméthylaminocarbonyl)-phényl]-5-éthoxy-3-méthyl-2-oxo-2,3-dihydro-indole-1-sulfonyl}-benzamide dans les conditions de l'exemple 20, à partir de l'acide 3-[1-(4-*tert*-butyfcarbamoyl-benzènesulfonyl)-5-éthoxy-3-méthyl-2-oxo-2,3-dihydro-1*H-*indol-3-yl]-4-chloro-benzoïque (exemple 48) et de la 3-pyridylméthylamine.
PF = 130°C
RMN ¹H 400MHz (DMSO-d₆): 1,15-1,27(m,3H); 1,38(s,9H); 1,79(s,3H); 3,87-3,93(m,2H); 4,67(d,2H); 6,47(s,1H); 6,91-6,98(m,1H); 7,38-7,41(m,1H); 7,8-7,92(m,3H); 7,99-8,05(m,2H); 8,09-8,11(m,2H); 8,24(s,1H); 8,38-8,9(m,1H); 8,75-8,79(m,1H); 8,88-8,9(m,1H)

Dans les mêmes conditions, on obtient les exemples suivants en partant de l'acide 3-[1-(4-*tert*-butylcarbamoyl-benzènesulfonyl)-5-éthoxy-3-méthyl-2-oxo-2,3-dihydro-1*H-*indol-3-yl]-4-chloro-benzoïque (exemple 48) et des amines commerciales appropriées.

**Tableau IV**

| | | | | | | | | | |
|---|---|---|---|---|---|---|---|---|---|
| 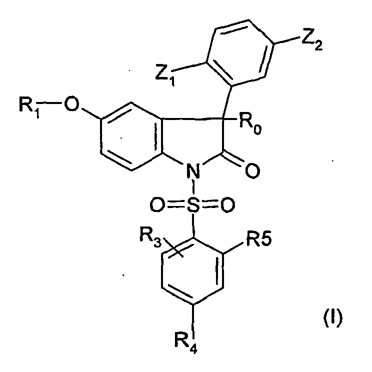 | | | | | | | | | |

| N° | R₀ | R₁ | Z₁ | Z₂ | R₄ | R₃ | R₅ | PF (°C) | MH+ |
|---|---|---|---|---|---|---|---|---|---|
| 50 | Me | Et | Cl | 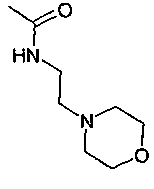 | 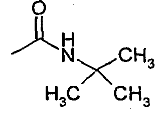 | H | H | 148 | 697 |
| 51 | Me | Et | Cl | 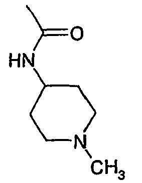 | 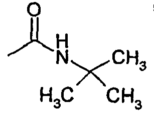 | H | H | 212 | 681 |
| 52 | Me | Et | Cl | 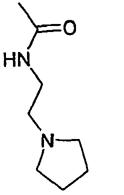 | 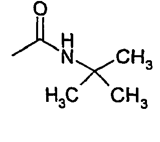 | H | H | 132 | 681 |
| 53 | Me | Et | Cl | 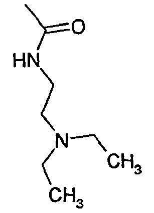 | 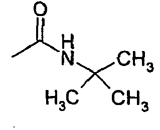 | H | H | 124 | 683 |

### EXEMPLE 54

### N-benzyl-4-chloro-3-{1-[4-(3,3-diéthyl-uréido)-2-méthoxy-benzènesulfonyl]-5-éthoxy-3-méthyl-2-oxo-2,3-dihydro-1H-indol-3-yl}-benzamide

### A) Acide 4-Chloro-3-[5-éthoxy-3-méthyl-1-(4-nitro-benzènesulfonyl)-2-oxo-2,3-dihydro-1H-indol-3-yl]-benzoïque

A la solution de 0,5g de 4-chloro-3-(5-éthoxy-3-méthyl-2-oxo-2,3-dihydro-1H-indol-3-yl)-benzoic acid lévogyre (préparation 2) dans 7,5mL de tétrahydrofuranne refroidie à - 20°C, on ajoute 0,340g de ter-butylate de potassium. On laisse remonter la température à 0°C, refroidi à -20°C pour ajouter 0,400g de chlorure de 4-nitro-2-méthoxybenzènesulfonyle.

Après une nuit d'agitation à 20°C, on traite à l'eau et extrait avec de l'acétate d'éthyle.

La phase organique est séchée sur sulfate de sodium, évaporée à sec, purifiée par chromatographie sur silice, en éluant avec un mélange dichlorométhane/méthanol dans les proportions 93/7 respectivement, pour donner le produit désiré sous forme d'une poudre blanche.
RMN ¹H 400MHz (DMSO-d₆): 1,21(t,3H); 1,75(s,3H); 3,81(s,3H); 3,85-3,95(m,2H); 6,41-6,48(m,1H); 6,92-6,99(m,1H); 7,35-7,4(m,1H); 7,68-7,73(m,1H); 7,85-7,9(m,1H); 7,96-8,05(m,2H); 8,25-8,3(m,2H)

### B) 4-Chloro-3-[5-chloro-1-(2-méthoxy-4-nitro-benzènesulfonyl)-3-méthyl-2-oxo-2,3-dihydro-1H-indol-3-yl]-N-pyridin-3-ylméthyl-benzamide

A la solution de 0,32g de l'acide préparé précédemment (étape A de l'exemple 54) dans 5mL de dichlorométhane refroidie à 0°C, on ajoute dans l'ordre 0,12mL de 3-pyridylméthylamine, 0,76g d'hexafluorophosphate de benzotriazolyl-N-oxytrisdiméthylamino phosphonium et 0,19mL de triéthylamine. Après une nuit d'agitation à 20°C, on ajoute 5mL d'eau, puis on sépare la phase organique sur une cartouche hydrophobe. On purifie ensuite par chromatographie sur une colonne de silice, en éluant avec un mélange dichlorométhane/méthanol dans les proportions 95/5 respectivement, pour obtenir le produit attendu sous forme d'une poudre jaune.
RMN ¹H 250MHz (DMSO-d₆): 1,2-1,3(m,3H); 1,79(s,3H); 3,81(s,3H); 3,85-3,99(m,2H); 4,54(d,2H); 6,48-6,5(m,1H); 6,95-7,01(m,1H); 7,35-7,48(m,2H); 7,69-7,8(m,2H); 7,85-7,9(m,1H); 7,99-8,06(m,2H); 8,2-8,33(m,2H); 8,46-8,5(m,1H); 8,59-8,62(m,1H)

### C) 3-[1-(4-Amino-2-méthoxy-benzènesulfonyl)-5-chloro-3-méthyl-2-oxo-2,3-dihydro-1H-indol-3-yl]-4-chloro-N-pyridin-3-ylméthyl-benzamide

A 0,37g de 4-chloro-3-[5-chloro-1-(2-méthoxy-4-nitro-benzènesulfonyl)-3-méthyl-2-oxo-2,3-dihydro-1H-indo]-3-yl]-N-pyridin-3-ylméthyl-benzamide (étape B de l'exemple 54) en suspension dans 5 mL d'ethanol on ajoute 0,158 g de fer puis 0,23 mL d'acide acétique. On chauffe le mélange réactionnel sous agitation à reflux pendant 3 heures. On évapore partiellement les solvants sous pression réduite puis on ajoute 10 mL d'une solution aqueuse saturée d'hydrogénocarbonate de sodium et 10 mL d'acétate d'éthyle. Le mélange réactionnel est agité 15 minutes à température ambiante, puis filtré et rincé avec l'acétate d'éthyle. Après décantation la phase organique est lavée avec une solution aqueuse saturée de chlorure de sodium. La phase organique est séchée sur Na₂SO₄, évaporée à sec, purifiée par chromatographie sur silice, en éluant avec un mélange dichlorométhane/méthanol dans les proportions 93/6 respectivement, pour donner le produit désiré sous forme d'une poudre blanche.
RMN ¹H 250MHz (DMSO-d₆):1,21(t,3H) ; 1,73(s,3H) ; 3,51(s,3H) ; 3,8-3,95(m,2H) ; 4,55(d,2H) ; 6,15-6,24(m,2H) ; 6,39-6,42(m,1H); 6,88-6,95(m,1H) ; 7,35-7,48(m,2H) ; 7,56-7,61 (m,1H) ; 7,65-7,7(m,1H) ; 7,75-7.89(m,2H) ; 8,19-8,21(m,1H) ; 8,47-8,5(m,1H) ; 8,58-8,62(m1H)

### D) Acide [4-(5-Chloro-3-{2-chloro-5-[(pyridin-3-ylméthyl)-carbamoyl]-phényl}-3-méthyl-2-oxo-2,3-dihydro-indole-1-sulfonyl)-3-méthoxy-phenyl]-carbamicphenyl ester

A la solution de 0,53g de 3-[1-(4-amino-2-méthoxy-benzènesulfonyl)-5-chloro-3-méthyl-2-oxo-2,3-dihydro-1H-indol-3-yl]-4-chloro-N-pyridin-3-ylméthyl-benzamide (étape C de l'exemple 54) dans 13mL de tétrahydrofuranne refroidie à 0°C, on ajoute 0,7mL d'une solution aqueuse de soude 1,5N puis lentement 0,08mL de chlorocarbonate de phényle. Après une nuit d'agitation à 20°C, on traite à l'eau et extrait avec de l'acétate d'éthyle. La phase organique est séchée sur sulfate de sodium, évaporée à sec, purifiée par chromatographie sur silice, en éluant avec un mélange dichlorométhane/méthanol 9/1, pour donner le produit désiré sous forme d'une mousse blanche.
RMN ¹H 400MHz (DMSO-d₆): 1,22(t,3H); 1,69-1,8(m,3H) ; 3,59(s,3H) ; 3,82-3,95(m,2H) ; 4,49-4,58(m,2H) ; 6,4-6,45(m,1H) ; 6,72-6,8(m,1H) ; 6,88-6,98(m,1H) ; 7,12-7,3(m,3H) ; 7,35-7,48(m,4H) ; 7,65-7,75(m,2H) ; 7,81-7,98(m,2H) ; 8,15-8,21 (m,1H) ; 8,41-8,49(m,1H) ; 8,56-8,61 (m,1H) ; 9,25-9,32(m, 1H)

### E) 4-Chloro-3-{5-chloro-1-[4-(3,3-diéthyl-uréido)-2-méthoxy-benzènesulfonyl]-3-méthyl-2-oxo-2,3-dihydro-1H-indol-3-yl}-N-pyridin-3-ylméthyl-benzamide

A la solution de 0,075g d'acide [4-(5-chloro-3-{2-chloro-5-[(pyridin-3-ylméthyl)-carbamoyl]-phényl}-3-méthyl-2-oxo-2,3-dihydro-indole-1-sulfonyl)-3-méthoxy-phényl]-carbamicphenyl ester (étape D de l'exemple 54) dans 1,5mL de dichorométhane refroidie à 0°C, on ajoute 0,05mL de diéthylamine. Après 24h d'agitation à 20°C, on traite à l'eau et sépare la phase organique sur une cartouche hydrophobe. La phase organique est séchée sur sulfate de sodium, évaporée à sec, purifiée par chromatographie sur silice, en éluant avec un mélange dichlorométhane/méthanol dans les proportions 95/5 respectivement, pour donner le produit désiré sous forme d'une poudre blanche.
PF = 148°C
RMN ¹H 400MHz (DMSO-d₆): 1,09(t,6H) ; 1,24(t,3H) ; 1,74(s,3H) ; 3,32-3,4(m,4H) ;
3,58(s,3H) ; 3,82-3,91(m,2H); 4,5-4,55(m,2H) ; 6,42-6,43(m,1H) ; 6,89-6,93(m,1H) ; 7,29-7,45(m,3H) ; 7,52-7,55(m,1H) ; 7,68-7,88(m,4H) ; 8,18-8,21(m,1H) ; 8,45-8,5(m,1H) ; 8,57-8,6(m,1H)

### EXEMPLE 55

### 4-[3-(5-Amino-2-chloro-phényl)-5-éthoxy-3-méthyl-2-oxo-2,3-dihydro-indole-1-sulfonyl]-N-tert-butyl-3-méthoxy-benzamide

On obtient le 4-[3-(5-Amino-2-chloro-phényl)-5-éthoxy-3-méthyl-2-oxo-2,3-dihydro-indole-1-sulfonyl]-*N-tert*-butyl-3-méthoxy-benzamide de la même manière que dans l'exemple 5, à partir de 3-(5-amino-2-chloro-phényl)-5-éthoxy-3-méthyl-2-oxo-2,3-dihydro-indole lévogyre (préparation 3)
PF = 166°C
RMN ¹H 400MHz (DMSO-d₆): 1,25(t,3H); 1,38(s,9H); 1,57(s,3H); 3,68(s,3H); 3,88-3,98(m,2H); 6,38(m,1H); 6,45-6,5(m,1H); 6,83-6,88(m,1H); 6,9-6,95(m,2H); 7,5-7,55(m,1H); 7,67-7,69(m,1H); 8-8,09(m,2H)

### EXEMPLE 56

### N-{3-[1-(4-tert-butylcarbamoyl-2-méthoxy-benzènesulfonyl)-5-éthoxy-3-méthyl-2-oxo-2,3-dihydro-1H-indol-3-yl]-4-chloro-phényl}-nicotinamide

A la solution de 0,15g du composé de l'exemple 55 dans 5mL de dichlorométhane refroidie à 0°C, on ajoute lentement 0,22mL de triéthylamine et 0,091g de chlorhydrate de chlorure de nicotinyle. Après une nuit d'agitation à 20°C, on hydrolyse avec 5mL de chlorure d'ammonium dilué. La phase organique décantée est relavée à l'eau, évaporée à sec. On purifie le résidu par chromatographie sur une colonne de silice en éluant avec un gradient dichlorométhane / méthanol pour obtenir le produit attendu sous forme d'une poudre blanche.
PF = 192°C
RMN ¹H 400 MHz (DMSO-d₆): 1,25(t,3H); 1,39(s,9H); 1,69(s,3H); 3,71(s,3H); 3,88-3,98(m,2H); 6,92-6,98(m,1H); 7,3-7,32(m,1H); 7,5-7,65(m,3H); 7,7-7,77(m,1H); 7,85-7,88(1H); 8,02-8,1 (m,2H); 8,21(m,1H); 8,3-8,35(m,1H); 8,78-8,8(m,1H); 9,15(m, 1H)

### EXEMPLE 57

### N-tert-butyl-4-{3-[2-chloro-5-(2-diméthylamino-acétylamino)-phényl]-5-éthoxy-3-méthyl-2-oxo-2,3-dihydro-indole-1-suifonyl}-3-méthoxy-benzamide

A la solution de 0,15g du composé de l'exemple 55 dans 5mL de dichlorométhane refroidie à 0°C, on ajoute dans l'ordre 0,053g de N,N-diméthyl-glycine, 0,283g de chlorure de bis(2-oxo-3-oxazolidinyl)phosphinique et 0,22mL de triéthylamine. Après une nuit d'agitation à 20°C, on ajoute 5mL d'eau, sépare la phase organique que l'on lave à l'eau encore 2 fois avant puis évapore sous vide. On purifie le résidu par chromatographie sur une colonne de silice en éluant avec un gradient dichlorométhane /méthanol pour obtenir le produit attendu sous forme d'une poudre blanche.
PF = 150°C
RMN ¹H 400MHz (DMSO-d₆): 1,25(t,3H); 1,38(s,9H); 1,65(s,3H); 2,29(s,6H); 3,09(s,2H); 3,7(s,3H); 3,88-3,98(m,2H); 6,9-6,96(m,1H); 7,19-7,22(m,1H); 7,48-7,55(m,2H); 7,68-7,71(m,1H); 7,75-7,81(m,1H); 8-8,08(m,3H)

Dans les mêmes conditions, on obtient les exemples suivants en partant du 4-[3-(5-amino-2-chloro-phényl)-5-éthoxy-3-méthyl-2-oxo-2,3-dihydro-indole-1-sulfonyl]-*N-tert-*butyl-3-methoxy-benzamide (exemple 55) et des acides commerciaux appropriées.

**Tableau V**

| | | | | | | | | | |
|---|---|---|---|---|---|---|---|---|---|
| 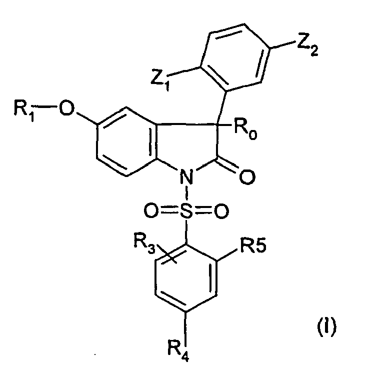 | | | | | | | | | |

| N° | R₀ | R₁ | Z₁ | Z₂ | R₄ | R³ | R⁵ | PF (°C) | MH+ |
|---|---|---|---|---|---|---|---|---|---|
| 58 | Me | Et | Cl | 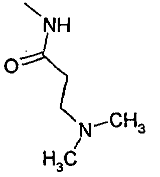 | 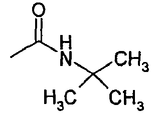 | H | OMe | 170 | 685 |
| 59 | Me | Et | Cl | 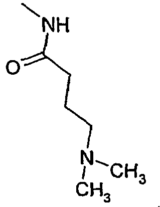 | 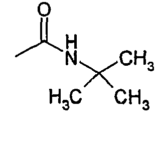 | H | OMe | 160 | 699 |
| 60 | Me | Et | Cl | 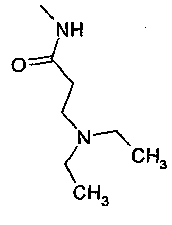 | 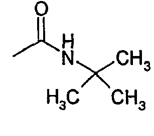 | H | OMe | 136 | 713 |
| 61 | Me | Et | Cl | 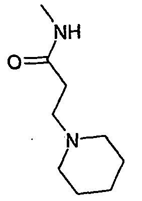 | 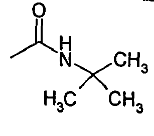 | H | OMe | 165 | 725 |
| 62 | Me | Et | Cl | 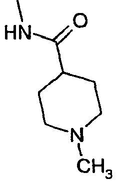 | 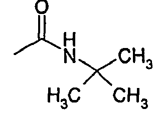 | H | OMe | 180 | 711 |

### EXEMPLE 63

### N-tert-butyl-4-[3-(2-chloro-5-pyrrolidin-1-ylméthyl-phényl)-5-éthoxy-3-méthyl-2-oxo-2,3-dihydro-indole-1-sulfonyl]-3-méthoxy-benzamide

### A) 3-(2-chloro-5-pyrrolidin-1-ylméthyl-phényl)-5-éthoxy-3-méthyl-2-oxo-2,3-dihydro-indole

A la solution de 0,3g de 4-chloro-3-(5-éthoxy-3-méthyl-2-oxo-2,3-dihydro-1*H-*indol-3-yl)-benzaldéhyde chiral (préparation 4 C) dans 6mL de dichlorométhane refroidie à 0°C, on ajoute dans l'ordre 0,19mL de pyrrolidine, 0,243g de triacétoxyborohydrure de sodium et 0,10mL d'acide acétique. Après une nuit d'agitation à 20°C, on évapore sous vide, reprend par 10mL d'acétate d'éthyle, lave avec 10mL de solution aqueuse de bicarbonate de sodium, sépare la phase organique, la sèche sur sulfate de sodium puis évapore sous vide. On purifie le résidu par chromatographie sur une colonne de silice en éluant avec un gradient dichlorométhane / méthanol pour obtenir le produit attendu sous forme d'une poudre blanche.
MS[(+)ESI,m/z]:385(MH+)
RMN ¹H 250MHz (DMSO-d₆): 1,2(t,3H) ; 1,64(s,3H) ; 1,69-1,8(m,4H) ; 3,28-3,35(m,4H) ; 3,64-3,7(m,2H) ; 3,80-3,91(m,2H) ; 6,3-6,32(m,1H) ; 6,7-6,81(m,2H) ; 7,25-7,28(m,2H) ; 7,62-7,68(m,1H)

### B) N-tert-Butyl-4-[3-(2-chloro-5-pyrrolidin-1-ylméthyl-phényl)-5-éthoxy-3-méthyl-2-oxo-2,3-dihydro-indole-1-sulfonyl]-3-méthoxy-benzamide

On obtient le *N-tert*-butyl-4-[3-(2-chloro-5-pyrrolidin-1-ylméthyl-phényl)-5-éthoxy-3-méthyl-2-oxo-2,3-dihydro-indole-1-sulfonyl]-3-méthoxy-benzamide de la même manière que dans l'exemple 5, à partir du composé préparé précédemment (étape A de l'exemple 63).
PF = 132°C
RMN ¹H 400MHz (DMSO-d₆): 1,22(t,3H); 1,39(s,9H); 1,69-1,79(m,7H) ; 2,4-2,52(m,4H) ; 3,6-3,71(m,5H); 3,85-3,95(m,2H) ; 6,92-6,98(m,1H) ; 7,21-7,31(m,2H); 7,49-7,58(m,2H) ; 7,65-7,71(m,2H) ; 8-8,06(m,2H)

Dans les mêmes conditions, on obtient les exemples suivants en partant de 4-chloro-3-(5-éthoxy-3-méthyl-2-oxo-2,3-dihydro-1*H-*indol-3-yl)-benzaldéhyde chiral (préparation 4 C) et des amines commerciales appropriées.

**Tableau VI**

| | | | | | | | | | |
|---|---|---|---|---|---|---|---|---|---|
| 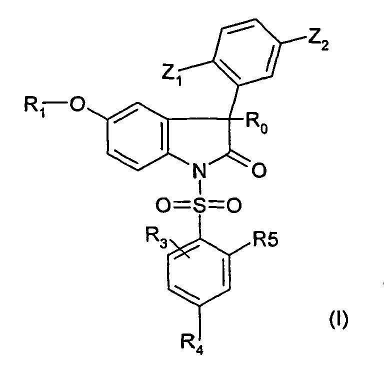 | | | | | | | | | |

| N° | R₀ | R₁ | Z₁ | Z₂ | R₄ | R₃ | R₅ | PF (°C) | Spectromèt rie de masse : MH+ |
|---|---|---|---|---|---|---|---|---|---|
| 64 | Me | Et | Cl | 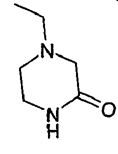 | 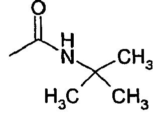 | H | OMe | 164 | 683 |
| 65 | Me | Et | Cl | 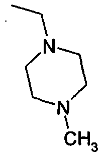 | 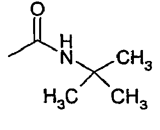 | H | OMe | 136 | 683 |

### EXEMPLE 66

### N-(2-fluoro-1,1-diméthyl-éthyl)-4-[3-(2-Chloro-phényl)-5-éthoxy-3-méthyl-2-oxo-2,3-dihydro-indole-1-sulfonyl]-benzamide

### A) Acide 4-[3-(2-Chloro-phényl)-5-éthoxy-3-méthyl-2-oxo-2,3-dihydro-indole-1-sulfonyl]-benzoïque

A la solution de 2g de 3-(2-chloro-phényl)-5-éthoxy-3-méthyl-1,3-dihydro-indol-2-one dextrogyre (Préparation 1) dans 20mL de tétrahydrofuranne refroidie à -10°C, on ajoute 0,70g d'une dispersion d'hydrure de sodium à 60% dans l'huile. On laisse remonter la température à 0°C, on refroidit à -10°C puis ajoute 1.61 g d'acide 4-chlorosulfonyl-benzoïque. Après 15 heures d'agitation à 20°C, on hydrolyse à l'eau et extrait avec de l'acétate d'éthyle. La phase organique est séchée sur sulfate de sodium, évaporée à sec. Le résidu est purifié par chromatographie sur silice, en éluant par le gradient du mélange DCM/MeOH de (100/0 ; v/v) à (90/10 ; v/v), pour donner le produit désiré sous forme de résine blanche, PF= 174°C.

### B) N-(2-fluoro-1,1-diméthyl-éthyl)-4-[3-(2-Chloro-phényl)-5-éthoxy-3-méthyl-2-oxo-2,3-dihydro-indole-1-sulfonyl]-benzamide

A la solution de 0.2g d' acide 4-[3-(2-Chloro-phényl)-5-éthoxy-3-méthyl-2-oxo-2,3-dihydro-indole-1-sulfonyl]-benzoïque préparé en A dans 3ml de dichlorométhane refroidie à 0°C, on ajoute dans l'ordre 0.115g de chlorure de bis(2-oxo-3-oxazolidinyl)phosphinique, 0.20ml de triéthylamine puis 0.063g de 2-fluoro-1,1-diméthyl-éthylamine préparé selon "Journal of Medecinal Chemistry, 1991, vol.34, 29-37". Après une nuit d'agitation à 20°C, on ajoute de l'eau, extrait avec du dichlorométhane, sépare la phase organique sur une cartouche hydrophobe, purifie par chromatographie sur une colonne de silice, en éluant par le gradient du mélange DCM/MeOH de (100/0 ; v/v) à (98/2 ; v/v), pour donner le produit désiré sous forme de poudre blanche, PF= 182°C.
RMN ¹H 400MHz (DMSO-d₆): 1,22(t,3H) ; 1,36(s,6H); 1,71(s,3H); 3,84-3,93(m,2H); 4,53(s,1H); 4,65(s,1H); 6,36(s,1H); 6,92-6,97(m,1H); 7,22-7,79(m,3H); 7,76-7,80(m,2H); 8,00-8,13(m,4H)

### EXEMPLE 67

### (2-{4-[3-(2-Chloro-phényl)-5-éthoxy-3-méthyl-2-oxo-2,3-dihydro-indole-1-sulfonyl]-benzoylamino}-2-méthyl-propyl)-carbamate de tert-butyle

### A) (2-Amino-2-méthyl-propyl)-carbamate de tert-butyle

A la solution de 1g de 2-méthyl-propane-1,2-diamine commerciale dans 10mL d'acétonitrile refroidie à -0°C, on ajoute 1.24g d'anhydride d'acide *ter*-butyl-carbonique. Après 2h d'agitation à 0°C, on laisse remonter la température à 20°C pendant 1h. On filtre sur fritté, évapore le filtrat et purifie le résidu d'évaporation par chromatographie sur une colonne de silice, en éluant par le gradient du mélange DCM/MeOH de (100/0 ; v/v) à (85/15 ; v/v), pour donner le produit désiré sous forme de cristaux, PF= 70°C.

### B) (2-{4-[3-(2-Chloro-phényl)-5-éthoxy-3-méthyl-2-oxo-2,3-dihydro-indole-1-sulfonyl]-benzoylamino}-2-méthyl-propyl)-carbamate de tert-butyle

Préparé dans des conditions semblables à l'étape B de l'exemple 66 à partir du même acide 4-[3-(2-Chloro-phényl)-5-éthoxy-3-méthyl-2-oxo-2,3-dihydro-indole-1-sulfonyl]-benzoïque et du (2-Amino-2-méthyl-propyl)-carbamate de *tert*-butyle préparé en A.
RMN ¹H 400MHz (DMSO-d₆): 1,20(t,3H) ; 1,29(s,6H); 1,34(s,9H); 3,17-3,20(m,2H); 3,83-3,91 (m,2H); 6,33(s,1H); 6,90-6,95(m,1H); 7,20-7,46(m,3H); 7,72-7,80(m,2H); 8,00-8,07(m,4H)

### EXEMPLE 68

### N-(2-Amino-1,1-diméthyl-éthyl)-4-[3-(2-chloro-phényl)-5-éthoxy-3-méthyl-2-oxo-2,3-dihydro-indole-1-sulfonyl]-benzamide

On obtient le chlorhydrate du composé désiré sous forme de poudre blanche par déprotection du composé de l'exemple 67 dans une solution éthérée d'acide chlorhydrique.
PF = 172°C

Dans des conditions semblables à l'étape B de l'exemple 66, on obtient les exemples rassemblés dans le tableau VII suivant, en partant de l'acide 4-[3-(2-Chloro-phényl)-5-éthoxy-3-méthyl-2-oxo-2,3-dihydro-indole-1-sulfonyl]-benzôique préparé en A de l'exemple 66 et des amines commerciales appropriées.

**Tableau VII**

| | | | | | | | | | |
|---|---|---|---|---|---|---|---|---|---|
| 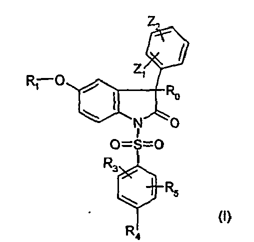 | | | | | | | | | |

| N° | R₀ | R₁ | Z₁ | Z₂ | R₄ | R₃ | R₅ | PF (°C) | MH+ |
|---|---|---|---|---|---|---|---|---|---|
| 69 | Me | Et | 2-Cl | H | 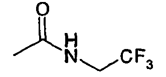 | H | H | 115 | 567 |
| 70 | Me | Et | 2-Cl | H | 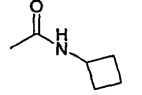 | H | H | 136 | 539 |
| 71 | Me | Et | 2-Cl | H | 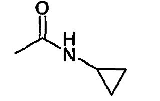 | H | H | 252 | 525 |
| 72 | Me | Et | 2-Cl | H | 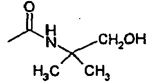 | H | H | 152 | 557 |
| 73 | Me | Et | 2-Cl | H | 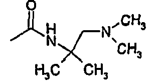 | H | H | 89 | 584 |
| 74 | Me | Et | 2-Cl | H | 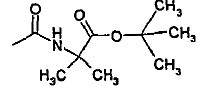 | H | H | 189 | 627 |

### EXEMPLE 75

### N-tert-Butyl-4-{3-[2-chloro-5-(1-(2,2,2-trifluoro-éthyl)-pipéridin-4-ylamine-1-carbonyl)-phényl]-5-éthoxy-3-méthyl-2-oxo-2,3-dihydro-indole-1-sulfonyl}-benzamide

### A) 3-[1-(4-tert-Butylcarbamoyl-benzènesulfonyl)-5-éthoxy-3-méthyl-2-oxo-2,3-dihydro-1H-indol-3-yl]-4-chloro-benzoate de 2,5-dioxo-pyrrolidin-1-yle

A la solution de 3g d'acide 3-[1-(4-*tert*-butylcarbamoyl-benzènesulfonyl)-5-éthoxy-3-méthyl-2-oxo-2,3-dihydro-1*H-*indol-3-yl]-4-chloro-benzoj'que (exemple 48) dans 34ml de tétrahydrofurane, refroidie à 0°C, on ajoute 0.61 g de N-hydroxysuccinimide. Après 20 mn d'agitation à 0°C, on ajoute 1.06g de dicylohexylcarbodiimide. Après une nuit d'agitation, on évapore le solvant sous vide, reprend le résidu avec du dichlorométhane, filtre sur fritté, évapore le filtrat puis purifie le résidu par chromatographie sur une colonne de silice, en éluant par le gradient du mélange DCM/MeOH de (100/0 ; v/v) à (95/5 ; v/v), pour donner le produit désiré sous forme de poudre blanche, PF= 168°C.

### B)N-tert-Butyl-4-{3-[2-chloro-5-(1-(2,2,2-trifluoro-éthyl)-pipéridin-4-ylamine-1-carbonyl)-phényl]-5-éthoxy-3-méthyl-2-oxo-2,3-dihydro-indole-1-sulfonyl}-benzamide

A la solution de 0.2g de 3-[1-(4-tert-Butylcarbamoyl-benzènesulfonyl)-5-éthoxy-3-méthyl-2-oxo-2,3-dihydro-1H-indol-3-yl]-4-chloro-benzoate de 2,5-dioxo-pyrrolidin-1-yle préparé en A dans 2.5ml de tétrahydrofurane, refroidie à 0°C, on ajoute 0.132g de 1-(2,2,2-trifluoro-éthyl)-pipéridin-4-ylamine préparé selon WO 01/29042 p.100 et 0.12ml de triéthylamine. Après une nuit d'agitation à 20°C, on ajoute de l'eau, extrait avec du dichlorométhane, sépare la phase organique sur une cartouche hydrophobe, purifie par chromatographie sur une colonne de silice, en éluant par le gradient du mélange DCM/MeOH de (100/0 ; v/v) à (97/3 ; v/v), pour donner le produit désiré sous forme de poudre blanche, PF= 160°C.
RMN ¹H 400MHz (DMSO-d₆): 1,22(t,3H); 1,38(s,9H); 1,50-1,85(m,7H); 2,40-2,52(m,2H); 2,90-2,97(m,2H); 3,12-3,23(m,2H) ; 3,85-3,94(m,2H); 6,44-6,45(m,1H); 6,92-6,97(m,1H); 7,34-7,38(m,1H); 7,76-8,15(m,7H)

Dans des conditions semblables à l'étape B de l'exemple 75, on obtient les exemples rassemblés dans le tableau VIII suivant, en partant du 3-[1-(4-tert-Butylcarbamoyl-benzènesulfonyl)-5-éthoxy-3-méthyl-2-oxo-2,3-dihydro-1H-indol-3-yl]-4-chloro-benzoate de 2,5-dioxo-pyrrolidin-1-yle préparé en A de l'exemple 75 et des amines, commerciales ou préparées selon des procédés décrits, appropriées.

**Tableau VIII**

| | | | | | | | | | |
|---|---|---|---|---|---|---|---|---|---|
| 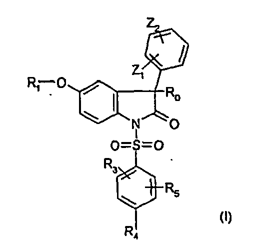 | | | | | | | | | |

| N° | R₀ | R₁ | Z₁ | Z₂ | R₄ | R₃ | R₅ | PF (°C) | MH+ |
|---|---|---|---|---|---|---|---|---|---|
| 76 | Me | Et | 2-Cl | 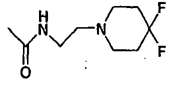 | 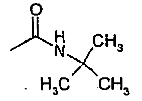 | H | H | 148 | 731 |
| 77 | Me | Et | 2-Cl | 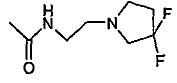 | 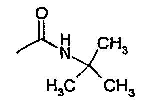 | H | H | 136 | 717 |
| 78 | Me | Et | 2-Cl | 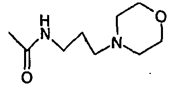 | 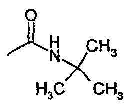 | H | H | 112 | 711 |
| 79 | Me | Et | 2-Cl | 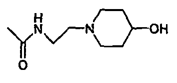 | 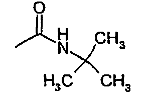 | H | H | 148 | 711 |
| 80 | Me | Et | 2-Cl | 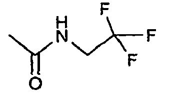 | 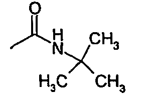 | H | H | 140 | 666 |
| 81 | Me | Et | 2-Cl | 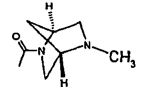 | 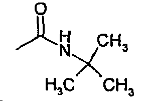 | H | H | 152 | 679 |
| 82 | Me | Et | 2-Cl | 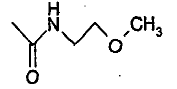 | 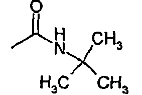 | H | H | 126 | 642 |
| 83 | Me | Et | 2-Cl | 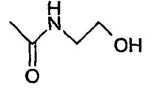 | 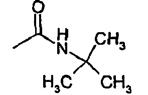 | H | H | 150 | 628 |
| 84 | Me | Et | 2-Cl | 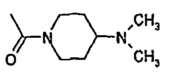 | 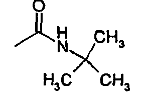 | H | H | 132 | 695 |
| 85 | Me | Et | 2-Cl | 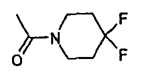 | 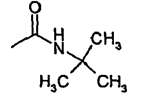 | H | H | 150 | 688 |
| 86 | Me | Et | 2-Cl | 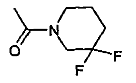 | 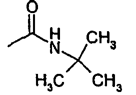 | H | H | 130 | 688 |
| 87 | Me | Et | 2-Cl | 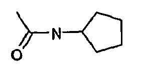 | 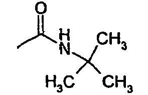 | H | H | 164 | 652 |
| 88 | Me | Et | 2-Cl | 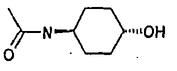 | 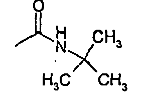 | H | H | 172 | 682 |
| 89 | Me | Et | 2-Cl | 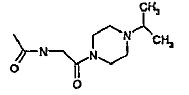 | 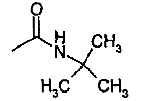 | H | H | 168 | 752 |
| 90 | Me | Et | 2-Cl | 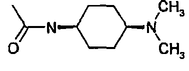 | 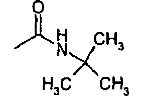 | H | H | 180 | 709 |
| 91 | Me | Et | 2-Cl | 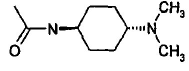 | 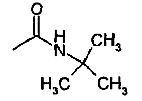 | H | H | 174 | 709 |

### EXEMPLE 92

### 3-[1-(4-tert-Butylcarbamoyl-benzènesulfonyl)-5-éthoxy-3-méthyl-2-oxo-2,3-dihydro-1H-indol-3-yl]-4-chloro-benzoate de (2-diméthylamino)-éthyle

A la solution de 0.2g d'acide 3-[1-(4-*tert*-butylcarbamoyl-benzènesulfonyl)-5-éthoxy-3-méthyl-2-oxo-2,3-dihydro-1*H-*indol-3-yl]-4-chloro-benzoïque (exemple 48) dans 2.5ml de dioxanne, refroidie à 0°C, on ajoute 0.116g de chlorure de bis(2-oxo-3-oxazolidinyl)phosphinique, 0.07ml de N,N-diméthyl-2-hydroxyéthylamine et 0,14mL de triéthylamine. Après une nuit d'agitation à 20°C, on évapore partiellement le solvant sous vide, ajoute 10mL d'une solution aqueuse de NaHCO3, extrait avec de l'acétate d'éthyle, sépare la phase organique que l'on sèche sur sulfate de sodium puis évapore sous vide.

On purifie le résidu par chromatographie sur une colonne de silice en éluant par le gradient du mélange DCM/MeOH de (100/0 ; v/v) à (90/10 ; v/v), pour donner le produit désiré sous forme de poudre blanche, PF= 118°C.
RMN ¹H 400MHz (DMSO-d₆): 1,22(t,3H) ; 1,38(s,9H); 1,77(s,3H); 2,23(s,6H); 2,60-2,67(m,2H); 3,84-3,93(m,2H); 4,33-4,46(m,2H); 6,48-6,50(m,1H); 6,94-6,98(m,1H); 7,43-7,46(m,1H); 7,80-8,15(m,7H)

Dans des conditions semblables à l'exemple 92, on obtient les exemples rassemblés dans le tableau IX suivant, en partant de l'acide 3-[1-(4-*tert*-butylcarbamoyl-benzènesulfonyl)-5-éthoxy-3-méthyl-2-oxo-2,3-dihydro-1*H-*indol-3-yl]-4-chloro-benzoïque (exemple 48) et des alcools commerciaux appropriées.

**Tableau IX**

| | | | | | | | | | |
|---|---|---|---|---|---|---|---|---|---|
| 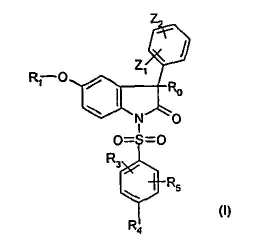 | | | | | | | | | |

| N° | R₀ | R₁ | Z₁ | Z₂ | R₄ | R₃ | R₅ | PF (°C) | MH+ |
|---|---|---|---|---|---|---|---|---|---|
| 93 | Me | Et | 2-Cl | 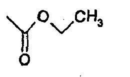 | 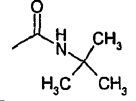 | H | H | 118 | 613 |
| 94 | Me | Et | 2-Cl | 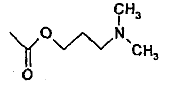 | 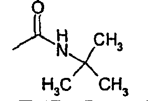 | H | H | 108 | 670 |
| 95 | Me | Et | 2-Cl | 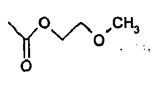 | 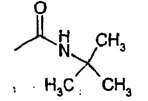 | H | H | 110 | 643 |
| 96 | Me | Et | 2-Cl | 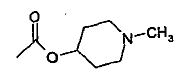 | 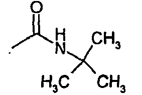 | H | H | 204 | 682 |

Dans les conditions de sulfonylation décrites dans l'exemple 2 et à partir de composés III, issus des préparations 5 à 19 précédents et de chlorure de benzènesulfonyles précédemment décrits, appropriés, on obtient les composés des exemples rassemblés dans le tableau X suivant :

**Tableau X**

| | | | | | | | | | |
|---|---|---|---|---|---|---|---|---|---|
| 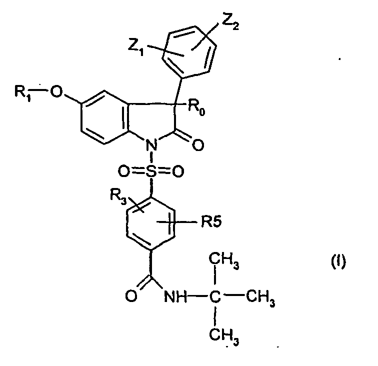 | | | | | | | | | |

| N° | N° de préparation du composé III précurseur | R₀ | R₁ | Z₁ | Z₂ | R₅ | R₃ | PF(°C) | MH⁺ |
|---|---|---|---|---|---|---|---|---|---|
| 97 | 6 dextrogyre | Me | Et | H | H | H | H | 83 | 507 |
| 98 | 6 lévogyre | Me | Et | H | H | H | H | 89 | 507 |
| 99 | 6 dextrogyre | Me | Et | H | H | H | 2-OMe | 148 | 537 |
| 100 | 6 lévogyre | Me | Et | H | H | H | 2-OMe | 151 | 537 |
| 101 | 5 dextrogyre | Me | Et | 2-F | H | H | H | 116 | 525 |
| 102 | 5 lévogyre | Me | Et | 2-F | H | H | H | 114 | 525 |
| 103 | 7 dextrogyre | Me | Et | 3-OMe | H | H | H | 101 | 537 |
| 104 | 7 lévogyre | Me | Et | 3-OMe | H | H | H | 100 | 537 |
| 105 | 7 dextrogyre | Me | Et | 3-OMe | H | H | 2-OMe | 108 | 567 |
| 106 | 8 dextrogyre | Me | Et | 2-OMe | H | H | H | 98 | 537 |
| 107 | 8 lévogyre | Me | Et | 2-OMe | H | H | H | 135 | 537 |
| 108 | 8 dextrogyre | Me | Et | 2-OMe | H | H | 2-OMe | 146 | 567 |
| 109 | 8 lévogyre | Me | Et | 2-OMe | H | H | 2-OMe | 140 | 567 |
| 110 | 9 dextrogyre | Me | Et | 4-Me | H | H | H | 177 | 521 |
| 111 | 9 dextrogyre | Me | Et | 4-Me | H | H | 2-OMe | 207 | 551 |
| 112 | 9 lévogyre | Me | Et | 4-Me | H | H | 2-OMe | 208 | 551 |
| 113 | 10 dextrogyre | Me | Et | 3-Me | H | H | H | 108 | 521 |
| 114 | 10 lévogyre | Me | Et | 3-Me | H | H | H | 93 | 521 |
| 115 | 10 dextrogyre | Me | Et | 3-Me | H | H | 2-OMe | 186 | 551 |
| 116 | 11 dextrogyre | Me | Et | 2-OCF3 | H | H | H | 172 | 591 |
| 117 | 11 lévogyre | Me | Et | 2-OCF3 | H | H | H | 177 | 591 |
| 118 | 11 dextrogyre | Me | Et | 2-OCF3 | H | H | 2-OMe | 213 | 621 |
| 119 | 12 dextrogyre | Me | Et | 2-CF3 | H | H | H | 144 | 575 |
| 120 | 12 lévogyre | Me | Et | 2-CF3 | H | H | H | 144 | 575 |
| 121 | 12 dextrogyre | Me | Et | 2-CF3 | H | H | 2-OMe | 214 | 605 |
| 122 | 13 dextrogyre | Me | Et | 2-Cl | 3-Cl | H | H | 124 | 575 |
| 123 | 13 lévogyre | Me | Et | 2-Cl | 3-Cl | H | H | 170 | 575 |
| 124 | 14 dextrogyre | Me | Et | 2-Cl | 6-F | H | H | 188 | 559 |
| 125 | 14 lévogyre | Me | Et | 2-Cl | 6-F | H | H | 128 | 559 |
| 126 | 14 dextrogyre | Me | Et | 2-Cl | 6-F | H | 2-OMe | 133 | 589 |
| 127 | 15 dextrogyre | Me | Et | 2-Cl | 5-OMe | H | H | 121 | 571 |
| 128 | 15 lévogyre | Me | Et | 2-Cl | 5-OMe | H | H | 161 | 571 |
| 129 | 15 lévogyre | Me | Et | 2-Cl | 5-OMe | H | 2-OMe | 128 | 601 |
| 130 | 16 dextrogyre | Me | Et | 2-OBn | H | H | H | 109 | 613 |
| 131 | 16 dextrogyre | Me | Et | 2-OBn | H | H | 2-OMe | 139 | 643 |
| 132 | 17 dextrogyre | Me | Et | 4-OBn | H | H | H | 142 | 613 |
| 133 | 17 lévogyre | Me | Et | 4-OBn | H | H | H | 143 | 163 |
| 134 | 17 dextrogyre | Me | Et | 4-OBn | H | H | 2-OMe | 113 | 643 |
| 135 | 18 lévogyre | Et | Et | H | H | H | H | 127 | 555 |
| 136 | 18 lévogyre | Et | Et | H | H | H | 2-OMe | 139 | 585 |
| 137 | 19 lévogyre | Pr | Et | H | H | H | H | 146 | 569 |

### EXEMPLE 138

### N-tert-Butyl-4-[5-éthoxy-3-(2-hydroxy-phényl)-3-méthyl-2-oxo-2,3-dihydro-indole-1-sulfonyl]-benzamide

A la solution de 0.3g du composé de l'exemple 130 dans 5ml de méthanol on ajoute 0.06g de poudre de Palladium sur Charbon (10% massique), 0.19g de formiate d'ammonium. Après 8 heures d'agitation à reflux, on refroidit, filtre sur un lit de célite et évapore le solvant sous vide. On reprend le résidu par du dichlorométhane, lave avec une solution aqueuse de chlorure de sodium, sépare la phase organique que l'on sèche sur sulfate de sodium puis évapore sous vide. On purifie le résidu par chromatographie sur une colonne de silice en éluant par le gradient du mélange cyclohexane/AcOEt de (100/0 ; v/v) à (70/30 ; v/v), pour donner le produit désiré sous forme de poudre blanche,
PF= 180°C.
RMN ¹H 400MHz (DMSO-d₆): 1,23(t,3H) ; 1,38(s,9H); 1,49(s,3H); 3,83-3,92(m,2H); 6,36-8,06(m, 11H)

Dans des conditions semblables, à partir des composés des exemples 131, 132, 133 et 134, on obtient les composés des exemples rassemblés dans le tableau XI suivant :

**Tableau XI**

| | | | | | | | | | |
|---|---|---|---|---|---|---|---|---|---|
| 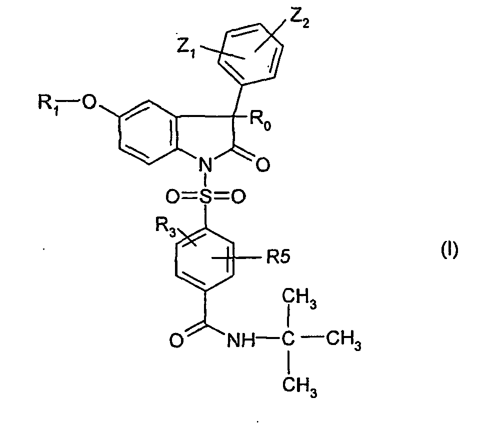 | | | | | | | | | |

| N° ex | N° d'exemple du Précurseur | R₀ | R₁ | Z₁ | Z₂ | R₅ | R₃ | PF(°C) | MH+ |
|---|---|---|---|---|---|---|---|---|---|
| 139 | 131 | Me | Et | 2-OH | H | H | 2-OMe | 203 | 553 |
| 140 | 132 | Me | Et | 4-OH | H | H | H | 137 | 523 |
| 141 | 133 | Me | Et | 4-OH | H | H | H | 134 | 523 |
| 142 | 134 | Me | Et | 4-OH | H | H | 2-OMe | 137 | 553 |

### EXEMPLE 143

### N-tert-Butyl-4-[3-(2-chloro-phényl)-3-méthyl-2-oxo-5-(2,2,2-trifluoro-éthoxy)-2,3-dihydro-indole-1-sulfonyl]-benzamide

### A) N-tert-Butyl-4-[3-(2-chloro-phényl)-5-hydroxy-3-méthyl-2-oxo-2,3-dihydro-indole-1-sulfonyl]-benzamide

A une solution de 3 g de N-tert-Butyl-4-[3-(2-chloro-phényl)-5-éthoxy-3-méthyl-2-oxo-2,3-dihydro-indole-1-sulfonyl]-benzamide (composé de l'exemple 12) en solution dans 75 ml de DCM, refroidie à 0°C, on ajoute 27,72 ml d'une solution de tribromure de bore 1 M dans du DCM. On laisse sous agitation à 20°C pendant 16 heures. On coule le milieu sur de la glace et ajoute lentement 45 ml de triéthylamine. La phase organique est lavée à l'eau, séchée sur sulfate de sodium et concentrée sous pression réduite. On purifie le résidu par chromatographie sur une colonne de silice en éluant par le gradient du mélange DCM/MeOH de (100/0 ; v/v) à (97/3 ; v/v), pour donner le produit désiré sous forme de poudre blanche, PF= 262°C.
MH+ = 513

### B) N-tert-Butyl-4-[3-(2-chloro-phényl)-3-méthyl-2-oxo-5-(2,2,2-trifluoro-éthoxy)-2,3-dihydro-indole-1-sulfonyl]-benzamide

A une solution de 0.18 g de N-tert-Butyl-4-[3-(2-chloro-phényl)-5-hydroxy-3-méthyl-2-oxo-2,3-dihydro-indole-1-sulfonyl]-benzamide en solution dans 3ml d'acétonitrile, refroidie à 0°C, on ajoute 0,137g de carbonate de césium puis 0.098g de trifluorométhanesulfonate de 2,2,2-trifluoroéthyle. On laisse sous agitation à 20°C pendant 16 heures puis on évapore partiellement le solvant sous vide. Le résidu est repris par du dichlorométhane et lavé avec une solution aqueuse de chlorure de sodium. La phase organique est séchée sur sulfate de sodium et concentrée sous pression réduite. On purifie le résidu par chromatographie sur une colonne de silice en éluant par le gradient du mélange cyclohexane/dichlorométhane de (30/70 ; v/v) à (0/100 ; v/v), pour donner le produit désiré sous forme de poudre blanche, PF= 134°C.
RMN ¹H 400MHz (DMSO-d₆): 1,39(s,9H); 1,72(s,3H); 4,62-4,70(m,2H); 6,57-6,59(m,1H); 7,08-7,5(m,4H); 7,75-8,12(m,6H)
MH+ = 595

### EXEMPLE 144

### N-tert-Butyl-4-[3-(2-chloro-phényl)-3-méthyl-2-oxo-5-hydroxy)-2,3-dihydro-indole-1-sulfonyl]-3-méthoxy-benzamide

Obtenu de manière semblable à l'étape A de l'exemple 143, à partir du composé de l'exemple 15.
PF= 166°C
RMN07/06/327 RMN 1H 400MHz (DMSO-d6): 1,38(s,9H); 1,65(s,3H); 3,69(s,3H); 6,16-6,17(m,1H); 6,73-6,77(m,1H); 7,29-8,05(m,8H)
MH+= 543

### EXEMPLE 145

### N-tert-Butyl-4-{3-[3-(3-diméthylamino-propoxy)-phényl]-5-éthoxy-3-méthyl-2-oxo-2,3-dihydro-indole-1-sulfonyl}-benzamide

### A) N-tert-Butyl-4-[3-(3-hydroxy-phényl)-5-éthoxy-3-méthyl-2-oxo-2,3-dihydro-indole-1-sulfonyl]-benzamide

A une solution de 0,98 g de N-tert-Butyl-4-[3-(3-méthoxy-phényl)-5-éthoxy-3-méthyl-2-oxo-2,3-dihydro-indole-1-sulfonyl]-benzamide (composé de l'exemple 103) en solution dans 30 ml de DCM, refroidie à 0°C, on ajoute 3.66 ml d'une solution de tribromure de bore 1M dans du DCM. On laisse sous agitation à 0°C pendant 30 mn puis on coule le milieu sur de la glace et ajoute lentement 1,5 ml de triéthylamine. La phase organique est lavée à l'eau, séchée sur sulfate de sodium et concentrée sous pression réduite. On purifie le résidu par chromatographie sur une colonne de silice en éluant par le gradient du mélange cyclohexane/AcOEt de (100/0 ; v/v) à (70/30 ; v/v), pour donner le produit désiré sous forme de poudre blanche, PF= 196°C.
MH+ = 523

### B) N-tert-Butyl-4-{3-[3-(3-chloropropoxy)-phényl]-5-éthoxy-3-méthyl-2-oxo-2,3-dihydro-indole-1-sulfonyl}-benzamide TLC.ALF6.132

A la solution de 0.2 g de N-tert-Butyl-4-[3-(3-hydroxy-phényl)-5-éthoxy-3-méthyl-2-oxo-2,3-dihydro-indole-1-sulfonyl]-benzamide en solution dans 4ml d'acétonitrile, refroidie à 0°C, on ajoute 0,15g de carbonate de césium puis 0.094g de 1-chloro-3-iodopropane.

On laisse sous agitation à 20°C pendant 16 heures puis on ajoute une solution aqueuse de chlorure de sodium. La phase organique est séchée sur sulfate de sodium et concentrée sous pression réduite. On purifie le résidu par chromatographie sur une colonne de silice en éluant par le gradient du mélange cyclohexane/AcOEt de (100/0 ; v/v) à (60/40 ; v/v), pour donner le produit désiré.
RMN ¹H 400MHz (DMSO-d₆): 1,27(t,3H) ; 1,37(s,9H); 1,64(s,3H); 2,08-2,16(m,2H);3,75(t,2H); 3,94-4,02(m,4H); 6,50-8,05(m,11H)
MH+ = 599

### C) N-tert-Butyl-4-{3-[3-(3-diméthylamino-propoxy)-phényl]-5-éthoxy-3-méthyl-2-oxo-2,3-dihydro-indole-1-sulfonyl}-benzamide SAR127943-1 ALF6.151

Dans un autoclave, on chauffe vers 60°C, pendant 24 heures, le mélange de 0.07g de N-tert-Butyl-4-{3-[3-(3-chloropropoxy)-phényl]-5-éthoxy-3-méthyl-2-oxo-2,3-dihydro-indole-1-sulfonyl}-benzamide, 2,5ml de DMF, 0.018g d'iodure de sodium, 0.038g de carbonate de sodium et 1.2ml d'une solution de diméthylamine 2M dans le THF. On ajoute de l'eau, extrait avec de l'acétate d'éthyle, sèche la phase organique, évapore à sec et purifie le résidu par chromatographie sur une colonne de silice en éluant par le gradient du mélange DCM/MeOH de (100/0 ; v/v) à (85/15 ; v/v), pour donner le produit désiré, PF= 86°C.
RMN ¹H 400MHz (DMSO-d₆): 1,27(t,3H) ; 1,37(s,9H); 1,64(s,3H); 1,77-1,85(m,2H); 2,19(s,6H) ; 2,32-2,46(m,2H) ; 3,80-4,01 (m,4H); 6,49-8,03(m,11H)
MH+ = 608

Les composés de l'invention ont fait l'objet d'essais pharmacologiques qui ont montré leur intérêt comme substances actives en thérapeutique.

Ils ont notamment été testés quant à leurs effets. Plus particulièrement, l'affinité des composés de l'invention pour les récepteurs V₂ de la vasopressine, a été déterminée dans un test de liaison *in vitro,* selon la technique décrite ci-dessous.
Dans ce qui suit.
- EDTA = ethylendiaminetetraacetic acid (acide éthylènediaminotétraacétique, en français),
- BSA = Bovine Serum Albumin (albumine de sérum bovin, en français),
- AVP = vasopressine,
- DMSO = diméthylsulfoxyde.

### A. Mesure d'affinité in vitro - CI₅₀ :

La mesure de l'affinité des composés de l'invention pour les récepteurs V₂ de la vasopressine a été réalisée dans des tests de liaison *in vitro,* comme décrit dans J. Pharmacol. Exp. Ther., (2002), 300: pp. 1122-1130.

Les membranes plasmatiques (environ 20 µg/mL) provenant de tissus ou lignée cellulaire CHO exprimant les récepteurs recombinants humains V₂ de la vasopressine, sont incubées pendant 45 minutes à 25 °C dans 200 µL de tampon TRIS-HCl (50mM ; pH 8,2) contenant 2 mM de MgCl₂, 1 mM d'EDTA, 0,1 % de BSA, 1/500 protease inhibitor cocktail (Sigma #P2714) et 3,5 nM de [H3]-AVP. La réaction est arrêtée par filtration et lavage sur filtres GF/B. La liaison non spécifique est déterminée en présence de 1 µM d'AVP. Les composés de l'invention, préalablement dissous à la concentration de 10⁻² M dans du DMSO, sont testés en gamme de dilution.

Pour chaque concentration les résultats sont exprimés en pourcentage d'inhibition de la liaison spécifique. Une CI₅₀ (concentration de produit inhibant 50% de la liaison spécifique) est déterminée pour chacun des produits en utilisant le logiciel interne Biost@t-SPEED v1.3 qui met en oeuvre le modèle logistique à 4 parametres de Ratkovsky et Reedy (1986). L'ajustement est obtenu par regression non linéaireà l'aide de l'algorithme de Marquardt du logiciel SAS v8.2 tournant sous UNIX.

L'affinité des composés de l'invention pour les récepteurs V₁ₐ et V_{1b} de la vasopressine et l'affinité des composés de l'invention pour les récepteurs de l'ocytocine (OT) a également été testée.

L'affinité des composés selon l'invention pour les récepteurs OT a été déterminée dans un test de liaison *in vitro* en utilisant la méthode décrite par J. Elands et al. dans Eur. J . Pharmacol. 1987, 147, 197-207. Cette méthode conciste à étudier in vitro le déplacement d'un analogue radioiodé de l'ocytocine aux récepteurs de l'ocytocine dans une préparation membranaire de récepteurs ocytociques humains.
L'affinité des composés selon l'invention pour les récepteurs humains V₁ₐ a été déterminée selon la méthode décrite par M Thibonnier et al. dans J. Biol. Chem. 1994, 269, 3304-3310. L'affinité des composés selon l'invention pour les récepteurs V_{1b} a été déterminée selon la méthode décrite par T. Sugimoto et al. dans J. Biol. Chem. 1994, 269, 27088-27092.
Comme indiqué dans le tableau A ci-dessous, les composés de la présente invention sont très affins et sélectifs pour les récepteurs V₂ de la vasopressine.
Les composés obtenus selon des exemples de la présente invention, donnés dans le tableau A ci-dessous, ne sont pas limitatifs et ne font qu'exemplifier l'invention.

**Tableau A**

| **N° ex.** | **CI50 V₂ (nM)** | **CI50 OT (nM)** | **CI50 V₁ₐ (nM)** | **CI50 V_{1b} (nM)** | **CI50 OT / CI50V₂** | **CI50 V₁ₐ / CI50V₂** | **CI50 V_{1b} / CI50V₂** |
|---|---|---|---|---|---|---|---|
| 12 | 1,9 | 140 | 450 | >1000 | 74 | >100 | >100 |
| 21 | 3,0 | >1000 | 340 | >1000 | >100 | >100 | >100 |
| 36 | 5,2 | 350 | >1000 | >1000 | 67 | >100 | >100 |
| 43 | 1,3 | 700 | 730 | >1000 | >100 | >100 | >100 |
| 62 | 2,5 | >1000 | 630 | >1000 | >100 | >100 | >100 |
| 72 | 1,3 | 390 | >1000 | 940 | >100 | >100 | >100 |
| 99 | 1,0 | 700 | 260 | >1000 | >100 | >100 | >100 |
| 144 | 2,6 | 130 | 310 | >1000 | 50 | >100 | >100 |

Afin d'illustrer la sélectivité des composés selon l'invention, les affinités mesurées sur les récepteurs V₁ₐ, V_{1b} et OT ont été comparées à celle mesurée sur le récepteur V₂ dans le tableau A ci-dessus. On considère que lorsque les CI₅₀ sont supérieures à 1 µM (1000 nM), les composés sont peu affins pour le récepteur testé.

La sélectivité des composés selon l'invention pour les récepteur V₂, peut être mise en évidence par les ratios calculés entre les différentes valeurs de CI₅₀ mesurées sur chaque récepteur et la CI₅₀ mesurée sur le récepteur V₂ : plus ce rapport est grand, plus les composés selon l'invention sont sélectifs des récepteurs V₂ . En l'occurrence, comme cela est montré dans le tableau A, les ratios de CI₅₀ des composés selon l'invention sont très supérieurs à 10, montrant ainsi leur sélectivité.

Les composés selon l'invention, affins et sélectifs des récepteurs V₂, présentent de bonnes propriétés pharmacologiques et sont particulièrement aptes à être utilisés pour la préparation de médicaments, en particulier de médicaments antagonistes de la liaison aux récepteurs V₂.

Selon un autre de ses aspects, l'invention a donc pour objet des médicaments qui comprennent au moins un composé de formule (I).

Les composés antagonistes des récepteurs V₂ de la vasopressine présentent des propriétés aquarétiques chez l'animal et chez l'homme (Cardiovascular Drug Review, (2001), 3: pp. 201-214). Ainsi, les composés selon l'invention possèdent un large éventail d'indications thérapeutiques et peuvent remplacer avantageusement les diurétiques classiques dans toutes les pathologies où ils sont préconisés chez l'homme et chez l'animal.

Ainsi, les composés selon l'invention peuvent être utiles notamment dans le traitement et/ou la prévention des affections des systèmes nerveux central et périphérique, du système cardiovasculaire, du système endocrinien et hépatique, de la sphère rénale, de la sphère gastrique, intestinale et pulmonaire, en ophtalmologie et dans les troubles du comportement sexuel, chez l'homme et chez l'animal.

Plus particulièrement, les composés selon l'invention peuvent être utilisés dans le traitement et/ou la prévention de différentes affections vasopressine-dépendantes ainsi que dans les dysfonctionnements de la sécrétion de la vasopressine comme le syndrome inapproprié de sécrétion de vasopressine (ou « SIADH », pour Syndrome of Inappropriate ADH Sécrétion), les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et l'angioplastie coronarienne transluminale percutanée (ou « PTCA », pour Percutaneous Transluminal Coronary Angioplasty), l'ischémie cardiaque, les dérèglements de l'hémostase notamment l'hémophilie, le syndrome de Von Willebrand ; les affections du système nerveux central, la douleur, la migraine, le vasospasme cérébral, l'hémorragie cérébrale, les oedèmes cérébraux, la dépression, l'anxiété, la boulimie, les états psychotiques, les troubles de la mémoire par exemple ; les rinopathies et les dysfonctionnement rénaux comme les oedèmes, le vasospasme rénal, la nécrose du cortex rénal, le syndrome néphrotique, les polykystoses rénales dans leurs différentes formes chez l'enfant et chez l'adulte (ou « PKD », pour Polycystic Kidney Disease), les hyponatriémies et, l'hypokaliémie, le diabète, les néphropathies diabétiques, le diabète insipide néphrogénique (NDI), le « NSIADH » (pour Nephrogenic Syndrome of Inappropriate Antidiuresis), le syndrome de Schwartz-Bartter ou la lithiase rénale, les infections urinaires ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, le diabète insipide et l'énurésie ; les affections du système hépatique tel que les cirrhoses du foie ; les ascites abdominales et tous les désordres provoquant une rétention d'eau anormale; les désordres surrénaliens (maladie de Cushing) et en particulier l'hypercorticisme et l'hyperaldosternonémie. Les composés selon l'invention peuvent également être utilisés dans le traitement et/ou la prévention des troubles du comportement sexuel, dans la surcharge pondérale ou l'excès de poids et l'obésité en remplaçant avantageusement les diurétiques classiques déjà utilisés pour cette indication. Chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatriémiques, de la maladie de Raynaud, du syndrome pulmonaire, du glaucome et de la prévention de la cataracte, dans les traitements post-opératoires, notamment après une chirurgie abdominale, cardiaque ou hémorragique et dans les traitements de troubles ou de maladies de l'oreille interne tels que la maladie de Ménière, accouphènes, les vertiges, les difficultés d'audition, notamment dans les graves, ou bourdonnements, les hydrops et notamment les hydrops endolymphatiques, l'ostéoporose.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé de formule (I) selon l'invention, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'exemple de l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,5 mg à 800 mg de principe actif par individu, plus particulièrement de 0,5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention concerne également une méthode de traitement et/ou de prévention des pathologies ci-dessus indiquées qui comprend l'administration, à un patient, d'une dose efficace d'un composé selon l'invention, ou d'un de ses hydrates ou solvats.

## Revendications

1. Composé répondant à la formule générale (I) : 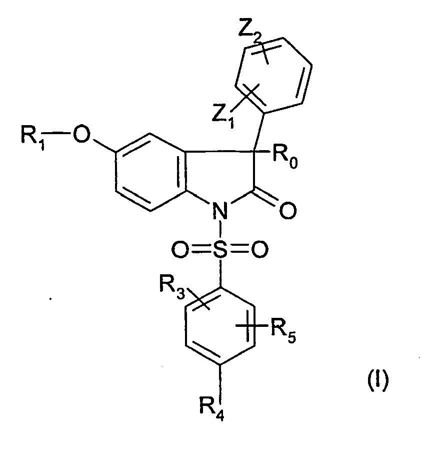 dans laquelle
• R₀ représente un groupe (C₁-C₄)alkyle, un groupe mono ou polyfluoro-(C₁-C₄)alkyle, un groupe -(CH₂)ₙ-cyclopropyle,
. ou bien R₀ représente un groupe (C₂-C₄)alcényle ou (C₂-C₄)alcynyle ;
• R₁ représente un atome d'hydrogène, un groupe (C₁-C₅)alkyle, un groupe mono ou polyfluoro-(C₁-C₅)alkyle, un groupe hydroxy-(C₁-C₅)alkyle, un groupe -(CH₂)ₘ-(C₃-C₅)cycloalkyle ;
• Z₁ représente un atome d'hydrogène ou d'halogène, un groupe (C₁-C₄)alkyle, un groupe mono ou polyfluoro-(C₁-C₄)alkyle, un groupe (C₁-C₄)alcoxy, un groupe mono ou polyfluoro-(C₁-C₄)alcoxy, un groupe -(CH₂)ₙ-cyclopropyle, ledit groupe cyclopropyle étant non substitué ou substitué par un ou plusieurs atomes de fluor ;
• Z₂ représente un atome d'halogène ou un groupe T₁W, dans lequel T₁ représente un groupe -(CH₂)ₙ- et W représente un atome d'hydrogène, un groupe (C₁-C₄)alkyle, mono ou polyfluoro-(C₁-C₄)alkyle ou un groupe cyclopropyle non substitué ou substitué par un ou plusieurs atomes de fluor,
■ ou bien W représente un groupe -C(O)NR₆R₇ dans lequel R₆ et R₇ représentent indépendamment l'un de l'autre un atome d'hydrogène, un groupe (C₁-C₆)alkyle, un groupe mono ou polyfluoro-(C₁-C₆)alkyle, un groupe -(CH₂)ₘ-(C₃-C₆)cycloalkyle ledit groupe cycloalkyle étant non substitué ou substitué par un ou plusieurs atomes de fluor , un hydroxy, un groupe NRR',
. ou bien R₆ et R₇ représentent indépendamment l'un de l'autre un groupe -(CH₂)ₚ-pyrrolidinyle, -(CH₂)ₚ-pipéridyle, -(CH₂)ₚ-pyridyle, lesdits groupes pyrrolidinyle, pipéridyle et pyridyle étant non substitués ou substitués par un ou plusieurs atomes d'halogène, un groupe (C₁-C₄)alkyle, un groupe mono ou polyfluoro(C₁-C₄)alkyle, un benzyle ou par un groupe -OR,
. ou bien R₆ et R₇ représentent indépendamment l'un de l'autre un groupe -(CH₂)_{q}-NRₐR_{b1}
. ou bien R₆ et R₇ représentent indépendamment l'un de l'autre un groupe -(CH₂)ₛ-C(O)NRₐR_{b},
. ou bien R₆ et R₇ représentent indépendamment l'un de l'autre un groupe -(CH₂)_{q}-OR,
. ou bien R₆ et R₇ forment ensemble avec l'atome d'azote auquel ils sont rattachés un hétérocycle monocyclique non substitué ou substitué par un ou plusieurs atomes de fluor, un ou plusieurs groupes (C₁-C₄)alkyle, mono ou polyfluoro-(C₁-C₄)alkyle, -NR'R, ou -OR,
. ou bien R₆ et R₇ forment ensemble avec l'atome d'azote auquel ils sont rattachés un hétérocycle bicyclique non substitué ou substitué par un ou plusieurs atomes de fluor, (C₁-C₄)alkyle, mono ou polyfluoro-(C₁-C₄)alkyle, -OR ou -NR'R ;
■ ou bien W représente un groupe -NR₈C(O)R₉ dans lequel :
. R₈ représente un atome d'hydrogène, un groupe (C₁-C₄)alkyle, un groupe mono ou polyfluoro-(C₁-C₄)alkyle,
. R₉ représente un atome d'hydrogène, un groupe (C₁-C₄)alkyle, un groupe mono ou polyfluoro(C₁-C₄)alkyle, -(CH₂)ᵣ-NR_{d}Rₑ, -(CH₂)ₘ-pyrrolidinyle, -(CH₂)ₘ-pipéridyle, -(CH₂)ₘ-pyridyle, lesdits groupes pyrrolidinyle, pipéridyle, ou pyridyle étant non substitués ou substitués par un ou plusieurs groupes (C₁-C₄)alkyle, halogène, mono ou polyfluoro-(C₁-C₄)alkyle, benzyle ;
■ ou bien W représente un groupe -NR₁₀R₁₁ dans lequel R₁₀ et R₁₁ représentent indépendamment l'un de l'autre un atome d'hydrogène, un groupe hydroxy, un (C₁-C₆)alkyle ou un groupe mono ou polyfluoro-(C₁-C₆) alkyle,
. ou bien R₁₀ et R₁₁ forment ensemble avec l'atome d'azote auquel ils sont rattachés un hétérocycle monocyclique non substitué ou substitué par un ou plusieurs groupes (C₁-C₄)alkyle, un groupe mono ou polyfluoro-(C₁-C₄)alkyle ou oxo ;
■ ou bien W représente un groupe -OR₁₂ dans lequel R₁₂ représente un atome d'hydrogène, un groupe (C₁-C₄)alkyle, un groupe mono ou polyfluoro-(C₁-C₄)alkyle, un benzyle ou un groupe -(CH₂)_{q}-NR'R ;
■ ou bien W représente un groupe -C(O)O R₁₉
• R₄ représente un groupe (C₁-C₄)alkyle, un groupe mono ou polyfluoro-(C₁-C₄)alkyle, un groupe -OR, (C₂-C₄) alcényle, nitro, COOR_{c}, un benzyloxy,
. ou bien R₄ représente un groupe -C(O)NR₁₃R₁₄ dans lequel R₁₃ et R₁₄ représentent indépendamment l'un de l'autre un atome d'hydrogène, un groupe (C₁-C₆)alkyle, ledit groupe alkyle étant non substitué ou substitué par un ou plusieurs atomes de fluor, un hydroxyl, un groupe NRR', un groupe (C₁-C₆)alkyloxycarbonylamino, un groupe (C₁-C₆)alkyloxycarbonyl, ou dans lequel R₁₃ et R₁₄ représentent indépendamment l'un de l'autre, un groupe -(CH₂)ₙ-(C₃-C₆)cycloalkyle, ledit groupe cycloalkyle étant non substitué ou substitué par un ou plusieurs atomes de fluor,
. ou bien R₄ représente un groupe -NR₁₅R₁₆ dans lequel R₁₅ et R₁₆ représentent indépendamment l'un de l'autre un atome d'hydrogène, un hydroxy, un groupe (C₁-C₄)alkyle , un groupe mono ou polyfluoro-(C₁-C₄)alkyle, un groupe -(CH₂)ₙ-(C₃-C₅)cycloalkyle non substitué ou substitué par un ou plusieurs atomes de fluor,
. ou bien R₁₅ et R₁₆ forment ensemble avec l'atome d'azote auquel ils sont rattachés, un hétérocycle monocyclique,
. ou bien R₄ représente un groupe -NR₁₇C(O)R₁₈ dans lequel:
. R₁₇ représente un atome d'hydrogène, un groupe (C₁-C₄)alkyle, un groupe mono ou polyfluoro-(C₁-C₄)alkyle,
. R₁₈ représente un groupe (C₁-C₆)alkyle, un groupe mono ou polyfluoro-(C₁-C₆)alkyle, -(CH₂)ₙ-(C₃-C₆)cycloalkyle, un groupe -NR_{d}Rₑ, un groupe phényle, ledit groupe phényle étant lui-même non substitué ou susbtitué par un ou plusieurs groupes (C₁-C₄)alkyle, un groupe mono ou polyfluoro-(C₁-C₄)alkyle ;
. R₁₉ représente un atome d'hydrogène, un groupe (C₁-C₆)alkyle, un groupe mono ou polyfluoro-(C₁-C₆)alkyle, un groupe -(CH₂)_{q}-NRₐR_{b}, un groupe -(CH₂)_{q}-OR, un groupe -(CH₂)ₚ-pyrrolidinyle ou un groupe -(CH₂)ₚ-pipéridyle, lesdits groupes pyrrolidinyle et pipéridyle étant non substitués ou substitués par un ou plusieurs atomes de fluor, un groupe (C₁-C₄)alkyle, un groupe mono ou polyfluoro(C₁-C₄)alkyle.
• R₃ et R₅ représentent indépendamment l'un de l'autre un atome d'hydrogène, d'halogène, un groupe (C₁-C₄)alkyle, un groupe mono ou polyfluoro-(C₁-C₄)alkyle, un groupe (C₁-C₄)alcoxy ou mono ou polyfluoro-(C₁-C₄)alcoxy ;
• Rₐ et R_{b} représentent indépendamment l'un de l'autre :
. un atome d'hydrogène, un groupe (C₁-C₄)alkyle, un groupe mono ou polyfluoro-(C₁-C₄)alkyle, un groupe -(CH₂)ₙ-cyclopropyle, ledit cyclopropyle étant non substitué ou substitué par un ou plusieurs atomes de fluor,
. ou bien Rₐ et R_{b} forment ensemble avec l'atome d'azote auquel ils sont rattachés un groupe hétérocycle monocycle, ledit groupe hétérocycle monocycle étant non substitué ou substitué par un ou plusieurs groupes hydroxyle, (C₁-C₄)alkyle, mono ou polyfluoro-(C₁-C₄)alkyle, (C₁-C₄)alcoxy, mono ou polyfluoro(C₁-C₄)alcoxy, ou par un groupe -NRR' ;
• R' et R représentent indépendamment l'un de l'autre un atome d'hydrogène, un groupe (C₁-C₄)alkyle ou mono ou polyfluoro-(C₁-C₄)alkyle ;
• R_{c} représente un atome d'hydrogène, un groupe (C₁-C₄)alkyle, mono ou polyfluoro-(C₁-C₄)alkyle ou un benzyle ;
• R_{d} et Rₑ représentent indépendamment l'un de l'autre un atome d'hydrogène, d'halogène, un groupe (C₁-C₆)alkyle ou mono ou polyhalogéno-(C₁-C₆) alkyle,
. ou bien R_{d} et Rₑ forment ensemble avec l'atome d'azote auquel ils sont rattachés un groupe hétérocycle monocycle, non substitué ou substitué par un ou plusieurs atomes de fluor, un ou plusieurs groupes (C₁-C₄)alkyle ou mono ou polyfluoro-(C₁-C₄)alkyle ou un groupe -OR ;
• m peut représenter la valeur 0, 1 ou 2 ;
• n peut représenter la valeur 0 ou 1 ;
• p peut représenter la valeur 0, 1, 2 ou 3 ;
• q peut représenter la valeur 2, 3, 4 ou 5 ;
• r peut représenter la valeur 0, 1, 2, 3 ou 4 ;
• s peut représenter la valeur 1, 2 ou 3 ;
à l'état de base, d'hydrate ou de solvat, sous forme d'isomères cis/trans ou de leurs mélanges.

2. Composé de formule générale (I) selon la revendication 1, **caractérisé en ce que** R₀ représente un groupe (C₁-C₃)alkyle, les autres groupes étant tels que définis pour le composé de formule générale (I) ; à l'état de base, d'hydrate ou de solvat, sous forme d'isomères cis/trans ou de leurs mélanges.

3. Composé de formule générale (I) selon l'une quelconque des revendications 1 ou 2, **caractérisé en ce que** R₀ représente un groupe (C₁-C₃)alkyle et R₁ représente un groupe (C₁-C₅)alkyle, les autres groupes étant tels que définis pour le composé de formule générale (I) ; à l'état de base, d'hydrate ou de solvat, sous forme d'isomères cis/trans ou de leurs mélanges.

4. Composé de formule générale (I) selon l'une quelconque des revendications 1, 2 ou 3, **caractérisé en ce que** R₀ représente un groupe méthyle et R₁ représente un groupe (C₁-C₂)alkyle, les autres groupes étant tels que définis pour le composé de formule générale (I) ; à l'état de base, d'hydrate ou de solvat, sous forme d'isomères cis/trans ou de leurs mélanges.

5. Composé de formule générale (I) selon l'une quelconque des revendications 1 à 4, **caractérisé en ce que** Z₁ représente un atome d'halogène, les autres groupes étant tels que définis pour le composé de formule générale (I) ; à l'état de base, d'hydrate ou de solvat, sous forme d'isomères cis/trans ou de leurs mélanges.

6. Composé de formule générale (I) selon l'une quelconque des revendications 1 à 5, **caractérisé en ce que** Z₂ représente un groupe T₁W, dans lequel T₁ représente un groupe -(CH₂)ₙ- avec n égal à 0 et W représente :
■ un groupe -C(O)OR₁₉,
■ un groupe -C(O)NR₆R₇,
dans lesquels R₁₉, R₆ et R₇ et les autres groupes sont tels que définis pour le composé de formule générale (I) ; à l'état de base, d'hydrate ou de solvat, sous forme d'isomères cis/trans ou de leurs mélanges.

7. Composé de formule générale (I) selon l'une quelconque des revendications 1 à 6, **caractérisé en ce que** Z₁ est en position -2 ; à l'état de base, d'hydrate ou de solvat, sous forme d'isomères cis/trans ou de leurs mélanges.

8. Composé de formule générale (I) selon l'une quelconque des revendications 1 à 7, **caractérisé en ce que** Z₁ est en position -2 et Z₂ est en position -5 ; à l'état de base, d'hydrate ou de solvat, sous forme d'isomères cis/trans ou de leurs mélanges.

9. Composé de formule générale (I) selon l'une quelconque des revendications 1 à 8, **caractérisé en ce que** R₄ représente un groupe -C(O)NR₁₃R₁₄, dans lequel R₁₃, R₁₄ et les autres groupes étant tels que définis pour le composé de formule générale (I) ; à l'état de base, d'hydrate ou de solvat, sous forme d'isomères cis/trans ou de leurs mélanges.

10. Composé de formule générale (I) selon l'une quelconque des revendications 1 à 9 **caractérisé en ce que** Z₂ représente un groupe T₁W, dans lequel T₁ représente un groupe -(CH₂)ₙ- avec n égal à 0 et W représente un groupe -OR₁₂ dans lequel R₁₂ représente un atome d'hydrogène ; à l'état de base, d'hydrate ou de solvat, sous forme d'isomères cis/trans ou de leurs mélanges.

11. Composé de formule générale (I) selon l'une quelconque des revendications 1 à 10 **caractérisé en ce que** Z₁ est en position -2 et Z₂ représente un halogène ; à l'état de base, d'hydrate ou de solvat, sous forme d'isomères cis/trans ou de leurs mélanges.

12. Composé de formule générale (I) selon la revendication 1 choisi parmi :
N-tert-Butyl-4-[3-(2-chloro-phényl)-5-éthoxy-3-méthyl-2-oxo-2,3-dihydro-indole-1-sulfonyl]-benzamide (ex.2)
N-tert-Butyl-4-[3-(2-fluoro-phényl)-5-éthoxy-3-méthyl-2-oxo-2,3-dihydro-indole-1-sulfonyl]-benzamide (ex.6)
3-[1-(4-tert-Butylcarbamoyl-benzènesulfonyl)-5-éthoxy-3-méthyl-2-oxo-2,3-dihydro-1H-indol-3-yl]-4-chloro-benzoate de méthyle(ex.9)
N-tert-Butyl-4-[3-(2-chloro-phényl)-5-éthoxy-3-méthyl-2-oxo-2,3-dihydro-indole-1-sulfonyl]-benzamide (ex.12)
N-Cyclopentyl-4-[3-(2-chloro-phényl)-5-éthoxy-3-méthyl-2-oxo-2,3-dihydro-indole-1-sulfonyl]-benzamide (ex.13)
N-tert-Butyl-3-méthoxy-4-[3-(2-chloro-phényl)-5-éthoxy-3-méthyl-2-oxo-2,3-dihydro-indole-1-sulfonyl]-benzamide (ex.15)
N-tert-Butyl-3-méthoxy-4-[3-(2-fluoro-phényl)-5-éthoxy-3-méthyl-2-oxo-2,3-dihydro-indole-1-sulfonyl]-benzamide (ex.16)
Acide 3-[1-(4-tert-Butylcarbamoyl-2-méthoxy-benzènesulfonyl)-5-éthoxy-3-méthyl-2-oxo-2,3-dihydro-1H-indol-3-yl]-4-chloro-benzoïque (ex.19)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-éthyl-benzamide(ex.21)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-pyridyl)-benzamide(ex.22)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(3-diméthylamino-propyl)-benzamide(ex.25)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(1-méthyl-pipéridin-4-yl)-benzamide(ex.28)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-pyrrolidin-1-yl-éthyl)-benzamide(ex.29)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-diéthylamino-éthyl)-benzamide(ex.33)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(2-morpholin-4-yl-éthyl)-benzamide(ex.36)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-[2-(4-méthyl-pipérazin-1-yl)-éthyl]-benzamide(ex.37)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-[3-(4-méthyl-pipérazin-1-yl)-propyl]-benzamide(ex.40)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-(R)-pyrrolidin-3-yl-benzamide(ex.43)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-pipéridin-4-yl-benzamide(ex.44)
4-Chloro-3-[1-(4-tert-butylcarbamoyl-2-méthoxy-benzènesulfonyl)-5-éthoxy-3-méthyl-2-oxo-2,3-dihydro-1H-indol-3-yl]-N-2-amino-éthyl-benzamide(ex.47)
Acide 3-[1-(4-tert-butylcarbamoyl-benzènesulfonyl)-5-éthoxy-3-méthyl-2-oxo-2,3-dihydro-1H-indol-3-yl]-4-chloro-benzoïque(ex.48)
4-Chloro-3-[1-(4-tert-butylcarbamoyl-benzènesulfonyl)-5-éthoxy-3-méthyl-2-oxo-2,3-dihydro-1H-indol-3-yl]-N-(1-méthyl-pipéridin-4-yl)-benzamide(ex.51)
4-Chloro-3-[1-(4-tert-butylcarbamoyl-benzènesulfonyl)-5-éthoxy-3-méthyl-2-oxo-2,3-dihydro-1H-indol-3-yl]-N-(2-diéthylamino-éthyl)-benzamide(ex.53)
N-tert-Butyl-4-{3-[2-chloro-5-(3-dimethylamino-propionylamino)-phényl]-5-éthoxy-3-méthyl-2-oxo-2,3-dihydro-indole-1-sulfonyl}-3-méthoxy-benzamide(ex.58)
1-Méthyl-pipéridine-4-carboxylic acid {3-[1-(4-tert-butylcarbamoyi-2-méthoxy-benzènesulfonyl)-5-éthoxy-3-méthyl-2-oxo-2,3-dihydro-1H-indol-3-yl]-4-chloro-phényl}-amide(ex.62)
N-(2-fluoro-1,1-diméthyl-éthyl)-4-[3-(2-chloro-phényl)-5-éthoxy-3-méthyl-2-oxo-2,3-dihydro-indole-1-sulfonyl]- benzamide(ex.66)
4-[3-(2-Chloro-phényl)-5-éthoxy-3-méthyl-2-oxo-2,3-dihydro-indole-1-sulfonyl]-N-(2-hydroxy-1,1-diméthyl-éthyl)-benzamide(ex.72)
4-Chloro-3-[1-(4-tert-butylcarbamoyl-benzènesulfonyl)-5-éthoxy-3-méthyl-2-oxo-2,3-dihydro-1H-indol-3-yl]-N-[2-(4-hydroxy-pipéridin-1-yl)éthyl]-benzamide(ex.79)
4-Chloro-3-[1-(4-tert-butylcarbamoyl-benzènesulfonyl)-5-éthoxy-3-méthyl-2-oxo-2,3-dihydro-1H-indol-3-yl]-N-2,2,2-trifluoroéthyl-benzamide(ex.80)
4-Chloro-3-[1-(4-tert-butylcarbamoyl-benzènesulfonyl)-5-éthoxy-3-méthyl-2-oxo-2,3-dihydro-1H-indol-3-yl]-N-2-hydroxy-éthyl-benzamide(ex.83)
4-Chloro-3-[1-(4-tert-butylcarbamoyl-benzènesulfonyl)-5-éthoxy-3-méthyl-2-oxo-2,3-dihydro-1H-indol-3-yl]-N-cyclopentyl-benzamide(ex.87)
4-Chloro-3-[1-(4-tert-butylcarbamoyl-benzènesulfonyl)-5-éthoxy-3-méthyl-2-oxo-2,3-dihydro-1H-indol-3-yl]-N-[trans-(4-hydroxy-cyclohexyl)]-benzamide(ex.88)
4-Chloro-3-[1-(4-tert-butylcarbamoyl-benzènesulfonyl)-5-éthoxy-3-méthyl-2-oxo-2,3-dihydro-1H-indol-3-yl]-N-[trans-(4-diméthylamino-cyclohexyl)]-benzamide(ex.90)
N-tert-Butyl-4-[3-mèthyl-3-phényl-5-éthoxy-2-oxo-2,3-dihydro-indole-1-sulfonyl]-benzamide (ex.98)
N-tert-Butyl-3-méthoxy-4-[3-méthyl-3-phényl-5-éthoxy-2-oxo-2,3-dihydro-indole-1-sulfonyl]-benzamide (ex.99)
N-tert-Butyl-4-[3-méthyl-3-(2-fluoro-phényl)-5-éthoxy-2-oxo-2,3-dihydro-indole-1-sulfonyl]-benzamide (ex.101)
N-tert-Butyl-4-[3-méthyl-3-(2-chloro-6-fluoro-phényl)-5-éthoxy-2-oxo-2,3-dihydro-indole-1-sulfonyl]-benzamide (ex.125) ;
à l'état de base, d'hydrate ou de solvat, sous forme d'isomères cis/trans ou de leurs mélanges.

13. Procédé de préparation d'un composé selon l'une quelconque des revendications 1 à 12, **caractérisé en ce que** :
A) on fait réagir un composé de formule (II) : 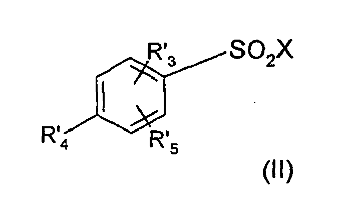 dans laquelle R'₃, R'₄ et R'₅ représentent respectivement et indépendemment les uns des autres des groupes précurseurs des groupes R₃, R₄ et R₅ ou bien représentent les groupes R₃, R₄ et R₅ tels que définis pour les composés de formule (I), et X représente un atome d'halogène,
avec un composé de formule (III) : 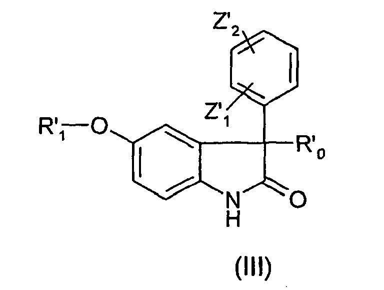 dans laquelle R'₀, R'₁, Z'₁ et Z'₂ représentent respectivement et indépendemment les uns des autres des groupes précurseurs des groupes R₀, R_{1'} Z₁ et Z₂, ou bien les groupes R₀, R₁, Z₁ et Z₂ tels que définis pour les composés de formule (I),
en présence d'un hydrure métallique, à des températures comprises entre -40° et 25°C, dans un solvant anhydre.
B) ou bien, on obtient les composés de formule (I) indirectement, via les composés de formule (I') : 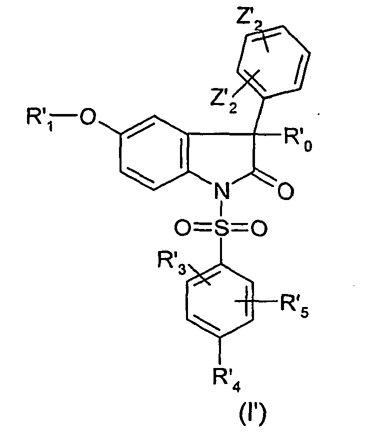
dans laquelle R'₀, R'₁, Z'₁, Z'₂, R'₃, R'₄ et R'₅ représentent respectivement et indépendemment les uns des autres des groupes précurseurs des groupes R₀, R_{1'} Z₁, Z₂, R₃, R₄ et R₅, ou bien les groupes R₀, R₁, Z₁, Z₂, R₃, R₄ et R₅ tels que définis pour les composés de formule (1), eux-mêmes obtenus par réaction d'un composé de formule formule (II) avec un composé de formule (III) tels que définis précédemment.

14. Médicament, **caractérisé en ce qu'**il comprend au moins un composé de formule (I) selon l'une quelconque des revendications 1 à 12.

15. Composition pharmaceutique, **caractérisée en ce qu'**elle comprend au moins un composé de formule (I) selon l'une quelconque des revendications 1 à 12, ainsi qu'au moins un excipient pharmaceutiquement acceptable.

16. Utilisation d'un composé de formule (I) selon l'une quelconque des revendications 1 à 12 pour la préparation d'un médicament destiné au traitement et/ou à la prévention des affections des systèmes nerveux central et périphérique, du système cardiovasculaire, du système endocrinien et hépatique, de la sphère rénale, de la sphère gastrique, intestinale et pulmonaire, en ophtalmologie et dans les troubles du comportement sexuel.

17. Utilisation d'un composé de formule (I) selon la revendication 16, pour la préparation d'un médicament destiné au traitement et/ou à la prévention de différentes affections vasopressine-dépendantes ainsi qu'aux dysfonctionnements de la sécrétion de la vasopressine comme le syndrome inapproprié de sécrétion de vasopressine, les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et l'angioplastie coronarienne transluminale percutanée, l'ischémie cardiaque, les dérèglements de l'hémostase notamment l'hémophilie, le syndrome de Von Willebrand ; les affections du système nerveux central, la douleur, la migraine, le vasospasme cérébral, l'hémorragie cérébrale, les oedèmes cérébraux, la dépression, l'anxiété, la boulimie, les états psychotiques, les troubles de la mémoire par exemple ; les rinopathies et les dysfonctionnement rénaux comme les oedèmes, le vasospasme rénal, la nécrose du cortex rénal, le syndrome néphrotique, les polykystoses rénales dans leurs différentes formes chez l'enfant et chez l'adulte, les hyponatriémies, l'hypokaliémie, le diabète, les néphropathies diabétiques, le diabète insipide néphrogénique, le « NSIADH », le syndrome de Schwartz-Bartter ou la lithiase rénale, les infections urinaires ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, le diabète insipide et l'énurésie ; les affections du système hépatique tel que les cirrhoses du foie ; les ascites abdominales et tous les désordres provoquant une rétention d'eau anormale; les désordres surrénaliens, maladie de Cushing, l'hypercorticisme et l'hyperaldosternonémie, des troubles du comportement sexuel, dans la surcharge pondérale ou l'excès de poids et l'obésité , dans la dysménorrhée ou le travail prématuré, des cancers pulmonaires à petites cellules, des encéphalopathies hyponatriémiques, de la maladie de Raynaud, du syndrome pulmonaire, du glaucome et de la prévention de la cataracte, dans les traitements post-opératoires, notamment après une chirurgie abdominale, cardiaque ou hémorragique et dans les traitements de troubles ou de maladies de l'oreille interne tels que la maladie de Ménière, accouphènes, les vertiges, les difficultés d'audition, notamment dans les graves, ou bourdonnements, les hydrops et notamment les hydrops endolymphatiques, l'ostéoporose.

## Claims

1. Compound corresponding to general formula (I): 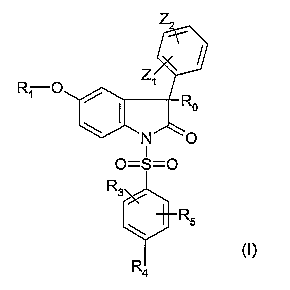 In which
• R₀ represents a (C₁-C₄)alkyl group, a mono or polyfluoro-(C₁-C₄)alkyl group, a -(CH₂)ₙ-cyclopropyl group,
. or alternatively R₀ represents a (C₂-C₄)alkenyl or (C₂-C₄)alkynyl group;
• R₁ represents a hydrogen atom, a (C₁-C₅)alkyl group, a mono or polyfluoro-(C₁-C₅)alkyl group, a hydroxy-(C₁-C₅)alkyl group, a -(CH₂)ₘ-(C₃-C₅)cycloalkyl group;
• Z₁ represents a hydrogen atom or halogen atom, a (C₁-C₄)alkyl group, a mono or polyfluoro-(C₁-C₄)alkyl group, a (C₁-C₄)alkoxy group, a mono or polyfluoro-(C₁-C₄)alkoxy group, a -(CH₂)ₙ-cyclopropyl group, said cyclopropyl group being unsubstituted or substituted with one or more fluorine atoms;
• Z₂ represents a halogen atom or a group T₁W, in which T₁ represents a group -(CH₂)ₙ- and W represents a hydrogen atom, a (C₁-C₄)alkyl group, mono or polyfluoro-(C₁-C₄)alkyl group or a cyclopropyl group unsubstituted or substituted with one or more fluorine atoms,
■ or alternatively W represents a group -C(O)NR₆R₇ in which R₆ and R₇ represent, independently of one another, a hydrogen atom, a (C₁-C₆) alkyl group, a mono or polyfluoro-(C₁-C₆)alkyl group, a -(CH₂)ₘ-(C₃-C₆)cycxoalkyl group, said cycloalkyl group being unsubstituted or substituted with one or more fluorine atoms, a hydroxyl or a group NRR',
. or alternatively R₆ and R₇ represent, independently of one another, a -(CH₂)ₚ-pyrrolidinyl, -(CH₂)ₚ-piperidyl, - (CH₂)ₚ-pyridyl group, said pyrrolidinyl, piperidyl and pyridyl groups being unsubstituted or substituted with one or more halogen atoms, a (C₁-C₄)alkyl group, a mono or polyfluoro(C₁-C₄)alkyl group, a benzyl or by a group -OR,
or alternatively R₆ and R₇ represent, independently of one another, a group
- (CH₂)_{q}-NRₐR_{b},
or alternatively R₆ and R₇ represent, independently of one another, a group
- (CH₂)ₛ-C(O)NRₐR_{b},
. or alternatively R₆ and R₇ represent, independently of one another, a group
- (CH₂)_{q}-OR,
. or alternatively R₆ and R₇ form, together with the nitrogen atom to which they are attached, a monocyclic heterocycle unsubstituted or substituted with one or more fluorine atoms, one or more (C₁-C₄)alkyl, mono or polyfluoro-(C₁-C₄)alkyl, -NR'R, or -OR groups,
. or alternatively R₆ and R₇ form, together with the nitrogen atom to which they are attached, a bicyclic heterocycle unsubstituted or substituted with one or more fluorine atoms, (C₁-C₄) alkyl, mono or polyfluoro-(C₁-C₄)alkyl, -OR or -NR'R groups;
■ or alternatively W represents a group -NR₈C(O)R₉ in which:
. R₈ represents a hydrogen atom, a (C₁-C₄)alkyl group, a mono or polyfluoro-(C₁-C₄)alkyl group,
. R₉ represents a hydrogen atom, a (C₁-C₄)alkyl group, a mono or polyfluoro(C₁-C₄)alkyl group, (CH₂)ᵣ-NR_{d}Rₑ, - (CH₂)ₘ-pyrrolidinyl, - (CH₂)ₘ-piperidyl, - (CH₂)ₘ-pyridyl, said pyrrolidinyl, piperidyl, or pyridyl groups being unsubstituted or substituted with one or more (C₁-C₄) alkyl, halogen, mono or polyfluoro-(C₁-C₄)alkyl, benzyl groups;
■ or alternatively W represents a group - NR₁₀R₁₁ in which R₁₀ and R₁₁ represent, independently of one another, a hydrogen atom, a hydroxyl group, a (C₁-C₆)alkyl or a mono or polyfluoro-(C₁-C₆)alkyl group,
. or alternatively R₁₀ and R₁₁ form, together with the nitrogen atom to which they are attached, a monocyclic heterocycle unsubstituted or substituted with one or more (C₁-C₄)alkyl groups, a mono or polyfluoro-(C₁-C₄)alkyl group or oxo;
■ or alternatively W represents a group -OR₁₂ in which R₁₂ represents a hydrogen atom, a (C₁-C₄) alkyl group, a mono or polyfluoro-(C₁-C₄)alkyl group, a benzyl or a group (CH₂)_{q}-NR'R;
■ or alternatively W represents a group - C(O)OR₁₉
• R₄ represents a (C₁-C₄)alkyl group, a mono or polyfluoro-(C₁-C₄)alkyl group, a group -OR, (C₂-C₄)alkenyl, nitro, COOR_{c}, a benzyloxy group,
. or alternatively R₄ represents a group -C(O)NR₁₃R₁₄ in which R₁₃ and R₁₄ represent, independently of one another, a hydrogen atom, a (C₁-C₆)alkyl group, said alkyl group being unsubstituted or substituted with one or more fluorine atoms, a hydroxyl, an NRR' group, a (C₁-C₆)alkyloxycarbonylamino group, a (C₁-C₆)alkyloxycarbonyl group, or in which R₁₃ and R₁₄ represent, independently of one another, a -(CH₂)ₙ-(C₃-C₆)cycloalkyl group, said cycloalkyl group being unsubstituted or substituted with one or more fluorine atoms,
. or alternatively R₄ represents a group -NR₁₅R₁₆ in which R₁₅ and R₁₆ represent, independently of one another, a hydrogen atom, a hydroxyl group, a (C₁-C₄)alkyl group, a mono or polyfluoro-(C₁-C₄)alkyl group, a -(CH₂)ₙ-(C₃-C₅) cycloalkyl group unsubstituted or substituted with one or more fluorine atoms,
■ or alternatively R₁₅ and R₁₆ form, together with the nitrogen atom to which they are attached, a monocyclic heterocycle,
. or alternatively R₄ represents a group -NR₁₇C(O)R₁₈ in which:
. R₁₇ represents a hydrogen atom, a (C₁-C₄)alkyl group, a mono or polyfluoro-(C₁-C₄)alkyl group,
. R₁₈ represents a (C₁-C₆) alkyl group, a mono or polyfluoro-(C₁-C₆)alkyl group, -(CH₂)ₙ-(C₃-C₆) cycloalkyl, a group -NR_{d}Rₑ, a phenyl group, said phenyl group itself being unsubstituted or substituted with one or more (C₁-C₄)alkyl groups, a mono or polyfluoro-(C₁-C₄)alkyl group;
R₁₉ represents a hydrogen atom, a (C₁-C₆)alkyl group, a mono or polyfluoro- (C₁-C₆) alkyl group, a group - (CH₂)_{q}-NRₐR_{b}, a group -(CH₂)_{q}-OR, a -(CH₂)ₚ-pyrrolidinyl group or a -(CH₂)ₚ-piperidyl group, said pyrrolidinyl and piperidyl groups being unsubstituted or substituted with one or more fluorine atoms, a (C₁-C₄)alkyl group, a mono or polyfluoro-(C₁-C₄)alkyl group.
• R₃ and R₅ represent, independently of one another, a hydrogen atom, a halogen atom, a (C₁-C₄)alkyl group, a mono or polyfluoro-(C₁-C₄)alkyl group, a (C₁-C₄)alkoxy or mono or polyfluoxo-(C₁-C₄)alkoxy group;
• Rₐ and R_{b} represent, independently of one another:
. a hydrogen atom, a (C₁-C₄)alkyl group, a mono or polyfluoro-(C₁-C₄) alkyl group, a -(CH₂)ₙ-cyclopropyl group, said cyclopropyl being unsubstituted or substituted with one or more fluorine atoms,
. or alternatively Rₐ and R_{b} form, together with the nitrogen atom to which they are attached, a monocyclic heterocyclic group, said monocyclic heterocyclic group being unsubstituted or substituted with one or more hydroxyl, (C₁-C₄)alkyl, mono or polyfluoro-(C₁-C₄)alkyl, (C₁-C₄)alkoxy, mono or polyfluoro (C₁-C₄) alkoxy groups, or by a group -NRR';
• R' and R represent, independently of one another, a hydrogen atom, a (C₁-C₄)alkyl group or mono or polyfluoro-(C₁-C₄)alkyl group;
• R_{c} represents a hydrogen atom, a (C₁-C₄)alkyl group, mono or polyfluoro-(C₁-C₄)alkyl group or a benzyl group;
• R_{d} and Rₑ represent, independently of one another, a hydrogen atom, a halogen atom, a (C₁-C₆)alkyl group or mono or polyhalogeno-(C₁-C₆)alkyl group,
. or alternatively R_{d} and Rₑ form, together with the nitrogen atom to which they are attached, a monocyclic heterocyclic group unsubstituted or substituted with one or more fluorine atoms, one or more (C₁-C₄)alkyl or mono or polyfluoro-(C₁-C₄)alkyl groups or a group -OR;
• m can represent the value 0, 1 or 2;
• n can represent the value 0 or 1;
• p can represent the value 0, 1, 2 or 3;
• q can represent the value 2, 3, 4 or 5;
• r can represent the value 0, 1, 2, 3 or 4;
• s can represent the value 1, 2 or 3;
as a base, hydrate or solvate, in the form of cis/trans isomers or mixtures thereof.

2. Compound of general formula (I) according to Claim 1, **characterized in that** R₀ represents a (C₁-C₃)alkyl group, the other groups being as defined for the compound of general formula (I); as a base, hydrate or solvate, in the form of cis/trans isomers or mixtures thereof.

3. Compound of general formula (I) according to either of Claims 1 or 2, **characterized in that** R₀ represents a (C₁-C₃)alkyl group and R₁ represents a (C₁-C₅)alkyl group, the other groups being as defined for the compound of general formula (I); as a base, hydrate or solvate, in the form of cis/trans isomers or mixtures thereof.

4. Compound of general formula (I) according to any one of Claims 1, 2 or 3, **characterized in that** R₀ represents a methyl group and R₁ represents a (C₁-C₂)alkyl group, the other groups being as defined for the compound of general formula (I); as a base, hydrate or solvate, in the form of cis/trans isomers or mixtures thereof.

5. Compound of general formula (I) according to any one of Claims 1 to 4, **characterized in that** Z₁ represents a halogen atom, the other groups being as defined for the compound of general formula (I); as a base, hydrate or solvate, in the form of cis/trans isomers or mixtures thereof.

6. Compound of general formula (I) according to any one of Claims 1 to 5, **characterized in that** Z₂ represents a group T₁W, in which T₁ represents a group -(CH₂)ₙ- with n equal to 0 and W represents:
■ a group -C(O)OR₁₉,
■ a group -C(O)NR₆R₇,
in which R₁₉, R₆ and R₇ and the other groups are as defined for the compound of general formula (I) ; as a base, hydrate or solvate, in the form of cis/trans isomers or mixtures thereof.

7. Compound of general formula (I) according to any one of Claims 1 to 6, **characterized in that** Z₁ is in position -2; as a base, hydrate or solvate, in the form of cis/trans isomers or mixtures thereof.

8. Compound of general formula (I) according to any one of Claims 1 to 7, **characterized in that** Z₁ is in position -2 and Z₂ is in position -5; as a base, hydrate or solvate, in the form of cis/trans isomers or mixtures thereof.

9. Compound of general formula (I) according to any one of Claims 1 to 8, **characterized in that** R₄ represents a group -C(O)NR₁₃R₁₄, in which R₁₃, R₁₄ and the other groups are as defined for the compound of general formula (I); as a base, hydrate or solvate, in the form of cis/trans isomers or mixtures thereof.

10. Compound of general formula (I) according to any one of Claims 1 to 9, **characterized in that** Z₂ represents a group T₁W, in which T₁ represents a group - (CH₂)ₙ- with n equal to 0 and W represents a group -OR₁₂ in which R₁₂ represents a hydrogen atom; as a base, hydrate or solvate, in the form of cis/trans isomers or mixtures thereof.

11. Compound of general formula (I) according to any one of Claims 1 to 10, **characterized in that** Z₁ is in position -2 and Z₂ represents a halogen; as a base, hydrate or solvate, in the form of cis/trans isomers or mixtures thereof.

12. Compound of general formula (I) according to Claim 1, chosen from:
N-tert-butyl-4-[3-(2-chlorophenyl)-5-ethoxy-3-methyl-2-oxo-2,3-dihydroindole-1-sulphonyl]benzamide (Example 2)
N-tert-butyl-4-[3-(2-fluoraphenyl)-5-ethoxy-3-methyl-2-oxo-2,3-dihydroindole-1-sulphonyl]benzamide (Example 6)
methyl 3-[1-(4-tert-butylcarbamoylbenzenesulphonyl)-5-ethoxy-3-methyl-2-axo-2,3-dihydro-1H-indol-3-y1]-4-chlorobenzoate (Example 9)
N-tert-butyl-4-[3-(2-chlorophenyl)-5-ethoxy-3-methyl-2-oxo-2,3-dihydroindole-1-sulphonyl]benzamide (Example 12)
N-cyclopentyl-4-[3-(2-chlorophenyl)-5-ethoxy-3-methyl-2-oxo-2,3-dihydroindole-1-sulphonyl]benzamide (Example 13)
N-tert-butyl-3-methoxy-4-[3-(2-chlorophenyl)-5-ethoxy-3-methyl-2-oxo-2,3-dihydroindole-1-sulphonyl]benzamide (Example 15)
N-tert-butyl-3-methoxy-4-[3-(2-fluorophenyl)-5-ethoxy-3-methyl-2-oxo-2,3-dihydroindole-1-sulphonyl]benzamide (Example 16)
3-[1-(4-tert-butylcarbamoyl-2-methoxybenzanesulphonyl)-5-ethoxy-3-methyl-2-oxo-2,3-dihydro-1H-indol-3-yl]-4-chlorobenzoic acid (Example 19)
4-chloro-3-[1-(4-tert-butylcarbamoyl-2-methoxybenzenesulphonyl)-5-ethoxy-3-methyl-2-oxo-2,3-dihydro-1H-indol-3-y1]-N-ethylbenzamide (Example 21)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(2-pyridyl)benzamide (Example 22)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(3-dimethylaminopropyl)benzamide (Example 25)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(1-methylpiperidin-4-yl)benzamide (Example 28)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(2-pyrrolidin-1-ylethyl) benzamide (Example 29)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(2-diethylaminoethyl)benzamide (Example 33)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(2-morpholin-4-ylethyl)benzamide (Example 36)
4-chloro-3-[1-(4-tert-butylcarbamoyl-2-methoxybenzenesulphonyl)-5-ethoxy-3-methyl-2-oxo-2,3-dihydro-1H-indol-3-yl]-N-[2-(4-methylpiperazin-1-yl)ethyl]benzamide (Example 37)
4-chloro-3-[1-(4-tert-butylcarbamoyl-2-methoxybenzenesulphonyl)-5-ethoxy-3-methyl-2-oxo-2,3-dihydro-1H-indol-3-yl]-N-[3-(4-methylpiperazin-1-yl)propyl]benzamide (Example 40)
4-chloro-3-[1-(4-tert-butylcarbamoyl-2-methoxybenzenesulphonyl)-5-ethoxy-3-methyl-2-oxo-2,3-dihydro-1H-indol-3-yl]-N-(R)-pyrrolidin-3-ylbenzamide (Example 43)
4-chloro-3-[1-(4-tert-butylcarbamoyl-2-methoxybenzenesulphonyl)-5-ethoxy-3-methyl-2-oxo-2,3-dihydro-1H-indol-3-yl]-N-piperidin-4-ylbenzamide (Example 44)
4-chloro-3-[1-(4-tert-butylcarbamoyl-2-methoxybenzenesulphonyl)-5-ethoxy-3-methyl-2-oxo-2,3-dihydro-1H-indol-3-yl]-N-2-aminoethylbenzamide (Example 47)
3-[1-(4-tert-butylcarbamoylbenzenesulphonyl)-5-ethoxy-3-methyl-2-oxo-2,3-dihydro-1H-indol-3-yl]-4-chlorobenzoic acid (Example 48)
4-chloro-3-[1-(4-tert-butylcarbamoylbenzenesulphonyl)-5-ethoxy-3-methyl-2-oxo-2,3-dihydro-1H-indol-3-yl]-N-(1-methylpiperidin-4-yl)benzamide (Example 51)
4-chloro-3-[1-(4-tert-butylcarbamoylbenzenesulphonyl)-5-ethoxy-3-methyl-2-oxo-2,3-dihydro-1H-indol-3-yl]-N-(2-diethylaminoethyl)benzamide (Example 53)
N-tert-butyl-4-{3-[2-chloro-5-(3-dimethylaminopropionylamino)phenyl]-5-ethoxy-3-methyl-2-oxo-2,3-dihydroindole-1-sulphonyl}-3-methoxybenzamide (Example 58)
1-methylpiperidine-4-carboxylic acid {3-[1-(4-tert-butylcarbamoyl-2-methoxybenzenesulphonyl)-5-ethoxy-3-methyl-2-oxo-2,3-dihydro-1H-indol-3-yl]-4-chlorophenyl}amide (Example 62)
N-(2-fluoro-1,1-dimethylethyl)-4-[3-(2-chlorophenyl)-5-ethoxy-3-methyl-2-oxo-2,3-dihydroindole-1-sulphonyl]benzamide (Example 66)
4-[3-(2-chlorophenyl)-5-ethoxy-3-methyl-2-oxo-2,3-dihydroindole-1-sulphonyl]-N-(2-hydroxy-1,1-dimethylethyl)benzamide (Example 72)
4-chloro-3-[1-(4-tert-butylcarbamoylbenzenesulphonyl)-5-ethoxy-3-methyl-2-oxo-2,3-dihydro-1H-indol-3-yl]-N-[2-(4-hydroxypiperidin-1-yl)ethyl]benzamide (Example 79)
4-chloro-3-[1-(4-tert-butylcarbamoylbenzenesulphonyl)-5-ethoxy-3-methyl-2-oxo-2,3-dihydro-1H-indol-3-yl]-N-2,2,2-trifluoroethylbenzamide (Example 80)
4-chloro-3-[1-(4-tert-butylcarbamoylbenzenesulphonyl)-5-ethoxy-3-methyl-2-oxo-2,3-dihydro-1H-indol-3-yl]-N-2-hydroxyethylbenzamide (Example 83)
4-chloro-3-[1-(4-tert-butylcarbamoylbenzenesulphonyl)-5-ethoxy-3-methyl-2-oxo-2,3-dihydro-1H-indol-3-yl]-N-cyclopentylbenzamide (Example 87)
4-chloro-3-[1-(4-tert-butylcarbamoylbenzenesulphonyl)-5-ethoxy-3-methyl-2-oxo-2,3-dihydro-1H-indol-3-yl]-N-[trans-(4-hydroxycyclohexyl)]benzamide (Example 88)
4-chloro-3-[1-(4-tert-butylcarbamoylbenzenesulphonyl)-5-ethoxy-3-methyl-2-oxo-2,3-dihydro-1H-indol-3-yl]-N-[trans-(4-dimethylaminocyclohexyl)]benzamide (Example 90)
N-tert-butyl-4-[3-methyl-3-phenyl-5-ethoxy-2-oxo-2,3-dihydroindole-1-sulphonyl]benzamide (Example 98)
N-tert-butyl-3-methoxy-4-[3-methyl-3-phenyl-5-ethoxy-2-oxo-2,3-dihydroindole-1-sulphonyl]benzamide (Example 99)
N-tert-butyl-4-[3-methyl-3-(2-fluorophenyl)-5-ethoxy-2-oxo-2,3-dihydroindole-1-sulphonyl]benzamide (Example 101)
N-tert-butyl-4-[3-methyl-3-(2-chloro-6-fluorophenyl)-5-ethoxy-2-oxo-2,3-dihydroindole-1-sulphonyl]benzamide (Example 125);
as a base, hydrate or solvate, in the form of cis/trans isomers or mixtures thereof.

13. Method of preparation of a compound according to any one of Claims 1 to 12, **characterized in that**:
A) a compound of formula (II): 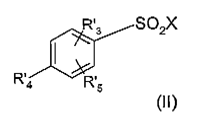 in which R'₃, R'₄ and R'₅ represent respectively, and independently of one another, precursor groups of the groups R₃, R₄ and R₅ or alternatively represent the groups R₃, R₄ and R₅ as defined for the compounds of formula (I), and X represents a halogen atom,
is reacted with a compound of formula (III): 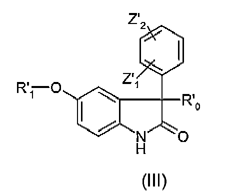 in which R'₀, R'₁, Z'₁ and Z'₂ represent respectively, and independently of one another, precursor groups of the groups R₀, R₁, Z₁ and Z₂, or alternatively the groups R₀, R₁, Z₁ and Z₂ as defined for the compounds of formula (I) ,
in the presence of a metal hydride, at temperatures between -40° and 25°C, in an anhydrous solvent.
B) or alternatively, the compounds of formula (I) are obtained indirectly, via the compounds of formula (I'): 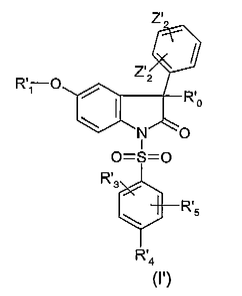
in which R'₀, R'₁, Z'₁, Z'₂, R'₃, R'₄ and R'₅ represent respectively, and independently of one another, precursor groups of the groups R₀, R₁, Z₁, Z₂, R₃, R₄ and R₅, or alternatively the groups R₀, R₁, Z₁, Z₂, R₃, R₄ and R₅ as defined for the compounds of formula (I), themselves obtained by reaction of a compound of formula (II) with a compound of formula (III) as defined previously.

14. Medicinal product, **characterized in that** it comprises at least one compound of formula (I) according to any one of Claims 1 to 12.

15. Pharmaceutical composition, **characterized in that** it comprises at least one compound of formula (I) according to any one of Claims 1 to 12, as well as at least one pharmaceutically acceptable excipient.

16. Use of a compound of formula (I) according to any one of Claims 1 to 12 for the preparation of a medicinal product intended for the treatment and/or prevention of disorders of the central and peripheral nervous systems, of the cardiovascular system, of the endocrine and hepatic system, of the renal system, of the gastric, intestinal and pulmonary system, in ophthalmology and in problems of sexual behaviour.

17. Use of a compound of formula (I) according to Claim 16, for the preparation of a medicinal product intended for the treatment and/or prevention of various vasopressin-dependent disorders as well as dysfunctions of vasopressin secretion such as the inappropriate syndrome of vasopressin secretion, cardiovascular disorders, such as hypertension, pulmonary hypertension, heart failure, circulatory failure, myocardial infarction, atherosclerosis or coronary vasospasm, in particular in smokers, unstable angina and percutaneous transluminal coronary angioplasty, ischaemic heart disease, disturbances of haemostasis notably haemophilia, Von Willebrand syndrome; disorders of the central nervous system, pain, migraine, cerebral vasospasm, cerebral haemorrhage, cerebral oedema, depression, anxiety, bulimia, psychotic states, for example memory disorders; renopathies and renal dysfunction such as oedema, renal vasospasm, necrosis of the renal cortex, nephrotic syndrome, renal polycystic diseases in their various forms in children and in adults, hyponatraemia, hypokalaemia, diabetes, diabetic nephropathies, nephrogenic diabetes insipidus, "NSIADH", Schwartz-Bartter syndrome or renal lithiasis, urinary tract infections; disorders of the gastric system, such as gastric vasospasm, portal hypertension, hepatocirrhosis, ulcers, vomiting pathology, for example nausea including nausea due to chemotherapy, motion sickness, diabetes insipidus and enuresis; disorders of the hepatic system such as hepatic cirrhosis; abdominal ascites and all disorders causing abnormal water retention; adrenal disorders, Cushing disease, hypercorticism and hyperaldosteronaemia, problems of sexual behaviour, overweight conditions or excessive weight and obesity, in dysmenorrhoea or premature labour, small cell lung cancers, hyponatraemic encephalopathies, Raynaud disease, pulmonary syndrome, glaucoma and prevention of cataract, in postoperative treatments, notably after abdominal, cardiac or haemorrhagic surgery and in treatments of disorders or diseases of the inner ear such as Ménière disease, tinnitus, vertigo, hearing difficulties, notably at low tones, or buzzing, hydrops and notably endolymphatic hydrops, osteoporosis.

## Patentansprüche

1. Verbindung der allgemeinen Formel (I): 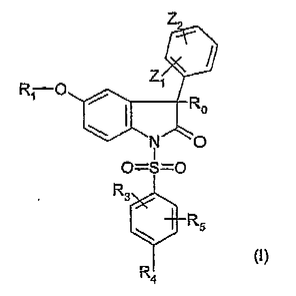 worin
• R₀ für eine (C₁-C₄)-Alkylgruppe, eine Mono- oder Polyfluor-(C₁-C₄)-alkylgruppe oder eine -(CH₂)ₙ-Cyclopropylgruppe steht,
• oder R₀ für eine (C₂-C₄)-Alkenyl- oder (C₂-C₄)-Alkinylgruppe steht;
• R₁ für ein Wasserstoffatom, eine (C1-C₅) - Alkylgruppe, eine Mono- oder Polyfluor-(C₁-C₅)-alkylgruppe, eine Hydroxy-(C₁-C₅)-alkylgruppe oder eine -(CH₂)ₘ-(C₃-C₅)-Cycloalkylgruppe steht;
• Z₁ für ein Wasserstoff- oder Halogenatom oder eine (C₁-C₄)-Alkylgruppe, eine Mono- oder Polyfluor-(C₁-C₄)-alkylgruppe, eine (C₁-C₄)-Alkoxygruppe, eine Mono- oder Polyfluor-(C₁-C₄)-alkoxygruppe oder eine -(CH₂)ₙ-Cyclopropylgruppe steht, wobei die Cyclopropylgruppe gegebenenfalls durch ein oder mehrere Fluoratome substituiert ist;
• Z₂ für ein Halogenatom oder eine Gruppe T₁W steht, worin T₁ für eine -(CH₂)ₙ-Gruppe steht und W für ein Wasserstoffatom, eine (C₁-C₄)-Alkylgruppe, eine Mono- oder Polyfluor-(C₁-C₄)-alkylgruppe oder eine Cyclopropylgruppe, die gegebenenfalls durch ein oder mehrere Fluoratome substituiert ist, steht,
. oder W für eine Gruppe -C(O)NR₆R₇ steht, worin R₆ and R₇ unabhängig voneinander für ein Wasserstoffatom, eine (C₁-C₆)-Alkylgruppe, eine Mono- oder Polyfluor-(C₁-C₆)-alkylgruppe oder eine -(CH₂)ₘ-(C₃-C₆)-Cycloalkylgruppe stehen, wobei die Cycloalkylgruppe gegebenenfalls durch ein oder mehrere Fluoratome, eine Hydroxygruppe oder eine Gruppe NRR' substituiert ist,
. oder R₆ und R₇ unabhängig voneinander für eine -(CH₂)ₚ-Pyrrolidinyl-, -(CH₂)ₚ-Piperidyl- oder -(CH₂)ₚ-Pyridylgruppe stehen, wobei die Pyrrolidinyl-, Piperidyl- und Pyridylgruppen gegebenenfalls durch ein oder mehrere Halogenatome, eine (C₂-C₄)-Alkylgruppe, eine Mono- oder Polyfluor-(C₁-C₄)-alkylgruppe, eine Benzylgruppe oder eine -OR-Gruppe substituiert sind,
. oder R₆ und R₇ unabhängig voneinander für eine Gruppe (CH₂)_{q}-NRₐR_{b} stehen,
. oder R₆ und R₇ unabhängig voneinander für eine Gruppe -(CH₂)ₛ-C(O)NRₐR_{b} stehen,
. oder R₆ und R₇ unabhängig voneinander für eine Gruppe -(CH₂)_{q}-OR stehen,
. oder R₆ und R₇ zusammen mit dem Stickstoffatom, an das sie gebunden sind, einen monocyclischen Heterocyclus bilden, der gegebenenfalls durch ein oder mehrere Fluoratome oder eine oder mehrere (C₁-C₄)-Alkyl-, Mono- oder Polyfluor-(C₂-C₄)-alkyl-, -NR'R- oder -OR-Gruppen substituiert ist,
. oder R₆ und R₇ zusammen mit dem Stickstoffatom, an das sie gebunden sind, einen bicyclischen Heterocyclus bilden, der gegebenenfalls durch ein oder mehrere Fluoratome oder eine oder mehrere (C₁-C₄) -Alkyl-, Mono- oder Polyfluor-(C₁-C₄)-alkyl-, -OR- oder -NR'R-Gruppen substituiert ist;
. oder W für eine Gruppe -NR₈C(O)R₉ steht, worin:
. R₈ für ein Wasserstoffatom oder eine (C₁-C₄)-Alkylgruppe oder eine Mono- oder Polyfluor-(C₁-C₄)-alkylgruppe steht,
. R₉ für ein Wasserstoffatom, eine (C₁-C₄)-Alkylgruppe, eine Mono- oder Polyfluor-(C₁-C₄)-alkylgruppe, -(CH₂)ᵣ -NR_{d}Rₑ, -(CH₂)ₘ-Pyrrolidinyl, -(CH₂)ₘ-Piperidyl oder (CH₂)ₘ-Pyridyl steht, wobei die Pyrrolidinyl-, Piperidyl- oder Pyridylgruppen gegebenenfalls durch ein oder mehrere (C₁-C₄)-Alkyl-, Halogen-, Mono- oder Polyfluor-(C₁-C₄)-alkyl- oder Benzylgruppen substituiert sind;
. oder W für eine Gruppe -NR₁₀R₁₁ steht, worin R₁₀ und R₁₁ unabhängig voneinander für ein Wasserstoffatom, eine Hydroxygruppe, eine (C₁-C₆)-Alkylgruppe oder eine Mono- oder Polyfluor-(C₁-C₆)-alkylgruppe stehen,
. oder R₁₀ und R₁₁ zusammen mit dem Stickstoffatom, an das sie gebunden sind, einen monocyclischen Heterocyclus bilden, der gegebenenfalls durch eine oder mehrere (C₁-C₄) -Alkylgruppen, eine Mono- oder Polyfluor-(C₁-C₄)-alkylgruppe oder Oxo substituiert ist;
. oder W für eine Gruppe -OR₁₂ steht, worin R₁₂ für ein Wasserstoffatom, eine (C₁-C₄)-Alkylgruppe, eine Mono- oder Polyfluor- (C₁-C₄) -alkylgruppe, eine Benzylgruppe oder eine Gruppe -(CH₂)_{q}-NR'R steht;
. oder W für eine Gruppe -C(O)OR₁₉ steht;
• R₄ für eine (C₁-C₄)-Alkylgruppe, eine Mono- oder Polyfluor-(C₁-C₄)-alkylgruppe, eine Gruppe -OR, eine (C₂-C₄)-Alkanylgruppe, eine Nitrogruppe, eine Gruppe COOR_{c} oder eine Benzyloxygruppe steht,
. oder R₄ für eine Gruppe -C(O)NR₁₃R₁₄ steht, worin R₁₃ and R₁₄ unabhängig voneinander für ein Wasserstoffatom, eine (C₁-C₆)-Alkylgruppe, wobei die Alkylgruppe gegebenenfalls durch ein oder mehrere Fluoratome substituiert ist, eine Hydroxygruppe, eine Gruppe NRR' eine (C₁-C₆)-Alkoxycarbonylaminogruppe oder eine (C₁-C₆)-Alkyl-oxycarbonylgruppe stehen oder worin R₁₃ and R₁₄ unabhängig voneinander für eine -(CH₂)ₙ-(C₃-C₆)-Cycloalkylgruppe stehen, wobei die Cycloalkylgruppe gegebenenfalls durch ein oder mehrere Fluoratome substituiert ist,
oder R₄ für eine Gruppe -NR₁₅R₁₆ steht, worin R₁₅ und R₁₆ unabhängig voneinander für ein Wasserstoffatom, eine Hydroxylgruppe, eine (C₁-C₄)-Alkylgruppe, eine Mono- oder Polyfluor-(C₁-C₄)-alkylgruppe oder eine -(CH₂)ₙ-(C₃-C₅)-Cycloalkylgruppe, die gegebenenfalls durch ein oder mehrere Fluoratome substituiert ist, stehen,
. oder R₁₅ und R₁₆ zusammen mit dem Stickstoffatom, an das sie gebunden sind, einen monocyclischen Heterocyclus bilden,
. oder R₄ für eine Gruppe -NR₁₇C(O)R₁₈ steht, worin:
. R₁₇ für ein Wasserstoffatom, eine (C₁-C₄)-Alkylgruppe oder eine Mono- oder Polyfluor-(C₁-C₄)-alkylgruppe steht,
. R₁₈ für eine (C₁-C₆) -Alkylgruppe, eine Mono- oder Polyfluor-(C₁-C₆)-alkylgruppe, eine -(CH₂)ₙ-(C₃-C₆)-Cycloalkylgruppe, eine Gruppe -NR_{d}Rₑ oder eine Phenylgruppe steht, wobei die Phenylgruppe selbst gegebenenfalls durch eine oder mehrere (C₁-C₄)-Alkylgruppen oder eine Mono- oder Polyfluor-(C₁-C₄)-alkylgruppe substituiert ist;
. R₁₉ für ein Wasserstoffatom, eine (C₁-C₆)-Alkylgruppe, eine Mono- oder Polyfluor- (C₃-C₆) - alkylgruppe, eine Gruppe (CH₂)_{q}-NRₐR_{b}, eine Gruppe
- (CH₂)_{q}-OR, eine -(CH₂)ₚ-Pyrrolidinylgruppe oder eine (CH₂)ₚ-Piperidylgruppe steht, wobei die Pyrrolidinyl- und Piperidylgruppen gegebenenfalls durch ein oder mehrere Fluoratome, eine (C₁-C₄)-Alkylgruppe oder eine Mono- oder Polyfluor-(C₁-C₅)-alkylgruppe substituiert sind;
• R₃ und R₅ unabhängig voneinander für ein Wasserstoff- oder Halogenatom, eine (C₁-C₄)-Alkylgruppe, eine Mono- oder Polyfluor-(C₁-C₄)-alkylgruppe, eine (C₁-C₄)-Alkoxygruppe oder eine Mono- oder Palyfluor-(C₁-C₄)-alkoxygruppe stehen;
• Rₐ und R_{b} unabhängig voneinander für:
. ein Wasserstoffatom, eine (C₁-C₄)-Alkylgruppe, eine Mono- oder Polyfluor- (C₁-C₄) -alkylgruppe oder eine -(CH₂)ₙ-Cyclopropylgruppe, wobei das Cyclopropyl gegebenenfalls durch ein oder mehrere Fluoratome substituiert ist, stehen,
. oder Rₐ und R_{b} zusammen mit dem Stickstoffatom, an das sie gebunden sind, eine monocyclische heterocyclische Gruppe bilden, die gebenenenfalls durch eine oder mehrere Hydroxylgruppen, (C₁-C₄)-Alkylgruppen, Mono- oder Polyfluor-(C₁-C₄)-alkylgruppen, (C₁-C₄-Alkoxygruppen, Mono- oder Polyfluor-(C₁-C₄)-alkoxygruppen oder eine Gruppe -NRR' substituiert ist;
• R' und R unabhängig voneinander für ein Wasserstoffatom, eine (C₁-C₄)-Alkylgruppe oder eine Mono- oder Holyfluor-(C₁-C₄)-alkylgruppe stehen;
• R_{c} für ein Wasserstoffatom, eine (C₁-C₄)-Alkylgruppe, eine Mono- oder Polyfluor-(C₁-C₄) - alkylgruppe oder eine Benzylgruppe steht;
• R_{d} und Rₑ unabhängig voneinander für ein Wasserstoffatom, ein Halogenatom, eine (C₁-C₆)-Alkylgruppe oder eine Mono- oder Polyhalogen-(C₁-C₆)-alkylgruppe stehen;
. oder R_{d} und Rₑ zusammen mit dem Stickstoffatom, an das sie gebunden sind, eine monocyclische heterocyclische Gruppe bilden, die gebenenenfalls durch ein oder mehrere Fluoratome, eine oder mehrere (C₁-C₄) -Alkylgruppen oder Mono- oder Polyfluor-(C₁-C₄)-alkylgruppen oder eine Gruppe -OR substituiert ist;
• m für den Wert 0, 1 oder 2 stehen kann;
• n für den Wert 0 oder 1 stehen kann;
• p für den Wert 0, 1, 2 oder 3 stehen kann;
• q für den Wert 2, 3, 4 oder 5 stehen kann;
• r für den Wert 0, 1, 2, 3 oder 4 stehen kann;
• s für den Wert 1, 2 oder 3 stehen kann;
in Basen-, Hydrat- oder Solvatform, in Form von cis/trans-Isomeren oder Gemischen davon.

2. Verbindung der allgemeinen Formel (I) nach Anspruch 1, **dadurch gekennzeichnet, daß** R₀ für eine (C₁-C₃)-Alkylgruppe steht, wobei die anderen Gruppen die für die Verbindung der allgemeinen Formel (I) angegebene Bedeutung besitzen; in Basen-, Hydrat- oder Solvatform, in Form von cis/trans-Isomeren oder Gemischen davon.

3. Verbindung der allgemeinen Formel (I) nach Anspruch 1 oder 2, **dadurch gekennzeichnet, daß** R₀ für eine (C₁-C₃)-Alkylgruppe steht und R₁ für eine (C₁-C₅)-Alkylgruppe steht, wobei die anderen Gruppen die für die Verbindung der allgemeinen Formel (I) angegebene Bedeutung besitzen; in Basen-, Hydrat- oder Solvatform, in Form von cis/trans-Isomeren oder Gemischen davon.

4. Verbindung der allgemeinen Formel (I) nach Anspruch 1, 2 oder 3, **dadurch gekennzeichnet, daß** R₀ für eine Methylgruppe steht und R₁ für eine (C₁-C₂)-Alkylgruppe steht, wobei die anderen Gruppen die für die Verbindung der allgemeinen Formel (I) angegebene Bedeutung besitzen; in Basen-, Hydrat- oder Solvatform, in Form von cis/trans-Isomeren oder Gemischen davon.

5. Verbindung der allgemeinen Formel (I) nach einem der Ansprüche 1 bis 4, **dadurch gekennzeichnet, daß** Z₁ für ein Halogenatom steht, wobei die anderen Gruppen die für die Verbindung der allgemeinen Formel (I) angegebene Bedeutung besitzen; in Basen-, Hydrat- oder Solvatform, in Form von cis/trans-Isomeren oder Gemischen davon.

6. Verbindung der allgemeinen Formel (I) nach einem der Ansprüche 1 bis 5, **dadurch gekennzeichnet, daß** Z₂ für eine Gruppe T₁W steht, worin T₁ für eine -(CH₂)ₙ-Gruppe mit n gleich 0 steht und W für:
• eine Gruppe -C(O)OR₁₉ oder
• eine Gruppe -C(O)NR₆R₇
steht, worin R₁₉, R₆ und R₇ und die anderen Gruppen die für die Verbindung der allgemeinen Formel (I) angegebene Bedeutung besitzen; in Basen-, Hydrat-oder Solvatform, in Form von cis/trans-Isomeren oder Gemischen davon.

7. Verbindung der allgemeinen Formel (I) nach einem der Ansprüche 1 bis 6, **dadurch gekennzeichnet, daß** Z₁ in 2-Position steht; in Basen-, Hydrat- oder Solvatform, in Form von cis/trans-Isomeren oder Gemischen davon.

8. Verbindung der allgemeinen Formel (I) nach einem der Ansprüche 1 bis 7, **dadurch gekennzeichnet, daß** Z₁ in 2-postition steht und Z₂ in 5-Position steht; in Basen-, Hydrat- oder Solvatform, in Form von cis/trans-Isomeren oder Gemischen davon.

9. Verbindung der allgemeinen Formel (I) nach einem der Ansprüche 1 bis 8, **dadurch gekennzeichnet, daß** R₄ für eine Gruppe -C(O)NR₁₃R₁₄ steht, worin R₁₃, R₁₄ und die anderen Gruppen die für die Verbindung der allgemeinen Formel (I) angegebene Bedeutung besitzen; in Basen-, Hydrat- oder Solvatform, in Form von cis/trans-Isomeren oder Gemischen davon.

10. Verbindung der allgemeinen Formel (I) nach einem der Ansprüche 1 bis 9, **dadurch gekennzeichnet, daß** Z₂ für eine Gruppe T₁W steht, worin T₁ für eine -(CH₂)ₙ-Gruppe mit n gleich 0 steht und W für eine Gruppe -OR₁₂ steht, worin R₁₂ für ein Wasserstoffatom steht; in Basen-, Hydrat- oder Solvatform, in Form von cis/trans-Isomeren oder Gemischen davon.

11. Verbindung der allgemeinen Formel (I) nach einem der Ansprüche 1 bis 10, **dadurch gekennzeichnet, daß** Z₁ in 2-Position steht und Z₂ für ein Halogen steht; in Basen-, Hydrat- oder Solvatform, in Form von cis/trans-Isomeren oder Gemischen davon.

12. Verbindung der allgemeinen Formel (I) nach Anspruch 1, ausgewählt unter:
N-tert.-Butyl-4-[3-(2-chlorphenyl)-5-ethoxy-3-methyl-2-oxo-2 ,3-dihydroindol-1-sulfonyl] benzamid (Bsp. 2)
N-tert.-Butyl-4-[3-(2-fluorphenyl)-5-ethoxy-3-methyl-2-oxo-2,3-dihydroindol-1-sulfonyl]benzamid (Bsp. 6)
3-[1-(4-tert.-Butylcarbamoylbenzolsulfonyl)-5-ethoxy-3-methyl-2-oxo-2,3-dihydro-1H-indol-3-yl]-4-chlorbenzoesäuremethylester (Bsp. 9)
N-tert.-Butyl-4-[3-(2-chlorphenyl)-5-ethoxy-3-methyl-2-oxo-2,3-dihydroindol-1-sulfonyl]benzamid (Bsp. 12)
N-Cyclopentyl-4-[3-(2-chlorphenyl)-5-ethoxy-3-methyl-2-oxo-2,3-dihydroindol-1-sulfonyl]benzamid (Bsp. 13)
N-tert.-Butyl-3-methoxy-4-[3-(2-chlvrphenyl)-5-ethoxy-3-methyl-2-oxo-2,3-dihydroindol-1-sulfonyl]benzamid (Bsp. 15)
N-tert.-Butyl-3-methoxy-4-[3-(2-fluorphenyl)-5-ethoxy-3-methyl-2-oxo-2,3-dihydroindol-1-sulfonyl]benzamid (Bsp. 16)
3-[1-(4-tert.-Butylcarbamoyl-2-methoxybenzolsulfonyl)-5-ethoxy-3-methyl-2-oxo-2,3-dihydro-1H-indol-3-yl]-4-chlorbenzoesäure (Bsp. 19)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-ethylbenzamid (Bsp. 21)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(2-pyrldyl)benzamid (Bsp. 22)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(3-dimethylaminopropyl)-benzamid (Bsp. 25)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(1-methylpiperidin-4-yl)-benzamid (Bsp. 28)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(2-pyrrolidin-1-ylethyl)-benzamid (Bsp. 29)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(2-diethylaminoethyl)-benzamid (Bsp. 33)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(2-morpholin-4-ylethyl)-benzamid (Bsp. 36)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-[2-(4-methylpiperazin-1-yl)ethyl]benzamid (Bsp. 37)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-[3-(4-methylpiperazin-1-yl)propyl]benzamid (Bsp. 40)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-(R)-pyrrolidin-3-yl-benzamid (Bsp. 43)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-piperidin-4-ylbenzamid (Bsp. 44)
4-Chlor-3-[1-(4-tert.-butylcarbamoyl-2-methoxybenzolsulfonyl)-5-ethoxy-3-methyl-2-oxo-2,3-dihydro-1H-indol-3-yl]-N-2-aminoethylbenzamid (Bsp. 47)
3-[1-(4-tert.-Butylcarbamoylbenzolsulfonyl)-5-ethoxy-3-methyl-2-oxo-2,3-dihydro-1H-indol-3-yl]-4-chlorbenzoesäure (Bsp. 48)
4-Chlor-3-[1-(4-tert.-butylcarbamoylbenzolsulfonyl)-5-ethoxy-3-methyl-2-oxo-2,3-dihydro-1H-indol-3-yl]-N-(1-methylpiperidin-4-yl)benzamid (Bsp. 51)
4-Chlor-3-[1-(4-tert.-butylcarbamoylbenzolsulfonyl)-5-ethoxy-3-methyl-2-oxo-2,3-dihydro-1H-indol-3-yl]-N-(2-diethylaminoethyl)benzamid (Bsp. 53)
N-tert.-Butyl-4-{3-[2-chlor-5-(3-dimethylaminopropionylamino)phenyl]-5-ethoxy-3-methyl-2-oxo-2,3-dihydroindol-1-sulfonyl}-3-methoxybenzamid (Bsp. 58)
1-Methylpiperidin-4-carbonsäure{3-[1-(4-tert.-butylcarbamoyl-2-methoxybenzolsulfonyl)-5-ethoxy-3-methyl-2-oxo-2,3-dihydro-1H-indol-3-yl]-4-chlorphenyl}amid (Bsp. 62)
N-(2-Fluor-1,1-dimethylethyl)-4-[3-(2-chlorphenyl)-5-ethoxy-3-methyl-2-oxo-2,3-dihydroindol-1-sulfonyl]benzamid (Bsp. 66)
4-[3-(2-Chlorphenyl)-5-ethoxy-3-methyl-2-oxo-2,3-dihydroindol-1-sulfonyl]-N-(2-hydroxy-1,1-dimethylethyl)benzamid (Bsp. 72)
4-Chlor-3-[1-(4-tert.-butylcarbamoylbenzolsulfonyl)-5-ethoxy-3-methyl-2-oxo-2,3-dihydro-1H-indol-3-yl]-N-[2-(4-hydroxypiperidin-1-yl)ethyl]-benzamid (Bsp. 79)
4-Chlor-3-[1-(4-tert.-butylcarbamoylbenzolsulfonyl)-5-ethoxy-3-methyl-2-oxo-2,3-dihydro-1H-indol-3-yl]-N-2,2,2-trifluorethylbenzamid (Bsp. 80)
4-Chlor-3-[1-(4-tert.-butylcarbamoylbenzolsulfonyl)-5-ethoxy-3-methyl-2-oxo-2,3-dihydro-1H-indol-3-yl]-N-2-hydroxyethylbenzamid (Bsp. 83)
4-Chlor-3-[1-(4-tert.-butylcarbamoylbenzolsulfonyl)-5-ethoxy-3-methyl-2-oxo-2,3-dihydro-1H-indol-3-yl]-N-cyclopentylbenzamid (Bsp. 87)
4-Chlor-3-[1-(4-tert.-butylcarbamoylbenzolsulfonyl)-5-ethoxy-3-methyl-2-oxo-2,3-dihydro-1H-indol-3-yl]-N-[trans-(4-hydroxycyclohexyl)]-benzamid (Bsp. 88)
4-Chlor-3-[1-(4-tert.-butylcarbamoylbenzolsulfonyl)-5-ethoxy-3-methyl-2-oxo-2,3-dihydro-1H-indol-3-yl]-N-[trans-(4-dimethylaminocyclohexyl)]-benzamid (Bsp. 90)
N-tert.-Butyl-4-[3-methyl-3-phenyl-5-ethoxy-2-oxo-2,3-dihydroindol-1-sulfonyl]benzamid (Bsp. 98)
N-tert.-Butyl-3-methoxy-4-[3-methyl-3-phenyl-5-ethoxy-2-oxo-2,3-dihydroindol-1-sulfonyl]benzamid (Bsp. 99)
N-tert.-Butyl-4-[3-methyl-3-(2-fluorphenyl)-5-ethoxy-2-oxo-2,3-dihydroindol-1-sulfonyl]benzamid (Bsp. 101)
N-tert.-Butyl-4-[3-methyl-3-(2-chlor-6-fluorphenyl)-5-ethoxy-2-oxo-2,3-dihydroindol-1-sulfonyl]benzamid (Bsp. 125);
in Basen-, Hydrat- oder Solvatform, in Form von cis/trans-Isomeren oder Gemischen davon.

13. Verfahren zur Herstellung einer Verbindung gemäß einem der Ansprüche 1 bis 12, **dadurch gekennzeichnet, daß** man:
A) eine Verbindung der Formel (II): 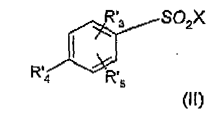 worin R'₃, R'₄ und R'₅ jeweils unabhängig voneinander für Vorläufergruppen der Gruppen R₃, R₄ und R₅ stehen oder für die Gruppen R₃, R₄ und R₅ gemäß der für die Verbindungen der Formel (I) angegebenen Definition stehen und X für ein Halogenatom steht,
in Gegenwart eines Metallhydrids bei Temperaturen zwischen -40 und 25°C in einem wasserfreien Lösungsmittel mit einer Verbindung der Formel (III) : 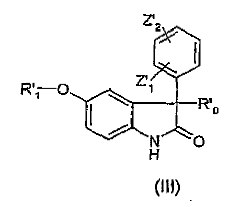 worin R'₀, R'₁₁, Z'₁ und Z'₂ jeweils unabhängig voneinander für Vorläufergruppen der Gruppen R₀, R₁, Z₁ und Z₂ stehen oder für die Gruppen R₀, R₁, Z₁ und Z₂ gemäß der für die Verbindungen der Formel (I) angegebenen Definition stehen,
umsetzt
B) oder die Verbindungen der Formel (I) indirekt über die Verbindungen der Formel (I') : 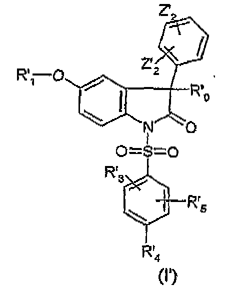
worin R'₀, R'₁₁, Z'₁, Z'₂, R'₃, R'₄ und R'₅ jeweils unabhängig voneinander für Vorläufergruppen der Gruppen R₀, R₁, Z₂, Z₂, R₃, R₄ und R₅ stehen oder für die Gruppen R₀, R₁, Z₁, Z₂, R₃, R₄ und R₅ gemäß der für die Verbindungen der Formel (I) angegebenen Definition stehen, die selbst durch Umsetzung einer Verbindung der Formel (II) mit einer Verbindung der Formel (III) gemäß obiger Definition erhalten werden, erhält.

14. Arzneimittel, **dadurch gekennzeichnet, daß** es mindestens eine Verbindung der Formel (I) gemäß einem der Ansprüche 1 bis 12 enthält.

15. Pharmazeutische Zusammensetzung, **dadurch gekennzeichnet, daß** sie mindestens eine Verbindung der Formel (I) gemäß einem der Ansprüche 1 bis 12 sowie mindestens einen pharmazeutisch unbedenklichen Hilfsstoff enthält.

16. Verwendung einer Verbindung der Formel (I) gemäß einem der Ansprüche 1 bis 12 zur Herstellung eines Arzneimittels zur Behandlung und/oder Prävention von Störungen des Zentralnervensystems oder peripheren Nervensystems, des Herz-KreislaufSystems, des endokrinen und hepatischen Systems, Störungen des Nierenbereichs, des Magen-, Darm- und Lungenbsreichs, in der Ophthalmologie und bei Störungen des Sexualverhaltens.

17. Verwendung einer Verbindung der Formel (I) nach Anspruch 16 zur Herstellung eines Arzneimittels zur Behandlung und/oder Prävention von verschiedenen Vasopressin-abhängigen Störungen sowie Dysfunktionen der Vasopressin-Sekretion wie dem Syndrom der inadäquaten Vasopressin-Sekretion, Herz-Kreislauf-Störungen, wie Hypertonie, pulmonaler Hypertonie, Herzinsuffizienz, Kreislaufinsuffizienz, Myokardinfarkt, Atherosklerose oder koronarem Vasospasmus, insbesondere bei Rauchern, instabiler Angina und perkutaner transluminaler Koronarangioplastie, Herzischämie, Störungen der Hämostase, insbesondere Hämophilie, Von-Willebrand-Syndrom; Störungen des Zentralnervensystems, Schmerzen, Migräne, zerebralem Vasospasmus, Hirnblutung, Hirnödemen, Depression, Angst, Bulimie, psychotischen Zuständen, beispielsweise Gedächtnisstörungen; Renopathien und Nierendysfunktion wie Ödemen, renalem Vasospasmus, Nierenrindennekrose, nephrotischem Syndrom, Nierenpolyzystosen in ihren verschiedenen Formen bei Kindern und Erwachsenen, Hyponatriämien, Hypokaliämien, Diabetes, diabetischen Nephropathien, nephrogenem Diabetes insipidus, "NSIADH", Schwartz-Bartter-Syndrom oder Nierensteinleiden, Infektionen der Harnwege; Störungen des Magensystems, wie Magen-Vasospasmus, portaler Hypertonie, Leberzirrhose, Geschwüren, Vomituspathologie, beispielsweise Übelkeit einschließlich Übelkeit infolge einer Chemotherapie, Bewegungskrankheit, Diabetes insipidus und Enuresis; Störungen des hepatischen Systems wie Leberzirrhose; Bauchwassersucht und alle Störungen, die eine abnormale Wasserretention verursachen; Nebennnierenstörungen, Morbus Cushing, Hyperkortizismus und Hyperaldosteronämie, Störungen des Sexualverhaltens, bei Übergewicht und Adipositas, bei Dysmenorrhoe oder vorzeitigen Wehen, kleinzelligem Lungenkrebs, hyponatriämischen Enzephalopathien, Raynaud-Krankheit, pulmonalem Syndrom, Glaukom und Verhinderung von Katarakt, bei postoperativen Behandlungen, insbesondere nach Bauchchirurgie, Herzchirurgie oder hämorrhagischer Chirurgie, und bei der Behandlung von Störungen oder Erkrankungen des Innenohrs wie Ménière-Krankheit, Tinnitus, Schwindelanfällen, Schwerhörigkeit, insbesondere bei tiefen Tönen, oder Ohrensausen, Hydrops und insbesondere endolymphatischem Hydrops und Osteoporose.
